# EUROPÄISCHE PATENTSCHRIFT

(11) **EP 3 142 499 B1**
(45) Veröffentlichungstag und Bekanntmachung des Hinweises auf die Patenterteilung: **30.10.2019**
(21) Anmeldenummer: 15718467.2
(22) Anmeldetag: 14.04.2015
(51) Int. Cl.: A23B 4/06

(54) **HOMOVANILLINSÄURE-ESTER, INSBESONDERE ZUM ERZIELEN EINES WÄRME- UND/ODER SCHÄRFEEINDRUCKS**
HOMOVANILLIC ACID ESTERS, IN PARTICULAR FOR ACHIEVING AN IMPRESSION OF HEAT AND/OR SPICINESS
ESTER D'ACIDE HOMOVALLINIQUE, EN PARTICULIER POUR OBTENIR UNE IMPRESSION DE CHALEUR ET/OU DE NETTETÉ

(30) Priorität: 16.04.2014 EP 14165020
(43) Veröffentlichungstag der Anmeldung: 22.03.2017
(73) Patentinhaber: Symrise AG, 37603 Holzminden (DE)
(72) Erfinder: BACKES, Michael, 37603 Holzminden (DE); LEY, Jakob Peter, 37603 Holzminden (DE); DEGENHARDT, Andreas, Maidenhead Berkshire SL6 4LL (GB); PAETZ, Susanne, 37671 Höxter (DE); REICHELT, Katharina, 37603 Holzminden (DE); RIESS, Thomas, 37603 Holzminden (DE); KLOSE, Bettina, 80637 München (DE); HENTSCHEL, Fabia, 37603 Holzminden (DE)
(74) Vertreter: Eisenführ Speiser
(86) Internationale Anmeldenummer: PCT/EP2015/058004
(87) Internationale Veröffentlichungsnummer: WO 2015/158677

(56) Entgegenhaltungen:
- WO-A1-03/106404
- US-A1- 2012 093 742
- SZOLCSÀNYI J ET AL: "SENSORY EFFECTS OF CAPSAICIN CONGENERS. I. RELATIONSHIP BETWEEN CHEMICAL STRUCTURE AND PAIN-PRODUCING POTENCY OF PUNGENT AGENTS", ARZNEIMITTEL FORSCHUNG. DRUG RESEARCH, ECV EDITIO CANTOR VERLAG, AULENDORF, DE, Bd. 25, Nr. 12, 1. Januar 1975 (1975-01-01), Seiten 1877-1881, XP009073143, ISSN: 0004-4172
- TAO G ET AL: "Eugenol and its structural analogs inhibit monoamine oxidase A and exhibit antidepressant-like activity", BIOORGANIC & MEDICINAL CHEMISTRY, PERGAMON, GB, Bd. 13, Nr. 15, 1. August 2005 (2005-08-01), Seiten 4777-4788, XP027637988, ISSN: 0968-0896 [gefunden am 2005-08-01]

## Beschreibung

Die vorliegende Erfindung betrifft insbesondere neue Verwendungen von Verbindungen der Formel (I) (wie hierin beschrieben), zum Teil auch neue Verbindungen der Formel (I) als solche, Verbindungen der Formel (I) enthaltende Aromakompositionen, neue Zubereitungen sowie neue Verfahren unter Verwendung von Verbindungen der Formel (I). Weitere Aspekte der vorliegenden Erfindung ergeben sich aus den Patentansprüchen und der nachfolgenden Beschreibung samt Beispielen.

Capsaicin [N-(4-Hydroxy-3-methoxybenzyl)-8-methyl-(6E)-nonensäureamid] und andere Capsaicinoide wie Nonivamid ([N-(4-Hydroxy-3-methoxybenzyl)-nonansäureamid) sind als scharf schmeckende und wärmeerzeugende Aromastoffe aus verschiedenen Capsicum-Arten, insbesondere Chilipfeffer, schon seit langem bekannt. Bei entsprechend geringer Dosierung der Capsaicinoide wird eine neutrale Schärfe und ein Wärmegefühl im Mund wahrgenommen, wobei die Schwelle zum schmerzhaften Schärfe- und Hitzeempfinden sehr schnell überschritten wird. Der Einsatz von Capsaicin in Lebensmitteln ist allerdings in der Europäischen Union nicht erlaubt (wurde von der Community Flavoring List in 2004 gestrichen), da das genotoxische Potential der Verbindung negativ bewertet wurde (European Food Safety Authority (EFSA), P., Italy, Opinion of the Scientific Committee on Food on Capsaicin. European Comission 2002, (SDF/CS/FLAV/FLAVOUR/8 ADD1 Final)). Zudem ist der Einsatz in Lebensmitteln oft schwierig, da Capsaicin einen sehr niedrigen Geschmacksschwellenwert und eine hohe Potenz als Scharfstoff (16,000,000 Scoville units, vgl. http://en.wikipedia.org/wiki/Capsaicin; Version des Eintrags mit letzten Änderungen vom 11. November 2011, 21:02 Uhr) hat. Capsaicin wird, auch auf Grund des hohen Preises des Reinstoffs, zudem nahezu ausschließlich in Form eines Capsicumextrakts verwendet, der neben weiteren Scharfstoffen noch Reste anderer, nach Capsicum schmeckender oder riechender Aromastoffe enthält und daher für den breiten Einsatz nur bedingt geeignet ist. Somit besteht trotz der guten sensorischen Eigenschaften ein Bedarf an weniger problematischen Scharfstoffen.

Das im weißen Pfeffer vorkommende Piperin (1-Piperoylpiperidin) verursacht zwar auch einen starken scharfen Eindruck (Römpp Lexikon Naturstoffchemie, Thieme 1997, S. 500), zeigt aber im Vergleich zu Capsaicin eine relative Schärfe von nur ca. 1 %. Darüber hinaus besitzt Piperin einen intensiven Eigengeschmack, der an Pfeffer erinnert, so dass die Anwendung in vielen Zubereitungen nur beschränkt erfolgen kann.

Auf Grund des lipophilen Charakters dieser vanilloiden Scharfstoffe ist der Einsatz des Schärfeeindrucks oft um einige Sekunden verzögert und hält auch besonders lange an, insbesondere in wenig lipophile Bestandteile (z.B. Triglyceride) enthaltenden Zubereitungen, wobei hier gleichzeitig die Löslichkeit nur unzureichend ist. Ähnliches gilt für Scharfstoffe wie Gingerol-[6] aus Ingwer oder Paradol-[6] aus Paradieskörnern, die beide zwar scharf schmecken, aber ein starken Nachgeschmack haben.

Zwar sind weitere (z.B. Starkenmann, C.; Cayeux, I.; Birkbeck, A. A., Exploring Natural Products for New Taste Sensations. Chimia 2011, 65, (6), 407-410) scharf schmeckende Stoffe wie das Driman Polygodial (aus Tasmanischem Pfeffer, *Tasmannia lanceolata*) oder Resiniferatoxin aus *Euphorbia resinifera* bekannt (Szallasi, A.; Biro, T.; Modarres, S.; Garlaschelli, L.; Petersen, M.; Klush, A.; Vidari, G.; Jonassohn, M.; De Rosa, S.; Sterner, O.; Blumberg, P. M.; Krause, J. E., Dialdehyde sesquiterpenes and other terpenoids as vanilloid. Eur. J. Pharmacol. 1998, 356, 81-89); die Drimane sind aber auf Grund ihrer Dialdehyd-Struktur nur begrenzt einsatzfähig, da sie mit freien Aminogruppen z.B. von Proteinen reagieren und somit ihre Wirkung einbüßen und das Resiniferatoxin ist stark toxisch und nicht geeignet für die menschliche Ernährung. Zudem sind diese Stoffe ebenfalls stark lipophil.

Der Methylester der Homovanillinsäure wurde in verschiedenen Hölzern, welche zur Lagerung von Wein und Spirituosen verwendet werden, nachgewiesen (z. B. Fernandez de Simon, B.; Esteruelas, E.; Munoz, A. M.; Cadahia, E.; Sanz, M., J. Agric. Food Chem. 2009, 57, 3217-3227.). Der Ethylester der Homovanillinsäure wurde hingegen in Wein und Spirituosen selber nachgewiesen, meistens in Zusammenhang mit einer Lagerung in Eichenfässern (z.B. Cabaroglu, T.; Canbas, A.; Baumes, R.; Bayonove, C.; Lepoutre, J. P.; Günata, Z., J. Food Sci. 1997, 62, 680-692. van Jaarsveld, F. P.; Hattingh, S.; Minnaar, P., S. Afr. J. Enol. Vitic. 2009, 30, 24-37.). Die geringen Konzentrationen von z.B. 2 µg/L reichen aber nicht, um einen wärme und/oder schärfeerzeugenden Effekt hervorzurufen.

In US 2009/0170942 A1 sind bestimmte Homovanillinsäure-Ester-Derivate und diverse (medizinische) Anwendungen davon beschrieben.

Zudem wird im Stand der Technik das Homovanillinsäure-Ester-Derivat Ethyl-2-(4-hydroxy-3-methoxyphenyl)acetat als Inhibitor der Monoaminoxidase A beschrieben (Tao G et al.: "Eugenol and its structural analogs inhibit monoamine oxidase A and exhibit antidepressant-like activity", Bioorganic & Medicinal Chemistry 2005, 13, 4777-4788).

Bei einer Untersuchung der Schmerzempfindung verschiedenster Capsaicin-Derivate in Rattenaugen wurden Methyl-, Propyl-, Octyl-, Nonyl- und Dodecylester der Homovanillinsäure getestet und als aktiv bewertet (Szolcsanyi, J.; Jancso-Gabor, A.: "Sensory Effects of Capsaicin Congeners", Arzneim.-Forsch.(Drug. Res.) 1975, 25, 1877-1881).

Im Gegensatz zu den oben genannten Scharfstoffen ist Ethanol ein kleines hydrophiles Molekül, das einen schnellen und angenehmen Schärfeeindruck verursacht, der aber nicht sehr lange vorhält. Da dies erst bei relativ hohen Konzentrationen von 0.5 % und mehr funktioniert, der Genuss von Ethanol aber gesundheitlich problematisch ist und bei dauerhafter Aufnahme auch zur Sucht führen kann, wird nach Aromaformulierungen gesucht, die den Schärfeverlauf von Ethanol simulieren können, ohne dabei die genannten Nachteile zu zeigen. Für diese Anwendung sind schon einige Scharfstoffe beschrieben worden, so z.B. wurden in EP 1,515,943-B1 bestimmte längerkettige Vanillomandelsäurealkylamide oder in WO 2009 065,239 Polygodial und Warburganal als Scharfstoffe zur Erzielung eines Ethanol-ähnlichen Schärfegefühls beschrieben; durch die ebenfalls vorhandene Lipophilie ist wiederum ein langanhaltender Schärfeeindruck zu beobachten, der von den Testern nicht als Ethanol-typischer Eindruck beschrieben wird.

Somit besteht der Bedarf an weniger lipophilen, sensorisch schnell einsetzenden und nicht langanhaltenden Scharfstoffen, die einen wärmenden Geschmackseindruck hervorrufen. Besonderer Bedarf besteht an Stoffen, die oben genannte Eigenschaften besitzen und natürlich vorkommen oder in lebensmittelüblichen Prozessen aus natürlich vorkommenden Lebensmittelbestandteilen oder Aromastoffen gebildet werden.

Diesem Bedarf kann nun erfindungsgemäß durch Verwendung einer Verbindung der Formel (I) 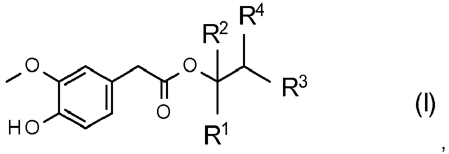 wobei
(i) R¹ und R² unabhängig voneinander ein Wasserstoffatom oder einen Alkylrest mit 1-2 Kohlenstoffatomen darstellen,
   R³ und R⁴ unabhängig voneinander ein Wasserstoffatom oder einen linearen oder verzweigten Alkylrest mit 1 bis 5 Kohlenstoffatomen (beispielsweise ausgewählt aus der Gruppe bestehend aus Methyl, Ethyl, 1-Propyl, 2-Propyl-, 1-Butyl, 2-Butyl, tert-Butyl, 2-Methylprop-1-yl, 1-, 2- oder 3-Pentyl-, 2-Methylbut-1-yl, 2-Methylbut-2-yl, 3-Methylbut-1-yl und 3-Methylbut-2-yl, vorzugsweise aus Methyl, Ethyl, 1-Propyl, 2-Propyl, 1-Butyl, 2-Butyl, tert-Butyl, 2-Methylprop-1-yl und 1-Pentyl), einen Phenylrest, einen Alkylphenylrest oder einen Phenylalkylrest oder einen linearen oder verzweigten Alkenylrest mit 2 bis 4 Kohlenstoffatomen (beispielsweise ausgewählt aus der Gruppe bestehend aus Ethenyl, Prop-2-en-1-yl, Prop-1-en-1-yl, Prop-1-en-2-yl, 1- oder 2-Cyclopropenyl-, But-1-en-1-yl, But-1-en-2-yl, But-1-en-3-yl, But-2-en-1-yl, But-3-en-1-yl, But-2-en-2-yl, 2-Methylprop-1-en-1-yl, 2-Methylprop-2-en-1-yl, 1,3-Butadien-1-yl, 1,3-Butadien-2-yl, und die jeweiligen gegebenenfalls möglichen Z- und E-Isomere) oder einen Alkenylphenylrest oder einen Phenylalkylenrest darstellen,
   oder
(ii) R¹ und R³ zusammen mit den sie verknüpfenden Kohlenstoffatomen einen Cyclohexylring bilden (die Reste R² und R⁴ sind dann Substituenten des Cycloalkylrings; s. hierzu z.B. Formel (la) weiter unten), der optional mit einem zusätzlichen Rest R⁵ substituiert ist, wobei R⁵ ein Alkylrest mit 1-2 Kohlenstoffatomen ist,
   R² ein Wasserstoffatom oder einen Alkylrest mit 1-2 Kohlenstoffatomen darstellt,
   R⁴ ein Wasserstoffatom oder einen linearen oder verzweigten Alkylrest mit 1 bis 5 Kohlenstoffatomen (beispielsweise ausgewählt aus der Gruppe bestehend aus Methyl, Ethyl, 1-Propyl, 2-Propyl-, 1-Butyl, 2-Butyl, tert-Butyl, 2-Methylprop-1-yl, 1-, 2- oder 3-Pentyl-, 2-Methylbut-1-yl, 2-Methylbut-2-yl, 3-Methylbut-1-yl und 3-Methylbut-2-yl, vorzugsweise aus Methyl, Ethyl, 1-Propyl, 2-Propyl, 1-Butyl, 2-Butyl, tert-Butyl, 2-Methylprop-1-yl und 1-Pentyl), einen Phenylrest, einen Alkylphenylrest oder einen Phenylalkylrest oder einen linearen oder verzweigten Alkenylrest mit 2 bis 4 Kohlenstoffatomen (beispielsweise ausgewählt aus der Gruppe bestehend aus Ethenyl, Prop-2-en-1-yl, Prop-1-en-1-yl, Prop-1-en-2-yl, 1- oder 2-Cyclopropenyl-, But-1-en-1-yl, But-1-en-2-yl, But-1-en-3-yl, But-2-en-1-yl, But-3-en-1-yl, But-2-en-2-yl, 2-Methylprop-1-en-1-yl, 2-Methylprop-2-en-1-yl, 1,3-Butadien-1-yl, 1,3-Butadien-2-yl, und die jeweiligen gegebenenfalls möglichen Z- und E-Isomere) oder einen Alkenylphenylrest oder einen Phenylalkylenrest darstellt,
oder eines physiologisch akzeptablen Salzes davon, wobei die phenolische Hydroxygruppe in Formel (I) deprotoniert ist, oder einer Mischung umfassend eine oder mehrere Verbindungen der Formel (I) und/oder physiologisch akzeptable Salze (insbesondere seine Natrium-, Kalium-, Ammonium-, Calcium-, Magnesium- oder Zinksalze) davon, wobei jeweils die phenolische Hydroxygruppe in Formel (I) deprotoniert ist, oder bestehend aus mehreren unterschiedlichen Verbindungen der Formel (I) und/oder Salzen davon, wobei jeweils die phenolische Hydroxygruppe in Formel (I) deprotoniert ist,
als Aromastoff bzw. Scharfstoff mit einem wärme- und/oder schärfeerzeugenden Effekt, d.h. als Stoff, der sensorisch einen Wärmeeindruck hervorrufen kann,
und
- als Aromastoff zum Verstärken eines angenehmen Geschmackseindrucks, ausgewählt aus der Gruppe bestehend aus wärmend, scharf und kühlend (weitere Details hierzu ergeben sich aus den Ausführungen weiter unten)
Rechnung getragen werden.

An dieser Stelle ist anzumerken, dass die hierin beschriebenen Vorteile und Effekte der Verbindungen der Formel (I) in der Regel für deren Salze (wie hierin beschrieben) entsprechend gelten.

Im Falle einer Mischung von unterschiedlichen Verbindungen der Formel (I) (wie hierin beschrieben) gilt im Rahmen des vorliegenden Textes, dass die unterschiedlichen Verbindungen z.B. nicht nur Verbindungen mit unterschiedlicher Summenformel, sondern auch verschiedene Stereoisomere mit identischer Summenformel sein können.

Bevorzugt ist eine Verwendung wie oben beschrieben, wobei für die Verbindung der Formel (I) bzw. eine, mehrere oder sämtliche Verbindungen der Formel (I) unabhängig voneinander in der Mischung gilt:
(i) R¹ und R² stellen unabhängig voneinander ein Wasserstoffatom oder Methyl dar,
   R³ und R⁴ stellen unabhängig voneinander ein Wasserstoffatom oder einen linearen oder verzweigten Alkylrest mit 1 bis 5 Kohlenstoffatomen oder einen Phenylrest, einen Alkylphenylrest oder einen Phenylalkylrest oder einen Alkenylphenylrest oder einen Phenylalkenylrest (beispielsweise wie oben beschrieben) dar,
   oder
(ii) Formel (I) entspricht der folgenden Formel (la) 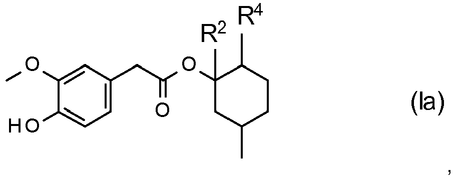 R² stellt ein Wasserstoffatom dar,
   R⁴ stellt 2-Propyl dar.

Bevorzugt ist auch eine Verwendung wie oben beschrieben, wobei für die Verbindung der Formel (I) bzw. eine, mehrere oder sämtliche Verbindungen der Formel (I) unabhängig voneinander in der Mischung gilt:
R¹ und R² stellen jeweils ein Wasserstoffatom dar,
R³ stellt ein Wasserstoffatom oder einen linearen oder verzweigten Alkylrest mit 1 bis 4 Kohlenstoffatomen oder einen Phenylrest, einen Alkylphenylrest oder einen Phenylalkylrest oder einen Alkenylphenylrest oder einen Phenylalkenylrest dar,
R⁴ stellt ein Wasserstoffatom dar.

Besonders bevorzugt ist die Verbindung der Formel (I) bzw. sind eine, mehrere oder sämtliche Verbindungen der Formel (I) in der Mischung ausgewählt bzw. aus der Gruppe bestehend aus
2-Phenylethyl-2-(4-hydroxy-3-methoxy-phenyl)acetat (**1**) 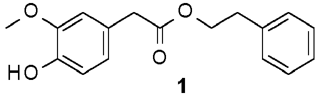
[(*E*)-Cinnamyl]-2-(4-hydroxy-3-methoxy-phenyl)acetat (**2**) 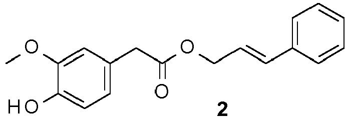
1-Ethylbutyl-2-(4-hydroxy-3-methoxy-phenyl)acetat (**3**) 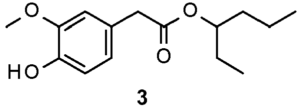
3-Methylbut-2-enyl-2-(4-hydroxy-3-methoxy-phenyl)acetat (**4**) 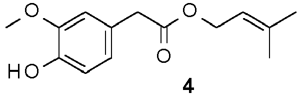
[(*E*)-Hex-2-enyl]-2-(4-hydroxy-3-methoxy-phenyl)acetat (**5**) 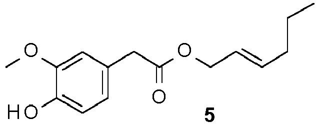
[(*Z*)-Hex-3-enyl]-2-(4-hydroxy-3-methoxy-phenyl)acetat (**6**) 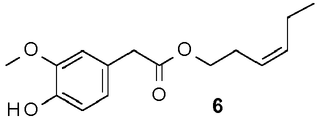
Isopropyl-2-(4-hydroxy-3-methoxy-phenyl)acetat (**7**) 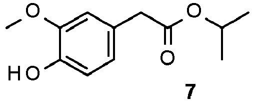
*sec*-Butyl-2-(4-hydroxy-3-methoxy-phenyl)acetat (**8**) 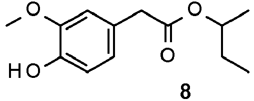
Isobutyl-2-(4-hydroxy-3-methoxy-phenyl)acetat (**9**) 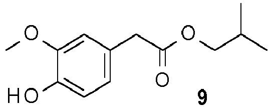
1,1-Dimethylpropyl 2-(4-hydroxy-3-methoxy-phenyl)acetat (**10**) 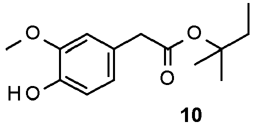
Isopentyl-2-(4-hydroxy-3-methoxy-phenyl)acetat (**11**) 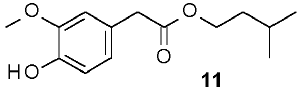
2-Methylbutyl-2-(4-hydroxy-3-methoxy-phenyl)acetat (**12**) 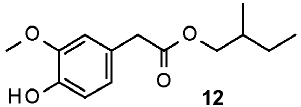
1-Methylpentyl-2-(4-hydroxy-3-methoxy-phenyl)acetat (**13**) 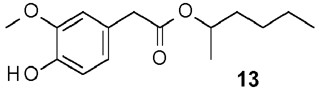
Heptyl-2-(4-hydroxy-3-methoxy-phenyl)acetat (**14**) 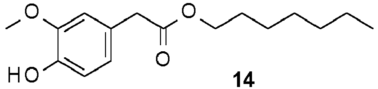
1-Methylhexyl-2-(4-hydroxy-3-methoxy-phenyl)acetat (**15**) 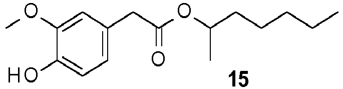
(2-Isopropyl-5-methyl-cyclohexyl)-2-(4-hydroxy-3-methoxy-phenyl)acetat (**16**) 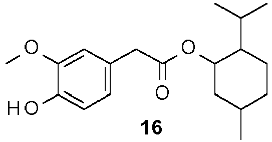
Ethyl-2-(4-hydroxy-3-methoxy-phenyl)acetat (**17**) 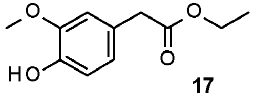
Propyl-2-(4-hydroxy-3-methoxy-phenyl)acetat (**18**) 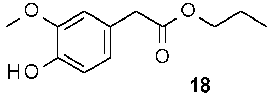
Butyl-2-(4-hydroxy-3-methoxy-phenyl)acetat (**19**) 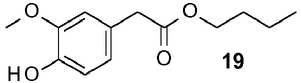
Pentyl-2-(4-hydroxy-3-methoxy-phenyl)acetat (**20**) 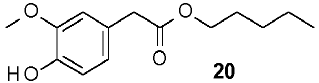
Hexyl-2-(4-hydroxy-3-methoxy-phenyl)acetat (**21**) 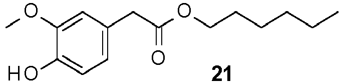
3-Phenylpropyl 2-(4-hydroxy-3-methoxy-phenyl)acetat (**22**) 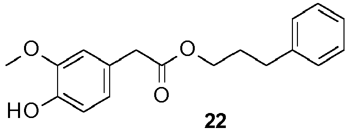
4-Phenylbutyl 2-(4-hydroxy-3-methoxy-phenyl)acetat (**23**)

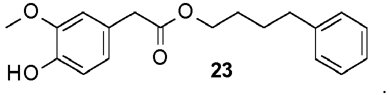

Die hierin beschriebenen Verbindungen eignen sich vorteilhafterweise zur Verwendung (insbesondere wie oben) in einer pharmazeutischen, einer der Ernährung, der Mundhygiene oder dem Genuss dienenden Zubereitung, vorzugsweise wobei die Gesamtmenge an Verbindung(en) der Formel (I) und/oder Salz(en) davon in der Zubereitung ausreicht, um
bei Gebrauch oder Verzehr der Zubereitung sensorisch einen wärmenden und/oder scharfen Effekt auf der Zunge oder im Mundraum hervorzurufen,
und
einen angenehmen Geschmackseindruck, ausgewählt aus der Gruppe bestehend aus wärmend, scharf und kühlend, zu verstärken (weitere Details hierzu ergeben sich aus den Ausführungen weiter unten).

Ein weiterer Aspekt der vorliegenden Erfindung betrifft neue Verbindungen der Formel (I), Salze davon deren Mischungen, nämlich eine Verbindung der Formel (I) oder ein physiologisch akzeptables Salz davon, wobei die phenolische Hydroxygruppe in Formel (I) deprotoniert ist, oder eine Mischung umfassend eine oder mehrere unterschiedliche Verbindungen der Formel (I) und/oder ein oder mehrere physiologisch akzeptable Salze davon, wobei die phenolische Hydroxygruppe in Formel (I) jeweils deprotoniert ist, oder bestehend aus mehreren unterschiedlichen Verbindungen der Formel (I) und/oder physiologisch akzeptablen Salzen davon, wobei die phenolische Hydroxygruppe in Formel (I) jeweils deprotoniert ist, 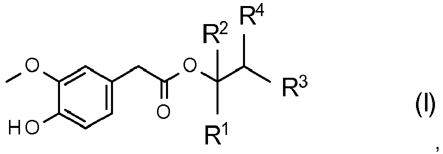 wobei
(i) R¹ und R² unabhängig voneinander ein Wasserstoffatom oder einen Alkylrest mit 1-2 Kohlenstoffatomen darstellen,
   R³ und R⁴ unabhängig voneinander ein Wasserstoffatom oder einen linearen oder verzweigten Alkylrest mit 1 bis 5 Kohlenstoffatomen (beispielsweise wie oben beschrieben), einen Phenylrest, einen Alkylphenylrest oder einen Phenylalkylrest oder einen linearen oder verzweigten Alkenylrest mit 2 bis 4 Kohlenstoffatomen (beispielsweise wie oben beschrieben) oder einen Alkenylphenylrest oder einen Phenylalkenylrest darstellen,
   oder
(ii) R¹ und R³ zusammen mit den sie verknüpfenden Kohlenstoffatomen einen Cyclohexylring bilden, der optional mit einem zusätzlichen Rest R⁵ substituiert ist, wobei R⁵ ein Alkylrest mit 1-2 Kohlenstoffatomen ist,
   R² ein Wasserstoffatom oder einen Alkylrest mit 1-2 Kohlenstoffatomen darstellt,
   R⁴ ein Wasserstoffatom oder einen linearen oder verzweigten Alkylrest mit 1 bis 5 Kohlenstoffatomen (beispielsweise wie oben beschrieben), einen Phenylrest, einen Alkylphenylrest oder einen Phenylalkylrest oder einen linearen oder verzweigten Alkenylrest mit 2 bis 4 Kohlenstoffatomen (beispielsweise wie oben beschrieben) oder einen Alkenylphenylrest oder einen Phenylalkenylrest darstellt,
mit der Maßgabe, dass die Verbindung der Formel (I) bzw. eine, mehrere oder sämtliche Verbindungen der Formel (I) in der Mischung ausgewählt ist bzw. sind aus der Gruppe bestehend aus
[(*E*)-Cinnamyl]-2-(4-hydroxy-3-methoxy-phenyl)acetat (**2**) 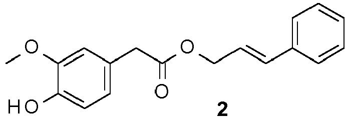
1-Ethylbutyl-2-(4-hydroxy-3-methoxy-phenyl)acetat (**3**) 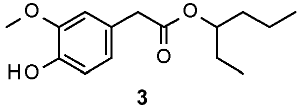
3-Methylbut-2-enyl-2-(4-hydroxy-3-methoxy-phenyl)acetat (**4**) 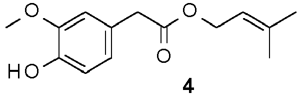
[(*E*)-Hex-2-enyl]-2-(4-hydroxy-3-methoxy-phenyl)acetat (**5**) 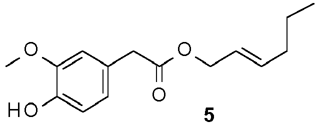
[(*Z*)-Hex-3-enyl]-2-(4-hydroxy-3-methoxy-phenyl)acetat (**6**) 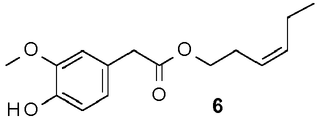
1,1-Dimethylpropyl-2-(4-hydroxy-3-methoxy-phenyl)acetat (**10**) 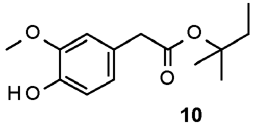
2-Methylbutyl-2-(4-hydroxy-3-methoxy-phenyl)acetat (**12**) 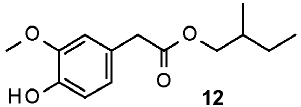
1-Methylpentyl-2-(4-hydroxy-3-methoxy-phenyl)acetat (**13**) 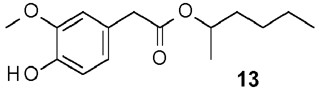
1-Methylhexyl-2-(4-hydroxy-3-methoxy-phenyl)acetat (**15**) 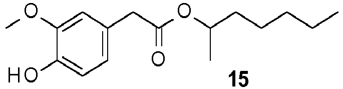
(2-Isopropyl-5-methyl-cyclohexyl)-2-(4-hydroxy-3-methoxy-phenyl)acetat (**16**) 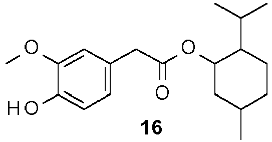
4-Phenylbutyl 2-(4-hydroxy-3-methoxy-phenyl)acetat (**23**)

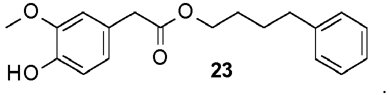

Im Übrigen gilt für die neuen Verbindungen der Formel (I) und deren Salze sowie Mischungen davon das oben im Zusammenhang mit den erfindungsgemäß zu verwendenden Verbindungen, Salzen und Mischungen Gesagte entsprechend.

Im Rahmen der vorliegenden Erfindung wurde festgestellt, dass Verbindungen der Formel (I) bzw. deren Salze und Mischungen davon, vorteilhafterweise ab Konzentrationen von 0.1 mg/kg, insbesondere von 1.0 mg/kg, schnell aufkommende und wenig lang anhaltende, angenehme Schärfe- bzw. Wärme- und leicht stechende Eindrücke vermitteln.

Die vorliegende Erfindung betrifft demnach insbesondere auch neue Aromakompositionen, nämlich eine Aromakomposition
(A) umfassend oder bestehend aus einer neuen, erfindungsgemäßen Mischung wie oben beschrieben,
   vorzugsweise wobei
   die Gesamtmenge an Verbindung(en) der Formel (I) und/oder Salz(en) davon in der Aromakomposition, bezogen auf das Gesamtgewicht der Aromakomposition, im Bereich von 100 bis 100.000 mg/kg liegt, vorzugsweise im Bereich von 250 bis 40.000 mg/kg, besonders bevorzugt im Bereich von 250 bis 15.000 mg/kg.

Vorzugsweiseumfasst die Aromakomposition zudem einen oder mehrere weitere, nicht der Formel (I) entsprechende Aromastoffe, beispielsweise ausgewählt aus der Gruppe bestehend aus
a) Wärme verursachende oder Scharfstoffe, bevorzugt ausgewählt aus der Liste bestehend aus: Capsaicinoiden, wie zum Beispiel Capsaicin, Dihydrocapsaicin oder Nonivamid; Gingerolen, wie zum Beispiel Gingerol-[6], Gingerol-[8], oder Gingerol-[10]; Shogaolen wie Shogaol-[6], Shogaol-[8], Shogaol-[10]; Gingerdionen, wie zum Beispiel Gingerdion-[6], Gingerdion-[8] oder Gingerdion-[10]; Paradolen, wie zum Beispiel Paradol-[6], Paradol-[8] oder Paradol-[10]; Dehydrogingerdionen, wie zum Beispiel Dehydrogingerdion-[6], Dehydrogingerdion-[8] oder Dehydrogingerdion-[10]; Piperin und Piperinderivaten;
b) als stechend oder beißend wahrnehmbare Stoffe, bevorzugt ausgewählt aus der Gruppe bestehend aus: aromatischen Isothiocyanaten, wie zum Beispiel Phenylethylisothiocyanat, Allylisothiocyanat, Cyclopropylisothiocyanat, Butylisothiocyanat, 3-Methylthiopropylisothiocyanat, 4-Hydroxybenzylisothiocyanat, 4-Methoxybenzylisothiocyanat;
c) als kribbelnd (tingling) beschriebenen Alkamiden, vorzugsweise ausgewählt aus der Gruppe bestehend aus 2E,4E-Decadiensäure-N-isobutylamid (trans-Pellitorin) (insbesondere solche wie beschrieben in WO 2004/043906, die bezüglich der darin offenbarten entsprechenden Verbindungen auf dem Wege der Verweisung Bestandteil dieser Anmeldung wird); 2E,4Z- Decadiensäure-N-isobutylamid (cis-Pellitorin) (insbesondere solche wie beschrieben in WO 2004/000787, die bezüglich der darin offenbarten entsprechenden Verbindungen auf dem Wege der Verweisung Bestandteil dieser Anmeldung wird); 2Z,4Z- Decadiensäure-N-isobutylamid; 2Z,4E- Decadiensäure-N-isobutylamid; 2E,4E-Decadiensäure-N-([2S]-2-methylbutyl)amid; 2E,4E-Decadiensäure-N-([2S]-2-methylbutyl)amid; 2E,4E-Decadiensäure-N-([2R]-2-methylbutylamid); 2E,4Z-Decadiensäure-N-(2-methylbutyl)amid; 2E,4E-Decadiensäure-N-piperid (Achilleamid); 2E,4E-Decadiensäure-N-piperid (Sarmentin); 2E-Decensäure-N-isobutylamid; 3E-Decensäure-N-isobutylamid; 3E-Nonensäure-N-isobutylamid; 2E,6Z,8E-Decatriensäure-N-isobutylamid (Spilanthol); 2E,6Z,8E-Decatriensäure-N-([2S]-2-methylbutyl)amid (Homospilanthol); 2E,6Z,8E-Decatriensäure-N-([2R]-2-methylbutyl)amid; 2E-Decen-4-insäure-N-isobutylamid; 2Z-Decen-4-insäure-N-isobutylamid; 2E,6Z,8E,10E-Dodecatetraensäure-N-(2-methylpropyl)amid (alpha-Sanshool); 2E,6Z,8E,10E-Dodecatetraensäure-N-(2-hydroxy-2-methylpropyl)amid (alpha-Hydroxysanshool); 2E,6E,8E,10E-Dodecatetraensäure-N-(2-hydroxy-2-methylpropyl)amid (gamma-Hydroxysanshool); 2E,4E,8Z,10E,12E-Tetradecapentaensäure-N-(2-hydroxy-2-methylpropyl)amid (gamma-Hydroxysanshool); 2E,4E,8E,10E,12E-Tetradecapentaensäure-N-(2-hydroxy-2-methylpropyl)amid (gamma-Hydroxyisosanshool); 2E,4E,8Z,10E,12E-Tetradecapentaensäure-N-(2-methyl-2-propenyl)amid (gamma-Dehydrosanshool); 2E,4E,8Z,10E,12E-Tetradecapentaensäure-N-(2-methylpropyl)amid (gamma-Sanshool); 2E,4E,8Z,11Z-Tetradecatetraensäure-N-(2-hydroxy-2-methylpropyl)amid (Bungeanool); 2E,4E,8Z,11E-Tetradecatetraensäure-N-(2-hydroxy-2-methylpropyl)amid (Isobungeanool); 2E,4E,8Z-Tetradecatriensäure-N-(2-hydroxy-2-methylpropyl)amid (Dihydrobungeanool) und 2E,4E -Tetradecadiensäure-N-(2-hydroxy-2-methylpropyl)amid (Tetrahydrobungeanool);
d) Stoffe mit physiologischer Kühlwirkung, vorzugsweise ausgewählt aus der folgenden Liste: Menthol und Mentholderivate (z.B. L-Menthol, D-Menthol, racemisches Menthol, Isomenthol, Neoisomenthol, Neomenthol) Menthylether (z.B. (L-Menthoxy)-1,2-propandiol, (L-Menthoxy)-2-methyl-1,2-propandiol, L-Menthyl-methylether), Menthylester (z.B. Menthylformiat, Menthylacetat, Menthylisobutyrat, Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat oder deren Gemischen), die Halbester von Mentholen mit einer Dicarbonsäure oder deren Derivate (z.B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), andere als in der vorliegenden Erfindung genannte Menthancarbonsäureamide (z.B. Menthancarbonsäure-N-ethylamid [WS3], Menthancarbonsäure-N-(p-methoxyphenyl)amid [SC1], Nα-(Menthancarbonyl)glycinethylester [WS5], Menthancarbonsäure-N-(4-cyanophenyl)amid, Menthancarbonsäure-N-(alkoxyalkyl)amide), Menthon und Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid [WS23]), Isopulegol oder seine Ester (I-(-)-Isopulegol, I-(-)-Isopulegolacetat), Menthanderivate (z.B. p-Menthan-3,8-diol), Cubebol oder synthetische oder natürliche Mischungen, enthaltend Cubebol, Pyrrolidonderivate von Cycloalkyldionderivaten (z.B. 3-Methyl-2(1-pyrrolidinyl)-2-cyclopenten-1-on) oder Tetrahydropyrimidin-2-one (z.B. Icilin oder verwandte Verbindungen, wie in WO 2004/026840 beschrieben), weitere Kühlwirkstoffe wie in WO2011061330 beschrieben, insbesondere Derivate verschieden substituierter Zimt- und 2-Phenoxysäuren, besonders bevorzugt Methylendioxyzimtsäure-N,N-diphenylamid, Methylendioxyzimtsäure-N-ethyl-N-phenylamid, Methylendioxyzimtsäure-N-pyridyl-N-phenylamid;
e) Stoffe mit adstringierender Wirkung, vorzugsweise ausgewählt aus der folgenden Liste: Catechine wie z.B. Epicatechine, Gallocatechine, Epigallocatechine sowie deren jeweiligen Gallussäureester, z.B. Epigallocatechingallat oder Epicatechingallat, dern Oligomere (Procyanidine, Proanthocyanidine, Prodelphinidine, Procyanirine, Thearubigenine, Theogalline) sowie deren C- und O-Glycoside; Dihydroflavonoide wie Dihydromyricetin, Taxifolin, sowie deren C- und O-Glycoside, Flavonole wie Myricetin, Quercetin sowie deren C- und O-Glycoside wie Quercetrin, Rutin, Gallussäaureester von Kohlenhydraten wie Tannin, Pentagalloylglucose oder deren Reaktionsprodukte wie Elligatannin, Aluminiumsalze, z.B. Alaun,

Insbesondere in den hierin beschriebenen erfindungsgemäß bzw. erfindungsgemäß bevorzugt zu verwendenden Konzentrationen haben Verbindungen der Formel (I) bzw. deren Salze oder Mischungen davon vorteilhafterweise häufig keine signifikanten anderen bzw. unerwünschten Aromawirkungen, können also besonders gut in vielen verschiedenen Aromatypen eingesetzt werden.

Besonders vorteilhaft sind Aromakompositionen, die Kombinationen von Verbindungen der Formel (I) bzw. deren Salzen mit einem oder mehreren anderen trigeminal (scharf, wärmend, stechend, beißend, kratzend, kühlend, betäubend, kribbelnd (tingling), adstringierend) wirksamen Stoffen enthalten, wobei deren trigeminaler (Haupt-)Effekt durch Verbindungen der Formel (I) bzw. deren Salze vorteilhafterweise moduliert werden kann. Dabei kann beispielsweise ein wärmender, scharfer oder kühlender Effekt verstärkt werden, während ein adstringierender Effekt abgeschwächt werden kann.

Bevorzugt ist daher auch eine Aromakomposition, die zudem einen oder mehrere nicht der Formel (I) entsprechende Stoffe mit unangenehmer, insbesondere bitterer Geschmacksqualität, oder einer adstringierenden, bitteren, trockenen, staubigen, mehligen, kalkigen und/oder metallischen Note enthält, vorzugsweise ausgewählt aus der Gruppe bestehend aus:
f) Xanthinalkaloide, Xanthine (Coffein, Theobromin, Theophyllin und Methylxanthine), Alkaloide (Chinin, Brucin, Strychnin, Nicotin), phenolische Glycoside (z.B. Salicin, Arbutin), Flavonoidglycoside (z.B. Neohespereidin, Hesperidin, Naringin, Quercitrin, Rutin, Hyperosid, Quercetin-3-O-glucosid, Myricetin-3-O-glycoside), Chalcone oder Chalconglycoside (z.B. Phloridzin, Phloridzinxyloside), hydrolisierbare Tannine (Gallus- oder Elagsäureester von Kohlenhydraten, z.B. Pentagalloylglucose, Tanninsäuren), nichthydrolisierbare Tannine (ggfs. galloylierte Catechine, Gallocatechine, Epigallocatechine oder Epicatechine und deren Oligomeren, z.B. Proanthyocyanidine oder Procyanidine, Thearubigenin), Flavone (z.B. Quercetin, Taxifolin, Myricetin), Phenole wie z.B. Salicin, Polyphenole (z.B. gamma-Oryzanol, Kaffeesäure oder deren Ester (z. B. Chlorogensäure und Isomere)), terpenoide Bitter- und Gerbstoffe (z.B. Limonoide wie Limonin oder Nomilin aus Zitrusfrüchten, Lupolone und Humolone aus Hopfen, Iridoide, Secoiridoide), Absinthin aus Wermut, Amarogentin aus Enzian, metallische Salze (insbesondere Kalium-, Magnesium- und Calciumsalze, Kaliumchlorid, Kaliumgluconat, Kaliumcarbonat, Kaliumsulfat, Kaliumlactat, Kaliumglutamat, Kaliumsuccinat, Kaliummalat, Natriumsulfat, Magnesiumsulfat, Aluminiumsalze, Zinksalze, Zinnsalze, Eisen-(II)-salze, Eisen-(III)-salze, Chrom-(II)-picolinat), pharmazeutische Wirkstoffe (z.B. Fluorchinolon-Antibiotika, Paracetamol, Aspirin, beta-Lactam-Antibiotika, Ambroxol, Propylthiouracil [PROP], Guaifenesin), Vitamine (beispielsweise Vitamin H, Vitamine aus der B-Reihe wie Vitamin B1, B2, B6, B12, Niacin, Panthotensäure), Denatoniumbenzoat, Sucraloseoctaacetat, Eisensalze, Aluminiumsalze, Zinksalze, Harnstoff, ungesättigte Fettsäuren, insbesondere ungesättigte Fettsäuren in Emulsionen, bitter/adstringierend schmeckende Aminosäuren (z.B. Leucin, Isoleucin, Valin, Tryptophan, Prolin, Histidin, Tyrosin, Lysin oder Phenylalanin) und bitter oder adstringierend schmeckende Peptide oder Proteine (insbesondere Peptide mit einer Aminosäure aus der Gruppe Leucin, Isoleucin, Valin, Tryptophan, Prolin oder Phenylalanin am N- oder C-Terminus), Saponine, insbes. Sojasaponine, Isoflavonoide (insbes. Genistein, Daidzein, Genistin, Daidzin, deren Glycoside und acylierten Glycoside);
g) Stoffe mit einem nicht unangenehmen Primärgeschmack (beispielsweise süß, salzig, würzig, sauer) und/oder -geruch, vorzugsweise ausgewählt aus der Gruppe der Süßstoffe oder Zuckeraustauschstoffe, vorzugsweise Kaliumsalze (insbesondere Kaliumchlorid, Kaliumgluconat, Kaliumcarbonat, Kaliumsulfat, Kaliumlactat, Kaliumglutamat, Kaliumsuccinat, Kaliummalat), Aspartam, Acesulfam K, Neotam, Superaspartam, Saccharin, Sucralose, Tagatose, Monellin, Stevioside, Rebaudioside, Rebaudiosid A, Rebaudiosid B, Rebaudiosid C, Rebaudiosid D, Rebaudiosid X, Rubusoside, Hernandulcin, Thaumatin, Miraculin, Glycyrrhizin, Glycyrrhetinsäure, Balansin A oder Balansin B, oder deren Derivate, Cyclamat oder die pharmazeutisch akzeptablen Salze der vorgenannten Verbindungen.

Die vorliegende Erfindung betrifft weiterhin eine pharmazeutische Zubereitung, der Ernährung, der Mundhygiene oder dem Genuss dienende Zubereitung, umfassend
(A) eine neue, erfindungsgemäße Mischung wie oben beschrieben,
   vorzugsweise wobei
   die Gesamtmenge an Verbindung(en) der Formel (I) und/oder Salz(en) davon in der Zubereitung, bezogen auf das Gesamtgewicht der Zubereitung, im Bereich von 0,1 bis 1.000 mg/kg liegt, vorzugsweise im Bereich von 1 bis 1.000 mg/kg, vorzugsweise im Bereich von 1 bis 750 mg/kg, besonders bevorzugt im Bereich von 5 bis 500 mg/kg,
   oder
(C) eine Aromakomposition wie oben beschrieben,
   vorzugsweise wobei
   die Gesamtmenge an Verbindung(en) der Formel (I) und/oder Salz(en) davon in der Zubereitung, bezogen auf das Gesamtgewicht der Zubereitung, im Bereich von 0,1 bis 1.000 mg/kg liegt, vorzugsweise im Bereich von 1 bis 1.000 mg/kg, vorzugsweise im Bereich von 1 bis 750 mg/kg, besonders bevorzugt im Bereich von 5 bis 500 mg/kg,
   wobei die Gesamtmenge an Verbindung(en) der Formel (I) und/oder Salz(en) davon in der Zubereitung ausreicht, um
   bei Gebrauch oder Verzehr der Zubereitung sensorisch einen wärmenden und/oder scharfen Effekt auf der Zunge oder im Mundraum hervorzurufen,
   und
   einen angenehmen Geschmackseindruck, vorzugsweise eines anderen in der Zubereitung enthaltenen Stoffes, ausgewählt aus der Gruppe bestehend aus wärmend, scharf und kühlend, zu verstärken (vgl. hierzu oben).

Bevorzugt umfasst eine erfindungsgemäße Zubereitung zudem
ein oder mehrere übliche Grund-, Hilfs- und Zusatzstoffe in einer Menge von, bezogen auf das Gesamtgewicht der Zubereitung, 5 bis 99,9999 Gew.-%, vorzugsweise 10 bis 80 Gew.-%,
und/oder
Wasser in einer Menge, bezogen auf das Gesamtgewicht der Zubereitung, bis zu 99,9999 Gew.-%, vorzugsweise in einer Menge von 5 bis 80 Gew.-%.

Bevorzugt ist ferner eine erfindungsgemäße Zubereitung, die neben Verbindungen der Formel (I) bzw. deren Salzen (wie oben definiert) zumindest eine weitere Substanz zum Verändern, Maskieren oder Vermindern des unangenehmen Geschmackseindrucks eines unangenehm schmeckenden Stoffes oder Stoffgemisches umfasst. Entsprechend liegt dann eine Kombination von zumindest zwei Geschmackskorrigenzien vor.

Der Ernährung oder dem Genuss dienende Zubereitungen im Sinne der Erfindung sind z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nicht-alkoholische Getränke (z.B. Kakao, Kaffee, Grüntee, Schwarztee, mit (Grün-, Schwarz-)Tee-Extrakten angereicherte (Grün-, Schwarz-)Tee-Getränke, Rooibos-Tee, andere Kräutertee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Vollfett- oder fettreduzierte oder fettfreie Milchgetränke, Milchreis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, isoliertes oder enzymatisch behandeltes Sojaprotein enthaltende Getränke, Sojamehl enthaltende Getränke, Sojalecithin-haltige Zubereitungen, fermentierte Produkte wie Tofu oder Tempe oder daraus gefertigte Produkte und Mischungen mit Fruchtzubereitungen und fakultativ Aromen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, eingekochte Gemüse), Knabberartikel (z.B. gebackene oder frittierte Kartoffelchips oder Kartoffelteigprodukte, Extrudate auf Mais- oder Erdnussbasis), Produkte auf Fett- und Ölbasis oder Emulsionen derselben (z.B. Mayonnaise, Remoulade, Dressings, jeweils Vollfett- oder fettreduziert), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden, Süßstoffzubereitungen, -tabletten oder -sachets, sonstige Zubereitung zum Süßen oder Weißen von Getränken oder anderen Nahrungsmitteln. Die Zubereitungen im Sinne der Erfindung können auch als Halbfertigware zur Herstellung weiterer der Ernährung oder dem Genuss dienenden Zubereitungen dienen.

Pharmazeutische Zubereitungen umfassen einen pharmazeutischen Wirkstoff. Vorteilhafte pharmazeutische Wirkstoffe sind beispielsweise steroidale entzündungshemmende Substanzen vom Kortikosteroiden-Typ wie z. B. Hydrocortison, Hydrocortison-Derivate wie Hydrocortison-17-butyrat, Dexamethason, Dexamethasonphosphat, Methylprednisolon oder Cortison. Vorteilhafte nichtsteroidale pharmazeutische Wirkstoffe sind beispielsweise Entzündungshemmer wie Oxicame wie Piroxicam oder Tenoxicam; Salicylate wie Aspirin® (Acetylsalicyl-säure), Disalcid, Solprin oder Fendosal; Essigsäure-Derivate wie Diclofenac, Fenclofenac, Indomethacin, Sulindac, Tolmetin, oder Clindanac; Fenamate wie Mefenamic, Meclofenamic, Flufenamic oder Niflumic; Propionsäure-Derivate wie Ibuprofen, Naproxen, Flurbiprofen, Benoxaprofen oder Pyrazole wie Phenylbutazon, Oxyphenylbutazon, Febrazon oder Azapropazon.

Besonders bevorzugte pharmazeutische Zubereitungen sind nicht verschreibungspflichtige Produkte und frei verkäufliche Arzneimittel, sogenannte OTC ("over the counter") -Präparate, enthaltend Wirkstoffe wie Paracetamol, Acetylsalicylsäure oder Ibuprofen, Vitamine (beispielsweise Vitamin H, Vitamine aus der B-Reihe wie Vitamin B1, B2, B6, B12, Niacin, Panthotensäure, vorzugsweise in Form von (Brause-)Tabletten oder Kapseln), Mineralien (vorzugsweise in Form von (Brause-)Tabletten oder Kapseln) wie Eisensalze, Zinksalze, Selensalze, Produkte enthaltend Wirkstoffe oder Extrakte von Spitzwegerich (z.B. in Hustensirup) oder Johanniskraut.

Die Zubereitungen im Sinne der Erfindung, welche auch unangenehm schmeckende Stoffe oder Stoffgemische enthalten können (vgl. hierzu oben), können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen sowie als Zubereitung mit funktionellen Inhaltsstoffen, als Nahrungsergänzungsmittel oder als bilanzierte Diäten.

Der Mundpflege dienende Zubereitungen im Sinne der Erfindung sind insbesondere Mund- und/oder Zahnpflegemittel wie Zahnpasten, Zahngele, Zahnpulver, Mundwässer, Kaugummis und andere Mundpflegemittel.

Zahnpflegemittel (als Basis für die Mundpflege dienende Zubereitungen), umfassen im Allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite®, Süßstoffe, wie z.B. Saccharin, andere Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Methylendioxyzimtsäure-N,N-diphenylamid, Methylendioxyzimtsäure-N-ethyl-N-phenylamid, Methylendioxyzimtsäure-N-pyridyl-N-phenylamid, Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigenzien.

Kaugummis (als weiteres Beispiel für die Mundpflege dienende Zubereitungen), umfassen im allgemeinen eine Kaugummibase, d.h. eine beim Kauen plastisch werdende Kaumasse, Zucker verschiedener Arten, Zuckeraustauschstoffe, Süßstoffe, Zuckeralkohole, andere Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), die im vorherigen Abschnitt genannten Kühlwirkstoffe, Feuchthaltemittel, Verdicker, Emulgatoren, Aromen und Stabilisatoren oder Geruchskorrigenzien.

Beispiele üblicher Grund-, Hilfs- und Zusatzstoffe für erfindungsgemäße Zubereitungen sind Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose), Zuckeralkohole (z.B. Sorbit), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. Taurin), Peptide, native oder prozessierte Proteine (z.B. Gelatine), Enzyme (z.B. Peptidasen), Nukleinsäuren, Nucleotide, andere als die erfindungsgemäß verwendeten Geschmackskorrigenzien für unangenehme Geschmackseindrücke (z. B. Hesperetin, Phloretin oder andere gemäß US 2008/0227867 zu verwendende Hydroxychalkonderivate sowie gegebenenfalls die dort beschriebenen Lactone), Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure, Milchsäure), zusätzliche Bitterstoffe (z.B. Chinin, Coffein, Limonin, Amarogentin, Humolone, Lupolone, Catechine, Tannine), Süßstoffe (z.B. Saccharin, Cyclamat, Aspartam, Neotam, Stevioside, Rebaudioside, Acesulfam K, Neohesperidindihydrochalkon, Thaumatin, Superaspartam), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, synthetische, natürliche oder naturidentische Aromastoffe oder Riechstoffe sowie Geruchskorrigenzien.

Die vorliegende Erfindung betrifft auch ein Verfahren zum Herstellen einer erfindungsgemäßen Zubereitung, insbesondere einer solchen wie hierin als bevorzugt beschrieben, umfassend folgende Schritte:
i) Bereitstellen
   (A) einer erfindungsgemäßen Mischung wie hierin beschrieben,
      vorzugsweise wobei
      die Gesamtmenge an Verbindung(en) der Formel (I) und/oder Salz(en) davon so ausgewählt ist, dass die Gesamtmenge in der herzustellenden Zubereitung, bezogen auf das Gesamtgewicht der Zubereitung, im Bereich von 0,1 bis 1.000 mg/kg liegt, vorzugsweise im Bereich von 1 bis 1.000 mg/kg, vorzugsweise im Bereich von 1 bis 750 mg/kg, besonders bevorzugt im Bereich von 5 bis 500 mg/kg,
      oder
   (C) eine erfindungsgemäße Aromakomposition wie hierin beschrieben,
      vorzugsweise wobei
      die Gesamtmenge an Verbindung(en) der Formel (I) und/oder Salz(en) so ausgewählt ist, dass die Gesamtmenge in der herzustellenden Zubereitung, bezogen auf das Gesamtgewicht der Zubereitung, im Bereich von 0,1 bis 1.000 mg/kg liegt, vorzugsweise im Bereich von 1 bis 1.000 mg/kg, vorzugsweise im Bereich von 1 bis 750 mg/kg, besonders bevorzugt im Bereich von 5 bis 500 mg/kg,
ii) Bereitstellen eines oder mehrerer weiterer Bestandteile der herzustellenden Zubereitung, und
iii) Inkontaktbringen oder Mischen der in Schritt ii) bereitgestellten weiteren Bestandteile mit dem/den in Schritt i) bereitgestellten Bestandteil(en), vorzugsweise in einer sensorisch wirksamen Menge.

Die beschriebenen erfindungsgemäßen Zubereitungen werden vorzugsweise hergestellt, indem ein erfindungsgemäß einzusetzender Ester der Homovanillinsäure als Substanz, als Lösung oder in Form einer Aromakomposition in eine der Ernährung, der Mundpflege oder dem Genuss dienende oder orale pharmazeutische Basis-Zubereitung eingearbeitet werden. Vorteilhafterweise können als Lösung vorliegende erfindungsgemäße Zubereitungen auch z.B. durch Sprühtrocknung in eine feste Zubereitung überführt werden.

Im Folgenden ist exemplarisch die Herstellung von erfindungsgemäßen Aromakompositionen (hier: enthaltend Ethylhomovanillat (**17**)) beschrieben, indem man
i) 1 Äquivalent Ascorbinsäure oder eines ihrer physiologisch akzeptablen Salze mit
ii) 1 Äquivalent Vanillylalkohol
iii) in einer (50/50; v/v) Mischung aus Wasser und einem Vertreter aus der Stoffgruppe der Alkohole, besonders bevorzugt sind Ethanol, Propanol, Butanol, Pentanol, Hexanol, Isopropylalkohol
iv) bei einer Temperatur von 100-150 °C (ggf. unter Druck)
v) für eine Zeit von 4-6 h
zur Reaktion bringt. Diese Aromazubereitung (primäres Reaktionsgemisch) enthält vorzugsweise 1.000-200.000 ppm, bevorzugt 10.000-100.000 ppm Ethylhomovanillat (**17**) und kann als solche oder gegebenenfalls weiter aufgereinigt in Mischung mit anderen Aromastoffen und Trägern als Aromakomposition verwendet werden. Diese Aromakompositionen enthalten vorzugsweise 100-100.000 mg/kg, bevorzugt 250-40.000 mg/kg besonders bevorzugt 250-15.000 mg/kg Ethylhomovanillat (**17**) oder physiologisch akzeptable Salze, insbesondere seine Natrium-, Kalium-, Ammonium-, Calcium-, Magnesium- oder Zinksalze, wobei die Konzentration von Ethylhomovanillat (**17**) bzw. Mischungen von Ethylhomovanillat (**17**) mit den entsprechenden Salzen im Endlebensmittel vorzugsweise 0,1-1.000 mg/kg, bevorzugt 1-750 mg/kg besonders bevorzugt 5-500 mg/kg entspricht.

Ascorbinsäure und Vanillylalkohol kommen jeweils in der Natur in Nahrungsmitteln vor und sind als Lebensmittelzusatzstoffe bzw. Aromastoffe zugelassen; besonders vorteilhaft ist daher der Einsatz von isolierter oder natürlich gewonnener Ascorbinsäure sowie von isoliertem oder natürlich gewonnenem Vanillylalkohol, wobei diese auch in Form von nicht vollständig aufgereinigten Extrakten oder Fraktionen eingesetzt werden können. Vanillylalkohol kommt z.B. in Bier (Flavor-Base, 9th Edition, Leffingwell & Associates, 2013) oder der Sitka-Fichte (*Picea sitchensis,* P. J. Kohlbrenner, C. Schuerch, Benzene-Alcohol-Soluble Extractives of Sitka Spruce, J. Org. Chem. 1959, 24(2), 166-172) vor.

Dabei sind die vorstehend beschriebenen erfindungsgemäßen Aromazubereitungen dadurch charakterisiert, dass sie neben Ethylhomovanillat (**17**) noch mindestens einen weiteren Stoff aus der folgenden Tab. 1 enthalten können (entsprechendes gilt für die hierin beschriebenen erfindungsgemäßen Aromakompositionen):

**Tab. 1:**

| **Nr.** | **Retentionszeit [min]*** | **Molare Masse m/z (Gef.: ESI+)** | **IPUAC Name** | **Struktur** |
|---|---|---|---|---|
| 1 | 2.88 | gef. 227.0546 ber. 226.047189 für C₁₀H₁₀O₆ | 3-(1,2-Dihydroxyethyl)-1,3-dihydroiso-benzofuran-1,4,5-triol | 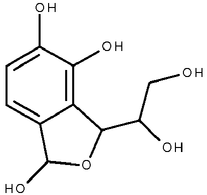 |
| 2 | 3.73 | gef. 313.0921 ber. 312.083969 für C₁₄H₁₆O₈ | 6-[(3-Hydroperoxy-4-hydroxyphenyl)-methyl]-3,6,6a-trihydroxy-3,3a-dihydro-2H-furo[3,2-b]furan-5-on* | 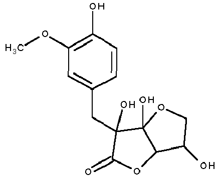 |
| 3 | 3.98 | gef. 313.0920 ber. 312.083969 für C₁₄H₁₆O₈ | 2-Hydroxy-3-(4-hydroxy-3-methoxy-phenyl)-2-(3-hydroxy-2-oxo-tetrahydrofuran-3-yl)propansäure | 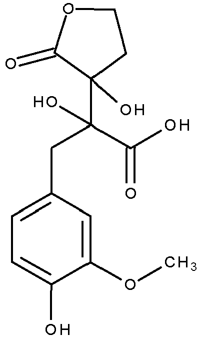 |
| 4 | 4.66 | gef. 251.0924 ber. 250.083575 für C₁₃H₁₄O₅ | 2-Hydroxy-3-(4-hydroxy-3-methoxy-phenyl)-4-(hydroxymethyl)cyclopent-2-en-1-on* | 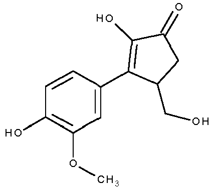 |
| 5 | 6.12 | gef. 387.1449 ber. 386.136004 für C₂₁H₂₂O₇ | 2-Hydroxy-3-[4-hydroxy-2-[(4-hydroxy-3-methoxy-phenyl)methyl]-5-methoxy-phenyl]-4-(hydroxymethyl)cyclopent-2-en-1-on | 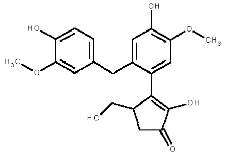 |
| 6 | 6.33 | gef. 251.0925 brr. 250.083575 für C₁₃H₁₄O₅ | 2-[(4-Hydroxy-3-methoxy-phenyl)-methylene]-5-(hydroxymethyl)-tetrahydrofuran-3-on | 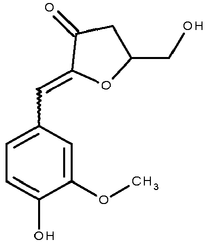 |
| 7 | 6.34 | gef. 251.0921 ber. 250.083575 für C₁₃H₁₄O₅ | 5-Hydroxy-6-[(4-hydroxy-3-methoxy-phenyl)methyl]-2,3-dihydropyran-4-on | 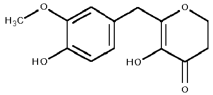 |
| 8 | 6.98 | gef. 341.1251 ber. 340.115269 für C₁₆H₂₀O₈ | Ethyl-2-hydroxy-3-(4-hydroxy-3-methoxy-phenyl)-2-(3-hydroxy-2-oxotetrahydro-furan-3-yl)propanoat | 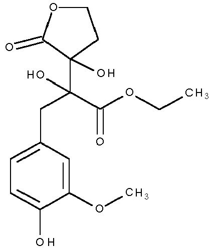 |
| 9 | 7.68 | gef. 233.0842 ber. 232.073010 für C₁₃H₁₂O₄ | 1-(2-Furyl)-2-(4-hydroxy-3-methoxy-phenyl)ethanon | 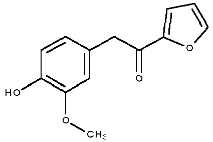 |
| 10 | 7.71 | gef. 387.1457 ber. 386.136004 für C₂₁H₂₂O₇ | 2-Hydroxy-3-[4-hydroxy-3-[(4-hydroxy-3-methoxyphenyl)methyl]-5-methoxyphenyl]-4-(hydroxymethyl)cyclopent-2-en-1-on | 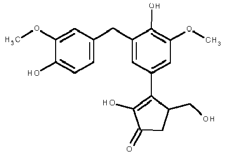 |
| 11 | 7.93 | gef. 233.0821 ber. 233.073010 für C₁₃H₁₂O₄ | 2-[(4-Hydroxy-3-methoxyphenyl)methylene]-5-methylfuran-3-on | 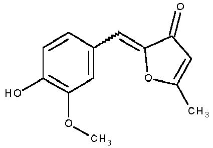 |
| 12 | 8.22 | gef. 297.1347 ber. 296.125440 für C₁₅H₂₀O₆ | Ethyl-3-[3-(4-hydroxy-3-methoxy-phenyl)-2-oxopropoxy]propanoat | 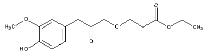 |
| 14 | 8.82 | gef. 465.1606 ber. 464.146569 für C₂₆H₂₄O₈ | 2-[3-(2-Furyl)-1,2-bis(4-hydroxy-3-methoxy-phenyl)-3-oxo-propyl]-5-methyl-furan-3-on | 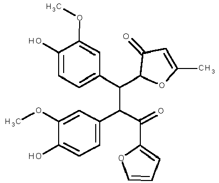 |
| 15 | 9.52 | gef. 369.1346 ber. 368.125440 für C₂₁H₂₀O₆ | 1-(2-Furyl)-2-[4-hydroxy-2-[(4-hydroxy-3-methoxyphenyl)methyl]-5-methoxyphenyl]ethanon | 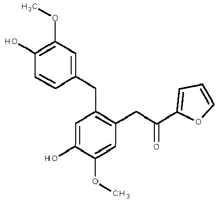 |

| | | | | |
|---|---|---|---|---|
| * s. Preobrazhenskaya, M.N. et al. Tetrahedron 1997, 53, 6971-6976 | | | | |

Die Aufreinigung des primären Reaktionsgemischs kann erfolgen durch eines oder mehrere der folgenden Verfahren:
a) gegebenenfalls Aufkonzentrierung des primären Reaktionsgemischs, vorzugsweise durch ein oder mehrere evaporative oder pervaporative Verfahren,
b) gegebenenfalls Behandlung des (gegebenenfalls in Schritt a) aufkonzentrierten) primären Reaktionsgemischs durch verteilungschromatographische (z.B. Gegenstromverteilungsverfahren wie FCPC, SCCC, Craig-Verfahren) oder adsorptionschromatographische Verfahren mit oder an Adsorbentien, vorzugsweise ausgewählt aus der Gruppe bestehend aus Kieselgel, modifiziertes Kieselgel, Aktivkohle, Zeolith, Bentonit, Kieselgur, Aluminiumoxid, basischer oder saurer oder neutraler, optional makroporöser, lonentauscher, bevorzugt im Batch- oder Säulenverfahren, gegebenenfalls unter Zuhilfenahme weiterer Extraktionsmittel, wodurch eine aufgereinigte Aromakomposition erhalten wird,
c) gegebenenfalls Trocknung der in Schritt b) erhaltenen aufgereinigten Aromakomposition, vorzugsweise durch ein evaporatives oder pervaporatives Verfahren,
d) gegebenenfalls ein- oder mehrmaliger Wiederholung der Schritte b) und c)
e) gegebenenfalls Mischen der in den vorherigen Schritten erhaltenen aufgereinigte Aromakomposition mit einem geeigneten Verdünnungsmittel oder einem Gemisch zweier oder mehrerer Verdünnungsmittel, bevorzugt ausgewählt aus der Gruppe bestehend aus Ethanol, Isopropanol, 1,2-Propylenglycol, Pflanzenöltriglyceriden, Diacetin, Triacetin und Glycerin, wobei vorzugsweise die Aromakomposition in Form einer Lösung erhalten wird.

Gemäß einer weiteren bevorzugten Ausführungsform werden zur Herstellung erfindungsgemäßer Zubereitungen erfindungsgemäß verwendete Verbindungen der Formel (I) bzw. deren Salze oder eine erfindungsgemäße Aromakomposition, insbesondere das primäre Reaktionsgemisch (wie oben exemplarisch beschrieben) oder die aufgereinigten Aromakompositionen (wie oben exemplarisch beschrieben) und gegebenenfalls andere Bestandteile der erfindungsgemäßen Zubereitung in Form von Emulsionen, in Liposomen, z.B. ausgehend von Phosphatidylcholin, in Microsphären, in Nanosphären oder auch in Kapseln, Granulaten oder Extrudaten aus einer für Lebens- und Genussmittel geeigneten Matrix, z.B. aus Stärke, Stärkederivaten, Cellulose oder Cellulosederivaten (z.B. Hydroxypropylcellulose), anderen Polysacchariden (z.B. Alginat), natürlichen Fetten, natürlichen Wachsen (z.B. Bienenwachs, Carnaubawachs) oder aus Proteinen, z.B. Gelatine, eingearbeitet. In einem bevorzugten Herstellungsverfahren werden die Verbindungen der Formel (I) bzw. deren Salze vor dem Einarbeiten mit einem oder mehreren geeigneten Komplexbildnern, beispielsweise mit Cycloglycanen, z.B. Cyclofructanen, Cyclodextrinen oder Cyclodextrinderivaten, bevorzugt alpha-, beta- und gamma-Cyclodextrin, komplexiert und in dieser komplexierten Form eingesetzt.

Im Folgenden wird die vorliegende Erfindung anhand ausgewählter, konkreter Beispiele näher beschrieben. Die Beispiele dienen nur zur Verdeutlichung der Erfindung, ohne diese einzuschränken bzw. darauf zu beschränken. Sofern nicht anders angegeben, beziehen sich alle Angaben auf das Gewicht.

### Beispiele

### Beispiel 1: Herstellung einer Aromazubereitung (primäres Reaktionsgemisch) enthaltend Ethylhomovanillat (17) durch Reaktion von Ascorbinsäure mit Vanillylalkohol

3 mmol Ascorbinsäure und 3 mmol Vanillylalkohol wurden in 10 mL Wasser/Ethanol (1/1; v/v) gelöst. Die Lösung wurde unter stetigem Rühren in der Mikrowelle (Mars Synthesis, CEM) in 7 min auf 100°C erhitzt. Anschließend wurde das Reaktionsgemisch weitere 6 h bei 100°C unter stetigem Rühren in der Mikrowelle erhitzt. Im unten abgebildeten LC-MS/QTOF-Chromatogramm sind die nach 6 h entstehenden in Tabelle 1 aufgelisteten Substanzen sowie Ethylhomovanillat (EHV, **17**) zu sehen. Ethylhomovanillat (**17**) ist zu 1.2% in dem primären Reaktionsgemisch enthalten.

Die Durchführung der obigen Reaktion mit den aufgeführten Verhältnissen an Ascorbinsäure und Vanillylalkohol mit einer Kochzeit von 4 h führt ebenfalls zur Entstehung von EHV.

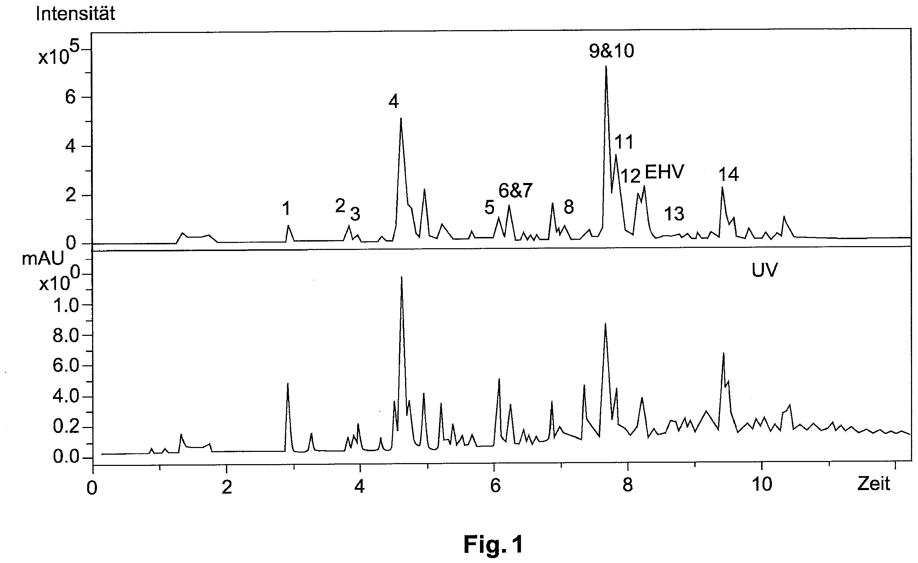

Siehe hierzu Fig. 1 (LC-MS/QTOF Chromatogramm des primäres Reaktionsgemischs nach 6 h bei 100°C; oberes Chromatogramm Massenspur ESI positiv, unteres Chromatogramm UV-VIS summiert; die Nummern stellen die Verbindungen nach Tabelle 1 dar und EHV steht für Ethylhomovanillat (**17**)).

### Beispiel 2: Isolierung von Ethylhomovanillat (17) aus einer Aromazubereitung (primäres Reaktionsgemisch)

Das primäre Reaktionsgemisch wird mittels Mitteldruckchromatographie (MPLC) (Säulenmaterial: Lewatit VP OC 1064; Wasser/Ethanol 3/1; v/v) vorfraktioniert. Anschließend erfolgt eine weitere Auftrennung via präparativer Hochdruckchromatographie (pHPLC) (Säule: Phenomenex Luna C18 5µ 150x21.2 mm; Flussrate 30mL/min; Detektion 210 nm) im isokratischen Modus (63% H₂O, 37% MeOH). Die abschließende Isolierung von Ethylhomovanillat (**17**) wird via semipräparativer Hochdruckchromatographie (sPHPLC) im Gradienten-Modus (Säule: YMC Triart C18 5µ 250x10mm; A: H₂O; B: MeOH; 0min 65% A, 35% B; 25min 40% A, 60% B; 30min 100% B; Flussrate 3 mL/min; Detektion: 250 nm) durchgeführt. Das erhaltene Ethylhomovanillat (**17**) wurde anschließend gefriergetrocknet und in einer Dosierung von 100 ppm auf 5%-ige Zucker-, 0.5%ige Salz-, 500 ppm Coffeinlösung und Wasser verkostet und sensorisch bewertet.

| **Medium** | **Geschmacksbeschreibung** |
|---|---|
| Wasser | scharf, stechend, leicht wärmend |
| 5%-ige Zuckerlösung | scharf, wärmend |
| 0.5%-ige Salzlösung | scharf |

### Beispiel 3: Herstellung und sensorische Evaluierung einer Fraktion (aufgereinigte Aromazubereitung) enthaltend Ethylhomovanillat (17)

Das primäre Reaktionsgemisch wird mittels LC-Taste® (nach WO 2006 111,476) im Gradientenmodus (Hamilton PRP-1 10 µ 250x21.5 mm; A: H₂O, B: EtOH; 0 min 100% A; 25 min 75% A, 25% B; 40 min 100% B; Flussrate: 10 mL/min, Ofentemperatur: 80 °C) in 12 Fraktionen geschnitten, wobei die Fraktionseinteilung anhand der UV Spur bei 210 nm erfolgte. Nach Einengung der Fraktionen auf 0.5 mL am Büchi Syncore bei 40 °C wurde der Rückstand in 10 mL Wasser gelöst. Von diesen Lösungen wurden jeweils 2 mL mit 18 mL 3.33%iger Zuckerlösung vermischt (entspricht einer Enddosierung von 96 mg/kg Ethylhomovanillat (**17**) für Fraktion 10 und 26 mg/kg Ethylhomovanillat für Fraktion 11 in 3%iger Zuckerlösung) und sensorisch bewertet.

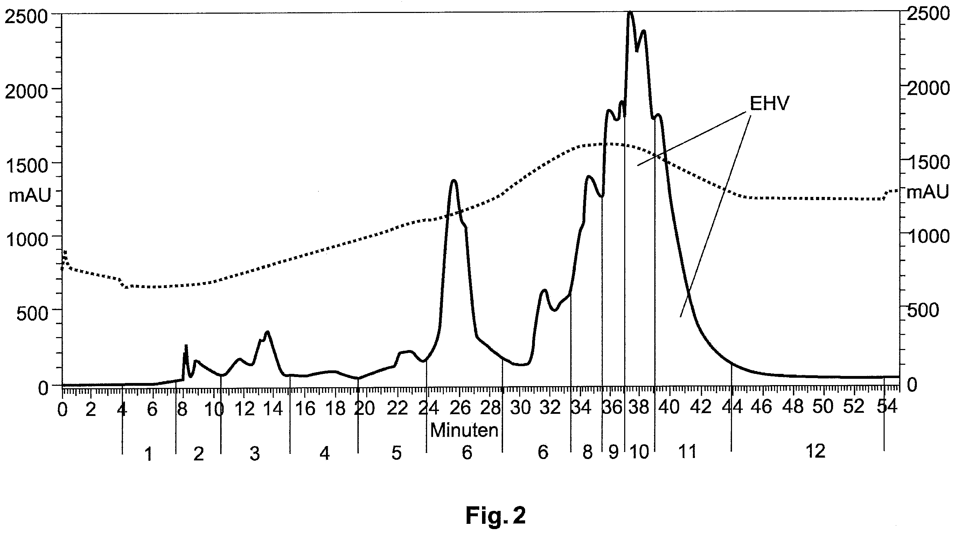

Siehe hierzu Fig. 2 (LC Taste Chromatogramm des primären Reaktionsgemischs; Fraktion 10 und 11 enthalten EHV)

**Tab. 2: Sensorisches Geschmacksprofil der Fraktionen 10 und 11 der LC Taste Trennung (siehe Fig. 2)**

| Fraktion | Geschmack | Intensität |
|---|---|---|
| 10 | wärmend | 5-6 |
| | medizinisch | 4-5 |
| | Paprika | 3-4 |
| | geräucherter Schinken (rauchig) | 3 |
| | scharf | 5 |
| 11 | wärmend | 2-3 |
| | geräucherter Schinken | 4-5 |
| | rauchig | 4 |
| | gegrillt, BBQ | 4 |
| | phenolisch | 3 |
| | Vanille | 2 |

### Beispiel 4: Synthese von Homovanillinsäureestern

### Methode A:

Homovanillinsäure (1.5-3 g) wurde mit dem jeweiligen Alkohol (äquimolar) in Toluol (100 mL) vorgelegt, konz. Schwefelsäure dazugegeben und für 5 h am Wasserabscheider zum Sieden erhitzt. Es wurde einmal mit ges. wäss. NaHCO₃-Lösung, zweimal mit Wasser oder wahlweise ges. wäss. NaCl-Lösung gewaschen und das Lösungsmittel im Vakuum entfernt. Das Produkt wurde nach säulenchromatographischer Reinigung an Kieselgel in ca. 70% Ausbeute erhalten.

### Methode B:

Homovanillinsäure (1.5-3 g) wurde in dem jeweiligen Alkohol (100 mL) und 0.2-0.5 Äquivalenten Schwefelsäure für 7 h bei 90 °C (Heizblocktemperatur) gerührt. Der größte Teil des Alkohols wurde im Vakuum entfernt, ges. wäss. NaHCO₃-Lösung und EtOAc dazugegeben, die org. Phase abgetrennt und die wäss. Phase einmal mit EtOAc extrahiert. Die vereinigten org. Phasen wurden je einmal mit ges. wäss. NaHCO₃- und mit Wasser oder wahlweise ges. wäss. NaCl-Lösung, über NaSO₄ getrocknet und das Lösungsmittel im Vakuum entfernt. Das Produkt wurde nach säulenchromatographischer Reinigung an Kieselgel in 90%-iger bis quantitativer Ausbeute erhalten.

### Isopropyl-2-(4-hydroxy-3-methoxy-phenyl)acetat (7, Methode B)

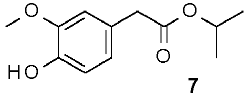

¹H-NMR (400 MHz, CDCl₃): δ = 6.85 (d, *J* = 8.1 Hz, 1H), 6.81 (dd, *J* = 2.0, 0.5 Hz, 1H), 6.76 (ddt, *J* = 8.0, 2.0, 0.6 Hz,1H), 5.60 (s, 1H), 5.01 (hept, *J* = 6.3 Hz, 1H), 3.88 (s, 3H), 3.496 (t, *J* = 0.5 Hz, 2H), 1.23 (d, *J* = 6.3 Hz, 6H).
¹³C-NMR (100 MHz, CDCl₃): *δ* = 171.5, 146.4, 144.7, 126.1, 122.1, 114.3, 111.7, 68.1, 55.9, 41.3, 21.8 (2C).
GCMS: *m*/*z* (%) = 224 [M⁺] (30), 137 (100), 122 (10), 107 (2), 94 (6), 77 (3), 66 (5), 51 (3), 43 (15).

### sec-Butyl-2-(4-hydroxy-3-methoxy-phenyl)acetat (8, Methode B)

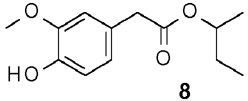

¹H-NMR (400 MHz, CDCl₃): *δ* = 6.85 (d, *J* = 8.1 Hz, 1H), 6.82 (d, *J* = 1.9 Hz, 1H), 6.79-6.74 (m, 1H), 5.57 (s, 1H), 4.92-4.78 (m, 1H), 3.88 (s, 3H), 3.51 (t, *J* = 0.5 Hz, 2H), 1.62-1.46 (m, 2H), 1.19 (d, *J* = 6.3 Hz, 3H), 0.85 (t, *J* = 7.5 Hz, 3H).
¹³C-NMR (100 MHz, CDCl₃): δ = 171.6, 146.4, 144.7, 126.2, 122.1, 114.3, 111.7, 72.7, 55.9, 41.4, 28.8, 19.4, 9.6.
GCMS: *m*/*z* (%) = 238 [M⁺] (30), 137 (100), 122 (8), 107 (2), 94 (5), 77 (2), 66 (3), 57 (20), 51 (2), 41 (8), 29 (8).

### Isobutyl-2-(4-hydroxy-3-methoxy-phenyl)acetate (9, Methode B)

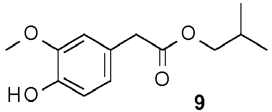

¹H-NMR (400 MHz, CDCl₃): *δ* = 6.85 (dd, *J* = 8.1, 0.3 Hz, 1H), 6.82-6.81 (m, 1H), 6.77 (ddt, *J* = 8.1, 2.0, 0.6 Hz, 1H), 5.60 (s, 1H), 3.87 (d, *J* = 0.3 Hz, 3H), 3.86 (d, *J* = 6.6 Hz, 2H), 3.54 (t, *J* = 0.5 Hz, 2H), 1.91 (dq, *J* = 6.7 Hz, 1H), 0.90 (d, *J* = 6.7 Hz, 6H).
¹³C-NMR (100 MHz, CDCl₃): δ = 172.0, 146.5, 144.7, 126.0, 122.1, 114.3, 111.7, 70.9, 55.9, 41.1, 27.7, 19.0 (2C).
GCMS: *m*/*z* (%) = 238 [M⁺] (30), 182 (5), 137 (100), 122 (9), 107 (2), 94 (6), 77 (2), 66 (3), 57 (11), 51 (2), 41 (8), 29 (7).

### Butyl-2-(4-hydroxy-3-methoxy-phenyl)acetat (19, Methode B)

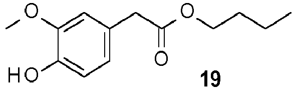

¹H-NMR (400 MHz, CDCl₃): δ = 6.85 (d, *J* = 8.1 Hz, 1H), 6.81 (dd, *J* = 2.0, 0.5 Hz, 1H), 6.76 (ddt, *J* = 8.1, 1.9, 0.6 Hz, 1H), 5.60 (s, 1H), 4.09 (t, *J* = 6.7 Hz, 2H), 3.87 (d, *J* = 0.3 Hz, 3H), 3.53 (t, *J* = 0.5 Hz, 2H), 1.67-1.55 (m, 2H), 1.42-1.29 (m, 2H), 0.91 (t, *J* = 7.4 Hz, 3H).
¹³C-NMR (100 MHz, CDCl₃): *δ* = 172.0, 146.5, 144.7, 126.0, 122.1, 114.3, 111.7, 64.7, 55.9, 41.1, 30.6, 19.1, 13.7.
GCMS: *m*/*z* (%) = 238 [M⁺] (27), 182 (2), 137 (100), 122 (9), 107 (2), 94 (5), 77 (2), 66 (2), 57 (4), 41 (5), 29 (8).

### Propyl-2-(4-hydroxy-3-methoxy-phenyl)acetat (18, Methode B)

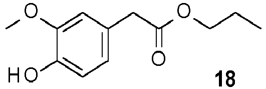

¹H-NMR (400 MHz, CDCl₃): *δ* = 6.85 (d, *J* = 8.0 Hz, 1H), 6.81 (d, *J* = 2.0 Hz, 1H), 6.76 (dd, *J* = 8.1, 2.0 Hz, 1H), 5.61-5.58 (m, 1H), 4.05 (t, *J* = 6.7 Hz, 2H), 3.88 (s, 3H), 3.54 (s, 2H), 1.71-1.52 (m, 2H), 0.91 (t, *J* = 7.4 Hz, 3H).
¹³C-NMR (100 MHz, CDCl₃): *δ* = 172.0, 146.5, 144.7, 126.0, 122.2, 144.3, 111.7, 66.4, 55.9, 41.1, 22.0, 10.4.
GCMS: *m*/*z* (%) = 224 [M⁺] (30), 137 (100), 122 (10), 107 (2), 94 (8), 77 (2), 66 (3), 51 (2) 43 (8).

### Ethyl-2-(4-hydroxy-3-methoxy-phenyl)acetat (17, Methode B)

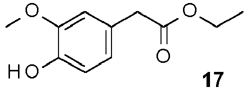

¹H-NMR (400 MHz, CDCl₃): δ = 6.85 (d, *J* = 8.05 Hz, 1H), 6.81 (d, *J* = 1.94 Hz, 1H), 6.76 (ddd, J *=* 8.01, 1.99, 0.50 Hz, 1H), 5.61 (d, *J* = 0.36 Hz, 1H), 4.15 (q, *J* = 7.13 Hz, 2H), 3.88 (s, 3H), 3.53 (d, *J =* 0.57 Hz, 2H), 1.25 (t, *J* = 7.13 Hz, 1H).
¹³C-NMR (100 MHz, CDCl₃): *δ* = 172.0, 146.5, 144.7, 125.9, 122.1, 114.4, 111.7, 60.8, 55.9, 41.0, 14.2.
GCMS: *m*/*z* (%) = 210 [M⁺] (30), 137 (100), 122 (11), 107 (2), 94 (8), 77 (2), 66 (3), 51 (3), 39 (3), 29 (8).

### Hexyl-2-(4-hydroxy-3-methoxy-phenyl)acetat (21, Methode A)

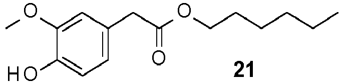

¹H-NMR (400 MHz, CDCl₃): *δ* = 6.85 (dd, *J* = 8.1, 0.3 Hz, 1H), 6.81 (d, *J* = 2.0 Hz, 1H), 6.76 (ddq, *J* = 8.1, 2.0, 0.5 Hz, 1H), 5.58 (s, 1H), 4.08 (t, *J* = 6.7 Hz, 2H), 3.88 (s, 3H), 3.53 (s, 2H), 1.65-1.56 (m, 2H), 1.36-1.20 (m, 6H), 0.87 (t, *J* = 7.0 Hz, 3H).
¹³C-NMR (100 MHz, CDCl₃): *δ* = 172.0, 146.5, 144.7, 126.0, 122.1, 114.3, 111.7, 65.0, 55.9, 41.1, 31.4, 28.6, 25.5, 22.5, 14.0.
GCMS: *m*/*z* (%) = 266 [M⁺] (30), 182 (8), 137 (100), 122 (8), 107 (2), 94 (4), 77 (2), 66 (2), 55 (3), 43 (13).

### 3-Phenylpropyl-2-(4-hydroxy-3-methoxy-phenyl)acetat (22, Methode A)

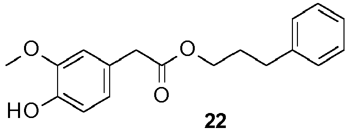

¹H NMR (600 MHz, CDCl₃): δ = 7.29 - 7.24 (m, 2H), 7.20 - 7.16 (m, 1H), 7.14 - 7.10 (m, 2H), 6.87 (d, J = 8.0 Hz, 1H), 6.81 (d, J = 2.0 Hz, 1H), 6.78 (dd, J = 8.1, 2.0 Hz, 1H), 5.57 (s, 1H), 4.10 (t, J = 6.5 Hz, 2H), 3.88 (s, 3H), 3.54 (s, 2H), 2.64 (dd, J = 8.5, 6.8 Hz, 2H), 1.98 - 1.90 (m, 2H).
¹³C NMR (151 MHz, CDCl₃): δ = 171.91, 146.46, 144.75, 141.09, 128.42, 128.37, 126.00, 125.88, 122.13, 114.36, 111.68, 64.10, 55.90, 41.08, 32.06, 30.16.
GCMS: m/z (%) = 300 [M+] (28), 182 (62), 137 (100), 122 (16), 118 (20), 91 (34), 77 (6), 65 (6), 51 (4), 28 (4).

### 4-Phenylbutyl-2-(4-hydroxy-3-methoxy-phenyl)acetat (23, Methode A)

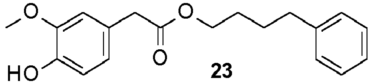

¹H NMR (600 MHz, CDCl₃): δ = 7.29 - 7.25 (m, 2H), 7.20 - 7.16 (m, 1H), 7.15 - 7.11 (m, 2H), 6.85 (d, *J* = 8.1 Hz, 1H), 6.79 (d, *J* = 2.0 Hz, 1H), 6.75 (dd, *J* = 8.0, 2.0 Hz, 1H), 5.60 (s, 1H), 4.10 (t, *J* = 6.1 Hz, 2H), 3.83 (s, 3H), 3.52 (s, 2H), 2.60 (t, *J* = 7.1 Hz, 2H), 1.69 - 1.60 (m, 4H).
¹³C NMR (151 MHz, CDCl₃): δ = 171.97, 146.45, 144.72, 141.97, 128.33, 128.32, 125.86, 125.81, 122.09, 114.35, 111.67, 64.67, 55.84, 41.06, 35.38, 28.17, 27.68.
GCMS: m/z (%) = 314 [M+] (48), 182 (36), 137 (100), 122 (10), 104 (20), 91 (44), 65 (4), 51 (2).

### [(Z)-Hex-3-enyl]-2-(4-hydroxy-3-methoxy-phenyl)acetat (6, Methode A)

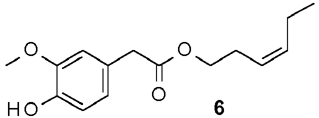

¹H-NMR (400 MHz, CDCl₃): δ = 6.85 (d, *J* = 8.1 Hz, 1H), 6.80 (d, *J* = 2.0 Hz, 1H), 6.76 (dd, *J* = 8.0, 2.0 Hz, 1H), 5.58 (s, 1H), 5.49 (ddt, *J* = 10.9, 7.3, 1.6 Hz, 1H), 5.29 (ddt, *J* = 10.7, 7.3, 1.5 Hz, 1H), 4.08 (t, *J* = 7.0 Hz, 2H), 3.88 (s, 3H), 3.53 (s, 2H), 2.43-2.32 (m, 2H), 2.03 (pd, *J* = 7.5, 1.6 Hz, 2H), 0.96 (t, *J* = 7.5 Hz, 3H).
¹³C-NMR (100 MHz, CDCl₃): *δ* = 171.9, 146.5, 144.7, 134.6, 125.8, 123.6, 122.1, 114.3, 111.7, 64.4, 55.9, 41.0, 26.7, 20.6, 14.2.
GCMS: *m*/*z* (%) = 264 [M⁺] (30), 182 (55), 137 (100), 122 (15), 94 (10), 82 (8), 67 (15), 55 (20), 41 (15).

### 2-Methylbutyl-2-(4-hydroxy-3-methoxy-phenyl)acetat (12, Methode B)

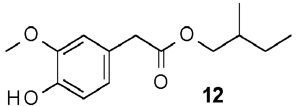

¹H-NMR (400 MHz, CDCl₃): *δ* = 6.86 (d, *J* = 8.1 Hz, 1H), 6.81 (d, *J* = 2.0 Hz, 1H), 6.77 (dd, *J* = 8.0, 2.0 Hz, 1H), 5.55 (s, 1H), 3.97 (dd, *J* = 10.7, 6.0 Hz, 1H), 3.90 (dd, *J* = 10.8, 6.7 Hz, 1H), 3.88 (s, 3H), 3.54 (s, 2H), 1.69 (dddd, *J* = 12.4, 7.8, 6.8, 5.8 Hz, 1H), 1.38 (dtd, *J* = 13.1, 7.5, 5.6 Hz, 1H), 1.23-1.08 (m, 1H), 0.88 (d, *J* = 6.8 Hz, 3H), 0.88 (t, *J* = 7.5 Hz, 3H).
¹³C-NMR (100 MHz, CDCl₃): *δ* = 172.0, 146.4, 144.7, 126.0, 122.1, 114.3, 111.7, 69.4, 55.9, 41.1, 34.1, 26.0, 16.3, 11.2.
GCMS: *m*/*z* (%) = 252 [M+] (30), 182 (10), 137 (100), 122 (8), 94 (7), 71 (5), 55 (4), 43 (18), 29 (10).

### 2-Phenylethyl-2-(4-hydroxy-3-methoxy-phenyl)acetat (1, Methode A)

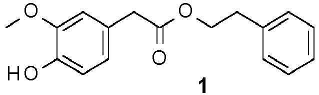

¹H-NMR (400 MHz, CDCl₃): *δ* = 7.29-7.24 (m, 2H), 7.24-7.19 (m, 1H), 7.16 - 7.12 (m, 2H), 6.85 (d, *J* = 7.9 Hz, 1H), 6.74 (d, *J* = 1.9 Hz, 1H), 6.72 (dd, *J* = 8.0, 1.9 Hz, 1H), 5.57 (s, 1H), 4.30 (t, *J* = 6.9 Hz, 2H), 3.84 (s, 3H), 3.51 (s, 2H), 2.91 (t, *J* = 6.9 Hz, 2H).
¹³C-NMR (100 MHz, CDCl₃): *δ* = 171.8, 146.4, 144.7, 137.7, 128.9 (2C), 128.4 (2C), 126.5, 125.7, 122.2, 114.3, 111.7, 65.3, 55.87, 41.1, 35.0.
GCMS: *m*/*z* (%) = 286 [M⁺] (30), 182 (48), 137 (100), 122 (12), 105 (30), 94 (11), 77 (12), 65 (8), 51 (7), 39 (5).

### Pentyl-2-(4-hydroxy-3-methoxy-phenyl)acetat (20, Methode A)

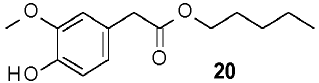

¹H-NMR (400 MHz, CDCl₃): *δ* = 6.85 (d, *J* = 8.0 Hz, 1H), 6.81 (d, *J* = 2.0 Hz, 1H), 6.76 (dd, *J* = 8.1, 2.0 Hz, 1H), 5.58 (s, 1H), 4.08 (t, *J* = 6.7 Hz, 2H), 3.88 (s, 3H), 3.53 (s, 2H), 1.61 (q, *J* = 6.7 Hz, 2H), 1.37-1.24 (m, 4H), 0.94-0.83 (m, 3H).
¹³C-NMR (100 MHz, CDCl₃): δ = 172.0, 146.5, 144.7, 126.0, 122.1, 114.3, 111.7, 65.0, 55.9, 41.1, 28.3, 28.0, 22.3, 14.0.
GCMS: *mlz* (%) = 252 [M⁺] (28), 182 (5), 137 (100), 122 (10), 94 (5), 66 (3), 43 (13), 29 (4).

### Heptyl-2-(4-hydroxy-3-methoxy-phenyl)acetat (14, Methode A)

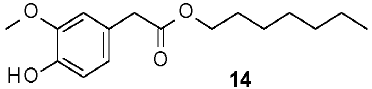

¹H-NMR (400 MHz, CDCl₃): *δ* = 6.85 (dd, *J* = 8.1, 1.1 Hz, 1H), 6.81 (t, *J* = 1.4 Hz, 1H), 6.76 (dt, *J* = 8.2, 1.4 Hz, 1H), 5.59 (d, *J* = 1.1 Hz, 1H), 4.08 (td, *J* = 6.8, 1.1 Hz, 2H), 3.88 (d, *J* = 1.2 Hz, 3H), 3.53 (s, 2H), 1.68-1.55 (m, 2H), 1.28 (td, *J* = 9.8, 9.3, 4.0 Hz, 8H), 0.94-0.82 (m, 3H).
¹³C-NMR (100 MHz, CDCl₃): *δ* = 172.0, 146.5, 144.7, 126.0, 122.1, 114.3, 111.7, 65.0, 55.9, 41.1, 31.7, 28.9, 28.6, 25.8, 22.6, 14.1.
GCMS: *mlz* (%) = 280 [M⁺] (34), 182 (12), 137 (100), 122 (8), 94 (5), 57 (11), 41 (8).

Alternativ können Homovanillinsäureester auch durch Umesterung erhalten werden, wie an Substanz **2** beispielhaft gezeigt:

### [(E)-Cinnamyl]-2-(4-hydroxy-3-methoxy-phenyl)acetat (2)

Ethylhomovanillat (5 g) wurde mit Zimtalkohol (7.5 g) und Natriummethylat-Lösung 25% (0.52 g) auf 150-170 °C erhitzt, ab ca. 130 °C Vakuum angelegt und für 1-3 h MeOH/EtOH aus der Reaktionsmischung heraus destilliert. Es wurde mit MTBE verdünnt, die org. Phase je einmal mit ges. wäss. NH₄Cl-Lösung und Wasser gewaschen und das Lösungsmittel im Vakuum entfernt. Überschüssiger Zimtalkohol wurde anschließend durch Destillation entfernt und das Produkt nach säulenchromatographischer Reinigung an Kieselgel oder nach fraktionierter Destillation in 40-50% Ausbeute erhalten.

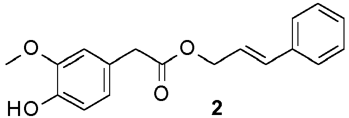

¹H NMR (400 MHz, CDCl₃): δ = 7.38 - 7.35 (m, 2H), 7.35 - 7.29 (m, 2H), 7.29 - 7.23 (m, 1H), 6.87 (d, *J* = 8.0 Hz, 1H), 6.82 (d, *J* = 2.0 Hz, 1H), 6.79 (dd, *J* = 8.0, 2.0 Hz, 1H), 6.60 (dt, *J* = 15.9, 1.5 Hz, 1H), 6.27 (dt, *J* = 15.9, 6.4 Hz, 1H), 5.56 (s, 1H), 4.75 (dd, *J* = 6.4, 1.4 Hz, 2H), 3.86 (s, 3H), 3.59 (s, 2H).
¹³C NMR (100 MHz, CDCl₃): δ = 171.64, 146.48, 144.80, 136.15, 134.17, 128.60, 128.08, 126.58, 125.69, 123.02, 122.19, 114.38, 111.71, 65.34, 55.89, 40.99.
GCMS: m/z (%) = 298 [M+] (12), 137 (100), 122 (8), 117 (68), 94 (8), 91 (12), 77 (4), 65 (4), 51 (4), 39 (6).

### Beispiel 5: Verkostung von Homovanillinsäureestern

Die zu verkostende Substanz wurde in Ethanol gelöst und die ethanolische Lösung dann mit 5%iger Zuckerlosung verdünnt (Endkonzentration: 25 ppm). Zur Verkostung wurde von 4 Prüfern der Mundraum jeweils mit ca. 5 mL der Zuckerlösung gespült und die Lösung wieder ausgespuckt. Die Schärfe wurde auf einer Skala 1 (sehr schwach) - 9 (sehr stark) eingeschätzt und das Profil bewertet.
a) Profil Hexyl-2-(4-hydroxy-3-methoxy-phenyl)acetat (**21**): Deutlich scharf, leicht verzögerter Effekt, wärmend, relativ schnelle Abnahme der Schärfe; Einschätzung der Schärfe 9.
b) Profil Butyl-2-(4-hydroxy-3-methoxy-phenyl)acetat (**19**): Unmittelbare Schärfe, schnelle Abnahme; Einschätzung der Schärfe: 7.
c) Profil Isobutyl-2-(4-hydroxy-3-methoxy-phenyl)acetat (**9**): Schnelles Einsetzen der Schärfe, wärmend, etwas beißende Schärfe; Einschätzung der Schärfe: 4-5.
d) Profil Propyl-2-(4-hydroxy-3-methoxy-phenyl)acetat (**18**): Schwache und verzögerte Schärfe, sich aufbauend wärmend, Tingling; Einschätzung der Schärfe: 4-5.
e) Profil Ethyl-2-(4-hydroxy-3-methoxy-phenyl)acetat (**17**): Etwas wärmend, etwas scharf; Einschätzung der Schärfe: 1-2.
f) Profil Isopropyl-2-(4-hydroxy-3-methoxy-phenyl)acetat (**7**): Spätes Einsetzen, Tingling, wärmend, scharf, etwas verzögert, nicht langlebig, angenehm; Einschätzung der Schärfe: 3.
g) Profil sec-Butyl-2-(4-hydroxy-3-methoxy-phenyl)acetat (**8**): sehr langsames Einsetzen, Tingling, hauptsächlich wärmend, Schärfe sich aufbauend, Einschätzung der Schärfe: 3.
h) Profil Octyl-2-(4-hydroxy-3-methoxy-phenyl)acetat (nicht erfindungsgemäß): Schärfe setzt langsam ein, spät, verzögert, fettig-fruchtige Nebennote, brennend; Einschätzung der Schärfe: 7.
i) Profil Decyl-2-(4-hydroxy-3-methoxy-phenyl)acetat (nicht erfindungsgemäß): Schwache Schärfe, verzögert, Birnen-artig und fettig-fruchtige Nebennote, brennend mehr im Hals als auf der Zunge; Einschätzung der Schärfe: 2.
k) 3-Phenylpropyl-2-(4-hydroxy-3-methoxy-phenyl)acetat (**22**):2 ppm*: Verzögerter Effekt, scharf, wärmend, leicht prickelnd; Einschätzung der Schärfe: 5.
l) 4-Phenylbutyl-2-(4-hydroxy-3-methoxy-phenyl)acetat (**23**):2,5 ppm*: Verzögerter Effekt, Schärfe, verzögert wärmend, brennend, leicht tingling; Einschätzung der Schärfe: 4-5.
m) [(E)-Cinnamyl]-2-(4-hydroxy-3-methoxy-phenyl)acetat (**2**):1,5 ppm*: Verzögerter Effekt, sehr scharf, etwas wärmend, etwas mundwässernd; Einschätzung der Schärfe: 5-6.
n) Heptyl-2-(4-hydroxy-3-methoxy-phenyl)acetat (**14**):1,5 ppm*: Leicht verzögerter Effekt, brennend, scharf, wärmend, Einschätzung der Schärfe: 3.

*Aufgrund starker Schärfe wurden diese Verbindungen lediglich bei Endkonzentrationen von 1.5-2.5 ppm verkostet.

### Beispiel 6: Isointensität von Homovanillinsäureestern im Vergleich zu Nonivamid und einem Capsicum-Extrakt

Die zu verkostende Substanz wurde in Ethanol gelöst und die ethanolische Lösung dann mit 5%iger Zuckerlösung verdünnt (Endkonzentration: 10 ppm). Zum Vergleich wurden Capsicum-Extrakt mit 1.000.000 SHU (0.3-10 ppm) und Nonivamid (0.1-1 ppm) in 5%iger Zuckerlösung in aufsteigender Konzentration vorbereitet. Zur Verkostung wurde von 4 Prüfern der Mundraum jeweils mit ca. 5 mL der Verkostungslösung gespült und die Lösung wieder ausgespuckt und gegen die Referenzreihen bewertet.

Die Schärfe von 10 ppm Hexyl-2-(4-hydroxy-3-methoxy-phenyl)acetat (**21**) ist vergleichbar mit der von 0.5 ppm Nonivamid.

Die Schärfe von 10 ppm Butyl-2-(4-hydroxy-3-methoxy-phenyl)acetat (**19**) ist vergleichbar mit der von 0.3 ppm Nonivamid.

Die Schärfe von 10 ppm Butyl-2-(4-hydroxy-3-methoxy-phenyl)acetat (**19**) ist vergleichbar mit der von 4.5 ppm Capsicum-Extrakt 1.000.000 SHU.

Die Schärfe von 10 ppm Hexyl-2-(4-hydroxy-3-methoxy-phenyl)acetat (**21**) ist vergleichbar mit der von 8.5 ppm Capsicum-Extrakt 1.000.000 SHU.

### Beispiel 7: Schwellenwerte von Homovanillinsäureestern

Die Schwellenwerte wurden nach der Methode ASTM E 679 - 91 ("Standard Practice for Determination of Odor and Taste Thresholds By a Forced-Choice Ascending Concentration Series Method of Limits1 ") bestimmt. Es handelt sich um die jeweilige Flavor Reizschwelle auf Vittel® Wasser.

Zum Beispiel der Schwellenwert von Hexyl-2-(4-hydroxy-3-methoxy-phenyl)acetat (**21**) in Wasser liegt bei 1.7 ppm (1700 ppb).

Zum Beispiel der Schwellenwert von Ethyl-2-(4-hydroxy-3-methoxy-phenyl)acetat (**17**) in Wasser liegt bei 29.5 ppm (29460 ppb).

Zum Beispiel der Schwellenwert von Butyl-2-(4-hydroxy-3-methoxy-phenyl)acetat (**19**) in Wasser liegt bei 3.5 ppm (3540 ppb).

### Beispiel 8: Schärfeprofil von Butyl-2-(4-hydroxy-3-methoxy-phenyl)acetat (19) im Vergleich zu Nonivamid

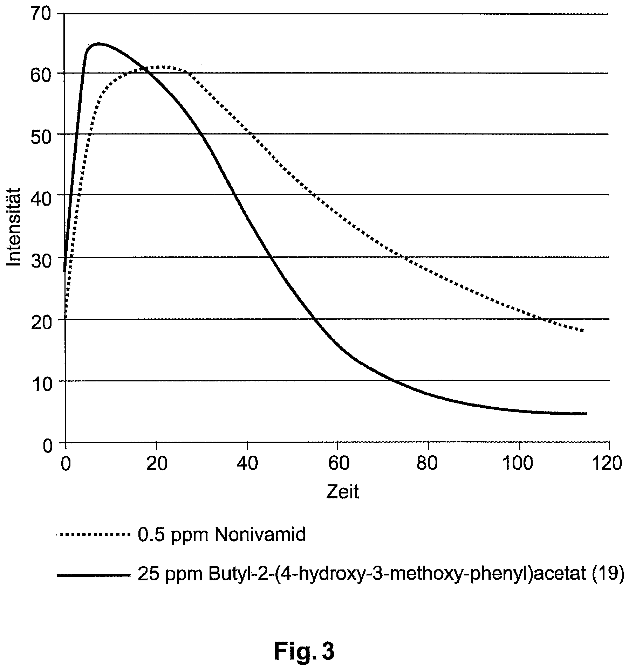

Zur Erstellung eines Zeit-Intensitäts-Profils wurde der Mundraum von geschulten Panelisten (n = 8-10) mit einem Schluck einer Probenlösung (5 mL einer 5% Zuckerlösung) gespült. Dann wurde die Intensität der Eigenschaft Schärfe in definierten Zeitabständen anhand einer Skala ohne feste Einteilung bewertet. Die zwei Verkostungslösungen (Butyl-2-(4-hydroxy-3-methoxy-phenyl)acetat (**19**) mit 25 ppm und Nonivamid mit 0.5 ppm der entsprechenden Substanz wurden dabei codiert und in gemischter Reihung platziert. Zwischen den beiden Verkostungsproben mit Brot und Wasser wieder neutralisiert und mit 5 mL 5%ige Zuckerlösung vorgespült. Die Daten wurden analysiert und graphisch in einer Zeit-Intensitätskurve wiedergegeben (s. Fig. 3 (Schärfeprofil Butyl-2-(4-hydroxy-3-methoxyphenyl)acetat (**19**) im Vergleich zu Nonivamid)).

Deutlich zu erkennen ist das schnellere Einsetzen der Schärfe von Butyl-2-(4-hydroxy-3-methoxyphenyl)acetat (**19**) gegenüber Nonivamid bei ähnlicher Gesamtintensität sowie die deutlich schnellere Abnahme der Schärfe.

### Beispiel 9: Kombination von Homovanillinsäureestern mit Paradieskörnerextrakt, Nonivamid und einem Capsicum-Extrakt

Es wurden verschiedene Kombinationen von Ethyl-2-(4-hydroxy-3-methoxy-phenyl)acetat (**17**) und Butyl-2-(4-hydroxy-3-methoxy-phenyl)acetat (**19**) mit Paradieskörnerextrakt (PN 300953, Symrise), Nonivamid und Capsicum-Extrakt 1.000.000 SHU sensorisch bewertet. Die Verkostungslösungen aus einem Homovanillinsäureester in ansteigender Konzentration (ppm) in Kombination mit einer trigeminalen Substanz bestimmter Konzentration auf Basis einer 5%igen Zuckerlösung (Tab. 3) wurden von 3 Prüfern bewertet. Hierfür wurde der Mundraum jeweils mit ca. 5 mL der jeweiligen Verkostungslösung gespült und die Lösung wieder ausgespuckt.

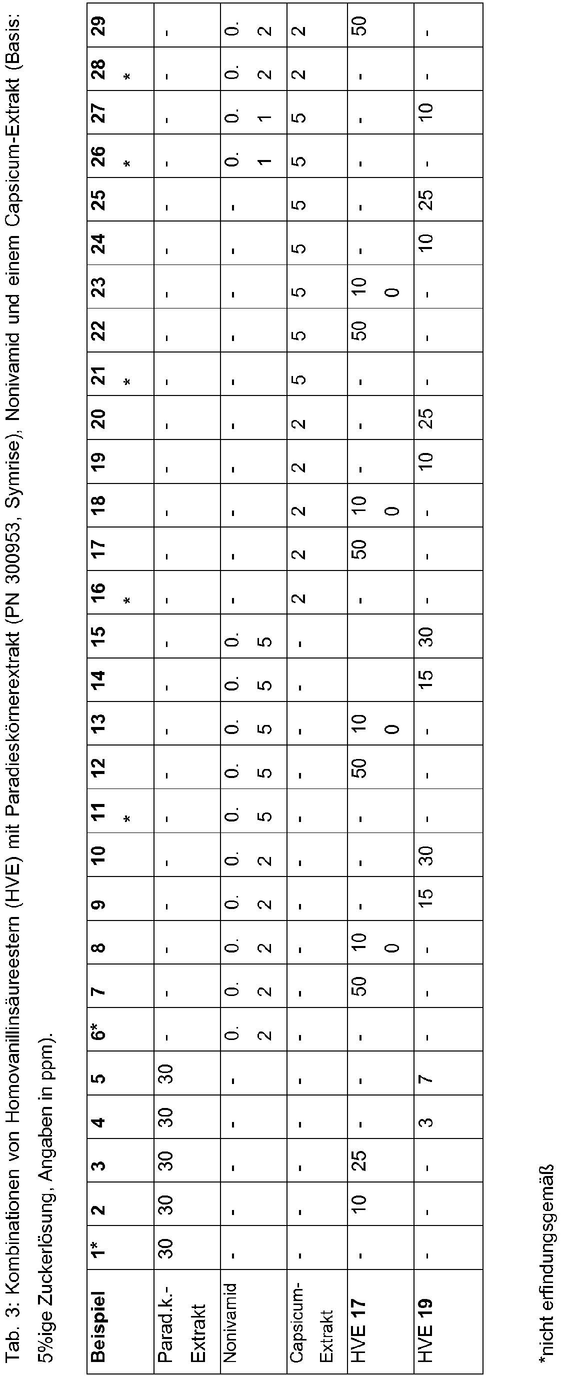

Homovanillinsäureester haben einen verstärkenden Einfluss auf die eingesetzten Verbindungen. Für die Kombination von Paradieskörnerextrakt (PN 300953, Symrise) mit Ethyl-2-(4-hydroxy-3-methoxyphenyl)acetat (**17**, Beispiele 2 und 3) wurde ein schnelleres Einsetzen der Schärfe festgestellt. Bei der Kombination von Ethyl-2-(4-hydroxy-3-methoxy-phenyl)acetat (**17**) mit Nonivamid (Beispiele 7 und 8 sowie 12 und 13) wurde in beiden geprüften Konzentrationen die Schärfe von Nonivamid verstärkt, wobei diese Verstärkung mehr als dem additiven Effekt entsprach. Weiterhin wurde ein schnelleres Einsetzen der Schärfe festgestellt. Derselbe verstärkende und schneller einsetzende Effekt wurde auch in der Kombination von Ethyl-2-(4-hydroxy-3-methoxy-phenyl)acetat (**17**) mit 2 ppm Capsicum-Extrakt 1.000.000 SHU festgestellt (Beispiele 17 und 18), wobei die Schärfe als angenehm und nicht lang nachklingend beurteilt wurde. Ohne die Kombination mit einem Homovanillylester wurde der Eintritt der Schärfe von Capsicum-Extrakt 1.000.000 SHU erst verzögert wahrgenommen (Beispiele 16* und 21*). Die Verstärkung der Schärfe war bei 2 ppm Capsicum-Extrakt 1.000.000 SHU deutlicher als bei 5 ppm. Bei der Kombination von 5 ppm Capsicum-Extrakt 1.000.000 SHU mit verschiedenen Mengen Ethyl-2-(4-hydroxy-3-methoxy-phenyl)acetat (**17**, Beispiele 22 und 23) wurde ein schnelles Abklingen der Schärfe deutlich. Zusätzlich wurde die Schärfe von Capsicum-Extrakt 1.000.000 SHU in der Kombination mit Ethyl-2-(4-hydroxy-3-methoxy-phenyl)acetat (**17**) in allen Konzentrationen um einen angenehmen wärmenden Effekt erweitert, der nach Abklingen des Schärfeempfindens zurückblieb. Für Butyl-2-(4-hydroxy-3-methoxy-phenyl)acetat (**19**) wurden dieselben Effekte bereits bei geringeren Konzentrationen festgestellt (Beispiele 19 und 20 sowie 24 und 25), wobei die Verstärkungen ebenfalls deutlicher als einem additiven Effekt waren. Auch eine Kombination von Nonivamid, Capsicum-Extrakt 1.000.000 SHU und Ethyl-2-(4-hydroxy-3-methoxy-phenyl)acetat (**17**, Beispiel 27) oder Butyl-2-(4-hydroxy-3-methoxy-phenyl)acetat (**19**, Beispiel 29) zeigte zusätzlich zu den Kombinationen ohne Homovanillinsäureester (Beispiele 26* und 28*) ein schnelleres Abklingen der Schärfe sowie zusätzlich einen wärmenden Effekt.

### Beispiel 10: Verstärkung der Alkoholschärfe einer ethanolischen Lösung durch Ethyl-2-(4-hydroxy-3-methoxy-phenyl)acetat (17)

Auf der Basis einer 5%igen Zuckerlösung mit 20% Ethanol (Beispiel 1) wurden verschiedene Konzentrationen (10-100 ppm) von Ethyl-2-(4-hydroxy-3-methoxy-phenyl)acetat (**17**), Flavon polymethoxyliert PMF 60 (Miritz) sowie Kombinationen (10-50 ppm) von Ethyl-2-(4-hydroxy-3-methoxy-phenyl)acetat (**17**) mit Flavon polymethoxyliert PMF 60 (Miritz) (20 ppm) mit einer zweiten Basislösung (5%ige Zuckerlösung mit 40% Ethanol) verglichen (Tab. 4). Hierfür wurde von 5 Prüfern der Mundraum jeweils mit ca. 5 mL der jeweiligen Verkostungslösung gespült, die Lösung wieder ausgespuckt und der Geschmackseindruck bewertet.

**Tab. 4: Kombinationen von Ethyl-2-(4-hydroxy-3-methoxy-phenyl)acetat (17) und Flavon polymethoxyliert PMF 60 (Miritz) in einer 5%igen Zuckerlösung mit 20% Ethanol (Angaben in ppm).**

| **Inhaltsstoff** | **1*** | **2** | **3** | **4** | **5** | **6*** | **7** | **8** | **9** |
|---|---|---|---|---|---|---|---|---|---|
| Ethyl-2-(4-hydroxy-3-methoxy-phenyl)acetat (**17**) | - | 10 | 20 | 50 | 100 | - | 10 | 20 | 50 |
| Flavon polymethoxyliert PMF 60 | - | - | - | - | - | 20 | 20 | 20 | 20 |

| | | | | | | | | | |
|---|---|---|---|---|---|---|---|---|---|
| *nicht erfindungsgemäß | | | | | | | | | |

Die Basislösung (5%ige Zuckerlösung mit 20% Ethanol, Beispiel 1) wurde als alkoholisch, brennend beschrieben. Die Zugabe von Ethyl-2-(4-hydroxy-3-methoxy-phenyl)acetat (**17**, Beispiel 2) führte zu einer Verstärkung und einer Verlängerung des brennenden Mundgefühls. Weitere Erhöhung auf 20 ppm und 50 ppm (Beispiele 2-5) ergab zusätzlich einen Schärfeeffekt. Flavon polymethoxyliert PMF 60 alleine (Beispiel 6) ergab in der Verkostungslösung eine Verstärkung des alkoholischen Geschmacks, aber kein brennendes Mundgefühl im Vergleich zu Beispiel 1. Durch die Kombination mit Ethyl-2-(4-hydroxy-3-methoxy-phenyl)acetat (**17**, Beispiele 7-9) wurde dieser Geschmack verstärkt, zusätzlich eine Verstärkung des brennenden Gefühls erzeugt und auch eine Verlängerung dieser Effekte erreicht, wobei der Einsatz von 20-50 ppm Ethyl-2-(4-hydroxy-3-methoxy-phenyl)acetat (**17**) sensorisch bevorzugt wurde. Im Vergleich zur zweiten Basislösung (5%igen Zuckerlösung mit 40% Ethanol) mit einem höheren Alkoholgehalt und starkem nasalen Effekt zeigte diese Kombination keine nasale Einwirkung.

### Beispiel 11: Wärmender Effekt von Homovanillinsäureestern im Vergleich zu Vanillylbutylether

Es wurden Testlösungen mit 4-10 ppm Homovanillinsäureester in einer 5%igen Zuckerlösung sensorisch bewertet und mit einer Testlösung von 10 ppm Vanillylbutylether verglichen. Ethyl-2-(4-hydroxy-3-methoxy-phenyl)acetat (**17**) hatte einen milderen Wärmeeffekt im Vergleich zu Vanillylbutylether. Butyl-2-(4-hydroxy-3-methoxy-phenyl)acetat (**19**) hatte einen deutlicheren Wärmeeffekt als Vanillylbutylether. Isobutyl-2-(4-hydroxy-3-methoxy-phenyl)acetat (**9**) zeigte einen Wärmeeffekt ähnlich zu Vanillylbutylether, der aber länger anhielt. Propyl-2-(4-hydroxy-3-methoxyphenyl)acetat (**18**) zeigte ebenfalls einen langanhaltenden Wärmeeffekt, der aber vergleichsweise später eintrat.

### Anwendungsbeispiele

### Anwendungsbeispiel 1: Typisch scharfe Aromakompositionen, enthaltend Homovanillinsäureester

Es wurden vermischt (alle Angaben, soweit nicht anders vermerkt, in Gew-%):

| **Inhaltsstoff** | **A** | **B** | **C** | **D** | **E** | **F** | **G** | **H** |
|---|---|---|---|---|---|---|---|---|
| 10 Gew.-% Pellitorin in 1,2-Propylenglycol/Diethylmalonat | 0.25 | 0.25 | 0.25 | 0.25 | 0.25 | 0.25 | 0.25 | 0.25 |
| Hesperetin | 2.50 | 2.50 | 2.50 | 2.50 | 2.50 | 2.50 | 2.50 | 2.50 |
| Phloretin | 1.50 | 1.50 | 1.50 | 1.50 | 1.50 | 1.50 | 1.50 | 1.50 |
| Ethyl-2-(4-hydroxy-3-methoxy-phenyl)acetat (**17**) | 3.00 | - | 1.50 | 1.00 | - | - | - | - |
| Butyl-2-(4-hydroxy-3-methoxy-phenyl)acetat (**19**) | - | 0.50 | 0.25 | - | - | - | - | - |
| Propyl-2-(4-hydroxy-3-methoxy-phenyl)acetat (**18**) | - | - | - | 1.50 | - | - | 1.00 | - |
| 3-Phenylpropyl 2-(4-hydroxy-3-methoxy-phenyl)acetat (**22**) | - | - | - | - | 0.05 | - | - | - |
| 4-Phenylbutyl 2-(4-hydroxy-3-methoxy-phenyl)acetat (**23**) | - | - | - | - | - | 0.07 | - | - |
| Heptyl-2-(4-hydroxy-3-methoxy-phenyl)acetat (**14**) | - | - | - | - | - | - | 0.10 | - |
| [(E)-Cinnamyl] 2-(4-hydroxy-3-methoxy-phenyl)acetat (**2**) | - | - | - | - | - | - | - | 0.06 |
| Propylenglycol | ad 100 | ad 100 | ad 100 | ad 100 | ad 100 | ad 100 | ad 100 | ad 100 |

Die Inhaltsstoffe (Stoffe bzw. Lösungen) werden in den oben angegebenen Mengenverhältnissen gemischt und dann mit Propylenglycol aufgenommen und durch leichtes Erwärmen vollständig gelöst.

### Anwendungsbeispiel 2: Sprühgetrocknete Aromakompositionen, enthaltend Homovanillinsäureester

Es wurden vermischt (alle Angaben, soweit nicht anders vermerkt, in Gew-%):

| **Inhaltsstoff** | **A** | **B** | **C** | **D** | **E** | **F** | **G** | **H** |
|---|---|---|---|---|---|---|---|---|
| Ethyl-2-(4-hydroxy-3-methoxy-phenyl)acetat (**17**) | 10.00 | - | - | - | - | - | - | - |
| Butyl-2-(4-hydroxy-3-methoxy-phenyl)acetat (**19**) | - | 3.50 | - | - | - | - | - | - |
| Propyl-2-(4-hydroxy-3-methoxy-phenyl)acetat (**18**) | - | - | 5.00 | - | - | - | - | - |
| Hexyl-2-(4-hydroxy-3-methoxy-phenyl)acetat (**21**) | - | - | - | 2.50 | - | - | - | - |
| 3-Phenylpropyl 2-(4-hydroxy-3-methoxy-phenyl)acetat (**22**) | - | - | - | - | 0.20 | - | - | - |
| 4-Phenylbutyl 2-(4-hydroxy-3-methoxy-phenyl)acetat (**23**) | - | - | - | - | - | 0.22 | - | - |
| Heptyl-2-(4-hydroxy-3-m ethoxy-phenyl)acetat (**14**) | - | - | - | - | - | - | 0.25 | - |
| [(E)-Cinnamyl] 2-(4-hydroxy-3-methoxy-phenyl)acetat (**2**) | - | - | - | - | - | - | - | 0.21 |
| Maltodextrin | ad 100 | ad 100 | ad 100 | ad 100 | ad 100 | ad 100 | ad 100 | ad 100 |

Die beiden Bestandteile werden in einer Mischung aus Ethanol und demineralisiertem Wasser gelöst und anschließend sprühgetrocknet.

### Anwendungsbeispiel 3: Herstellung von Mundwasser-Aromen unter Verwendung der oben beschriebenen Geschmackstoffe:

Es wurden vermischt (alle Angaben, soweit nicht anders vermerkt, in Gew-%):

| **Inhaltsstoff** | **A** | **B** | **C** | **D** | **E** | **F** | **G** | **H** | **I** |
|---|---|---|---|---|---|---|---|---|---|
| Anethol | 30 | 30 | 30 | 30 | 30 | 30 | 30 | 30 | 30 |
| Eucalyptol | 25 | 25 | 25 | 25 | 25 | 25 | 25 | 25 | 25 |
| L-Menthol | 36.00 | 36.00 | 38.00 | 36.20 | 36.00 | 36.90 | 39.85 | 34.90 | 39.90 |
| Optacool A | - | 8 | - | 8 | - | 8 | - | 8 | - |
| Coolact 10 | 5 | - | 5 | - | 5 | - | 5 | - | 5 |
| Ethyl-2-(4-hydroxy-3-methoxy-phenyl)acetat (**17**) | 4.00 | - | - | - | 3.00 | - | - | 2.00 | - |
| Butyl-2-(4-hydroxy-3-methoxy-phenyl)acetat (**19**) | - | 1.00 | - | - | - | - | - | - | - |
| Propyl-2-(4-hydroxy-3-methoxy-phenyl)acetat (**18**) | - | - | 2.00 | - | 1.00 | - | - | - | - |
| Hexyl-2-(4-hydroxy-3-methoxy-phenyl)acetat (**21**) | - | - | - | 0.80 | - | - | - | - | - |
| 3-Phenylpropyl 2-(4-hydroxy-3-methoxy-phenyl)acetat (**22**) | - | - | - | - | - | 0.10 | - | - | - |
| 4-Phenylbutyl 2-(4-hydroxy-3-methoxy-phenyl)acetat (**23**) | - | - | - | - | - | - | 0.15 | - | - |
| Heptyl-2-(4-hydroxy-3-methoxy-phenyl)acetat (**14**) | - | - | - | - | - | - | - | 0.10 | - |
| [(E)-Cinnamyl] 2-(4-hydroxy-3-methoxy-phenyl)acetat (**2**) | - | - | - | - | - | - | - | - | 0.10 |
| Total | 100 | 100 | 100 | 100 | 100 | 100 | 100 | 100 | 100 |

### Anwendungsbeispiel 4: Herstellung von Aromen mit Pfefferminzgeschmack unter Verwendung von erfindungsgemäßen Substanzen:

Es wurden vermischt (alle Angaben, soweit nicht anders vermerkt, in Gew-%):

| **Inhaltsstoff** | **A** | **B** | **C** | **D** | **E** | **F** | **G** | **H** | **I** | **K** |
|---|---|---|---|---|---|---|---|---|---|---|
| Pfefferminzöl *Mentha arvensis* | 56 | 59.00 | 58.50 | 58.80 | 56 | 59 | 60 | 60 | 59.5 | 60 |
| L-Menthon | 20 | 20 | 20 | 20 | 20 | 20 | 20 | 20 | 20 | 20 |
| L-Menthol | 20 | 20 | 20 | 20 | 20 | 20 | 20 | 20 | 20 | 20 |
| Ethyl-2-(4-hydroxy-3-methoxy-phenyl)acetat (**17**) | 4.00 | - | - | - | 3.00 | - | - | - | - | - |
| Butyl-2-(4-hydroxy-3-methoxy-phenyl)acetat (**19**) | - | 1.00 | - | - | - | 0.50 | - | - | 0.50 | - |
| Propyl-2-(4-hydroxy-3-methoxy-phenyl)acetat (**18**) | - | - | 1.50 | - | 1.00 | 0.50 | - | - | - | - |
| Pentyl-2-(4-hydroxy-3-methoxy-phenyl)acetat (**20**) | - | - | - | 1.20 | - | - | - | - | - | - |
| 3-Phenylpropyl 2-(4-hydroxy-3-methoxy-phenyl)acetat (**22**) | - | - | - | - | - | - | 0.20 | - | - | - |
| 4-Phenylbutyl 2-(4-hydroxy-3-methoxy-phenyl)acetat (**23**) | - | - | - | - | - | - | - | 0.22 | - | - |
| Heptyl-2-(4-hydroxy-3-methoxy-phenyl)acetat (**14**) | - | - | - | - | - | - | - | - | 0.25 | - |
| [(E)-Cinnamyl] 2-(4-hydroxy-3-methoxy-phenyl)acetat (2) | - | - | - | - | - | - | - | - | - | 0.21 |
| Total | 100 | 100 | 100 | 100 | 100 | 100 | 100 | 100 | 100 | 100 |

Die Aromakompositionen wurden in nachfolgend beschriebenen Anwendungsbeispielen eingesetzt.

### Anwendungsbeispiel 5: Alkoholreduziertes Getränk

Herstellung eines Biermix-Getränkes mit reduziertem Alkoholgehalt oder ohne Alkohol. Alle Angaben sind in Gew-%.

| **Bestandteil** | **A*** | **B** | **C** | **D** | **E** | **F** | **G** | **H** | **I** |
|---|---|---|---|---|---|---|---|---|---|
| Zuckersirup | 4 | 4 | 4 | 4 | 4 | 4 | 4 | 4 | 4 |
| Bier (4.9% vol.) | 50 | - | - | - | - | - | - | - | - |
| Bier (alkohol- und kalorienreduziert, 2.8% vol.) | - | - | 50 | - | 50 | - | 50 | - | 50 |
| Bier (alkoholfrei, 0%) | - | 50 | - | 50 | - | 50 | - | 50 | - |
| Zitronensäure | 0.15 | 0.15 | 0.15 | 0.15 | 0.15 | 0.15 | 0.15 | 0.15 | 0.15 |
| Ascorbinsäure | 0.01 | 0.01 | 0.01 | 0.01 | 0.01 | 0.01 | 0.01 | 0.01 | 0.01 |
| Grapefruitsaft | 6 | 6 | 6 | 6 | 6 | 6 | 6 | 6 | 6 |
| Grapefruitaroma | 0.20 | 0.20 | 0.20 | 0.20 | 0.20 | 0.20 | 0.20 | 0.20 | 0.20 |
| Aromakomposition (Anwendungsbeispiel 1A) | - | 0.20 | 0.20 | - | - | - | - | - | - |
| Aromakomposition (Anwendungsbeispiel 1B) | - | - | - | 0.20 | 0.20 | - | - | - | - |
| Aromakomposition (Anwendungsbeispiel 1E) | - | - | - | - | - | 0.20 | 0.20 | - | - |
| Aromakomposition (Anwendungsbeispiel 1H) | - | - | - | - | - | - | - | 0.20 | 0.20 |
| Wasser | ad 100 | ad 100 | ad 100 | ad 100 | ad 100 | ad 100 | ad 100 | ad 100 | ad 100 |
| Kohlensäure | 0.70 | 0.70 | 0.70 | 0.70 | 0.70 | 0.70 | 0.70 | 0.70 | 0.70 |

| | | | | | | | | | |
|---|---|---|---|---|---|---|---|---|---|
| *nicht erfindungsgemäß | | | | | | | | | |

### Anwendungsbeispiel 6: Anwendungen in einer Zahnpasta ('Silica opaque')

Alle Angaben, soweit nicht anders vermerkt, in Gew-%.

| **Bestandteil** | **A** | **B** | **C** | **D** | **E** | **F** | **G** | **H** | **I** | **K** |
|---|---|---|---|---|---|---|---|---|---|---|
| Entionisiertes Wasser | 26.53 | 26.53 | 26.53 | 26.53 | 26.53 | 26.53 | 26.53 | 26.53 | 26.53 | 26.53 |
| Sorbitol 70% | 45 | 44.975 | 45 | 44.975 | 45 | 44.975 | 45 | 44.975 | 45 | 44.975 |
| Solbrol M Na-Salz | 0.15 | 0.15 | 0.15 | 0.15 | 0.15 | 0.15 | 0.15 | 0.15 | 0.15 | 0.15 |
| Trinatriumphosphat | 0.10 | 0.10 | 0.10 | 0.10 | 0.10 | 0.10 | 0.10 | 0.10 | 0.10 | 0.10 |
| Saccharin | 0.20 | 0.20 | 0.20 | 0.20 | 0.20 | 0.20 | 0.20 | 0.20 | 0.20 | 0.20 |
| Natriummonofluorphosphat | 1.12 | 1.12 | 1.12 | 1.12 | 1.12 | 1.12 | 1.12 | 1.12 | 1.12 | 1.12 |
| PEG 1500 | 5 | 5 | 5 | 5 | 5 | 5 | 5 | 5 | 5 | 5 |
| Sident 9 (Abrasive Silica) | 10 | 10 | 10 | 10 | 10 | 10 | 10 | 10 | 10 | 10 |
| Sident 22 S (Dickende Silica) | 8 | 8 | 8 | 8 | 8 | 8 | 8 | 8 | 8 | 8 |
| Natriumcarboxymethylcellul ose | 0.90 | 0.90 | 0.90 | 0.90 | 0.90 | 0.90 | 0.90 | 0.90 | 0.90 | 0.90 |
| Titan (IV) oxid | 0.50 | 0.50 | 0.50 | 0.50 | 0.50 | 0.50 | 0.50 | 0.50 | 0.50 | 0.50 |
| Natriumlaurylsulfat (SLS) | 1.50 | 1.50 | 1.50 | 1.50 | 1.50 | 1.50 | 1.50 | 1.50 | 1.50 | 1.50 |
| Pellitorin-Lösung PLM (enthaltend 10% Pellitorin) | - | 0.025 | - | 0.025 | - | 0.025 | - | 0.025 | - | 0.025 |
| Aroma Typ Pfefferminze (Anwendungsbeispiel 4A) | 1.00 | 1.00 | - | - | - | - | - | - | - | - |
| Aroma Typ Pfefferminze (Anwendungsbeispiel 4B) | - | - | 1.00 | 1.00 | - | - | - | - | - | - |
| Aroma Typ Pfefferminze (Anwendungsbeispiel 4C) | - | - | - | - | 1.00 | 1.00 | - | - | - | - |
| Aroma Typ Pfefferminze (Anwendungsbeispiel 4G) | - | - | - | - | - | - | 1.00 | 1.00 | - | - |
| Aroma Typ Pfefferminze (Anwendungsbeispiel 4K) | - | - | - | - | - | - | - | - | 1.00 | 1.00 |
| Total | 100 | 100 | 100 | 100 | 100 | 100 | 100 | 100 | 100 | 100 |

### Anwendungsbeispiel 7: Anwendung in einer Zahnpasta (Calciumcarbonat-Base)

Alle Angaben, soweit nicht anders vermerkt, in Gew-%.

| **Bestandteil** | **A** | **B** | **C** | **D** | **E** | **F** | **G** | **H** | **I** | **K** |
|---|---|---|---|---|---|---|---|---|---|---|
| Entionisiertes Wasser | 27.5 | 27.48 | 27.5 | 27.48 | 27.5 | 27.48 | 27.5 | 27.48 | 27.5 | 27.48 |
| Saccharin | 0.20 | 0.20 | 0.20 | 0.20 | 0.20 | 0.20 | 0.20 | 0.20 | 0.20 | 0.20 |
| Solbrol M Natriumsalz | 0.20 | 0.20 | 0.20 | 0.20 | 0.20 | 0.20 | 0.20 | 0.20 | 0.20 | 0.20 |
| Natriummonofluorphosphat | 0.80 | 0.80 | 0.80 | 0.80 | 0.80 | 0.80 | 0.80 | 0.80 | 0.80 | 0.80 |
| Sorbitol 70% | 29 | 29 | 29 | 29 | 29 | 29 | 29 | 29 | 29 | 29 |
| Calciumcarbonat | 35 | 35 | 35 | 35 | 35 | 35 | 35 | 35 | 35 | 35 |
| Sident 22 S (Verdickende Silica) | 2.50 | 2.50 | 2.50 | 2.50 | 2.50 | 2.50 | 2.50 | 2.50 | 2.50 | 2.50 |
| Natriumcarboxymethylcell ulose | 1.30 | 1.30 | 1.30 | 1.30 | 1.30 | 1.30 | 1.30 | 1.30 | 1.30 | 1.30 |
| Titandioxid | 0.50 | 0.50 | 0.50 | 0.50 | 0.50 | 0.50 | 0.50 | 0.50 | 0.50 | 0.50 |
| Natriumlaurylsulfat | 2 | 2 | 2 | 2 | 2 | 2 | 2 | 2 | 2 | 2 |
| Pellitorin-Lösung PLM (enthaltend 10% Pellitorin) | - | 0.02 | - | 0.02 | - | 0.02 | - | 0.02 | - | 0.02 |
| Aroma Typ Pfefferminze (Anwendungsbeispiel 4D) | 1.00 | 1.00 | - | - | - | - | - | - | - | - |
| Aroma Typ Pfefferminze (Anwendungsbeispiel 4E) | - | - | 1.00 | 1.00 | - | - | - | - | - | - |
| Aroma Typ Pfefferminze (Anwendungsbeispiel 4F) | - | - | - | - | 1.00 | 1.00 | - | - | - | - |
| Aroma Typ Pfefferminze (Anwendungsbeispiel 4H) | - | - | - | - | - | - | 1.00 | 1.00 | - | - |
| Aroma Typ Pfefferminze (Anwendungsbeispiel 4I) | - | - | - | - | - | - | - | - | 1.00 | 1.00 |
| Total | 100 | 100 | 100 | 100 | 100 | 100 | 100 | 100 | 100 | 100 |

Durch die Verwendung der erfindungsgemäßen Substanzen wird ein schnell einsetzendes angenehmes Schärfeprofil mit wärmenden Aspekten erreicht. Zusätzlich wird ein verstärkter mundwässernder Effekt erzielt.

### Anwendungsbeispiel 8: Anwendung in einem zuckerfreien Kaugummi

| **Teil** | **Inhaltsstoff** | **Gew.-%** |
|---|---|---|
| A | Kaugummibase, Company "Jagum T" | 30.00 |
| B | Sorbit, pulverisiert | 39.00 |
| | Isomalt® (Palatinit GmbH) | 9.50 |
| | Xylit | 2.00 |
| | Mannit | 3.00 |
| | Aspartam® | 0.10 |
| | Acesulfam® K | 0.10 |
| | Emulgum® (Colloides Naturels, Inc.) | 0.30 |
| C | Sorbitol, 70 % | 14.00 |
| | Glycerin | 1.00 |
| D | Aromakompositionen gemäß Anwendungsbeispiel 4 | 1.00 |

Die Teile A bis D werden gemischt und intensiv geknetet. Die erhaltene Rohmasse kann dann z.B. in Form von dünnen Streifen zu verzehrfertigen Kaugummis verarbeitet werden.

### Anwendungsbeispiel 9: Mundwasser ("ready to use" ohne Alkohol)

Alle Angaben, soweit nicht anders vermerkt in Gew-%.

| **Bestandteil** | **A** | **B** | **C** | **D** | **E** | **F** | **G** | **H** | **I** | **K** |
|---|---|---|---|---|---|---|---|---|---|---|
| Cremophor RH 455 | 1.80 | 1.80 | 1.80 | 1.80 | 1.80 | 1.80 | 1.80 | 1.80 | 1.80 | 1.80 |
| Entionisiertes Wasser | 87.57 | 87.5575 | 87.57 | 87.5575 | 87.57 | 87.5575 | 87.57 | 87.5575 | 87.57 | 87.5575 |
| Sorbitol 70% | 10 | 10 | 10 | 10 | 10 | 10 | 10 | 10 | 10 | 10 |
| Natriumfluorid | 0.18 | 0.18 | 0.18 | 0.18 | 0.18 | 0.18 | 0.18 | 0.18 | 0.18 | 0.18 |
| Natriumsaccharin 450 | 0.10 | 0.10 | 0.10 | 0.10 | 0.10 | 0.10 | 0.10 | 0.10 | 0.10 | 0.10 |
| Solbrol M Natriumsalz | 0.15 | 0.15 | 0.15 | 0.15 | 0.15 | 0.15 | 0.15 | 0.15 | 0.15 | 0.15 |
| Pellitorin-Lösung PLM (enthaltend 10% Pellitorin) | - | 0.0125 | - | 0.0125 | - | 0.0125 | | 0.0125 | | 0.0125 |
| Mundwasser Aroma (Anwendungsbeispiel 3A) | 0.20 | 0.20 | - | - | - | - | - | - | - | - |
| Mundwasser Aroma (Anwendungsbeispiel 3B) | - | - | 0.20 | 0.20 | - | - | - | - | - | - |
| Mundwasser Aroma (Anwendungsbeispiel 3C) | - | - | - | - | 0.20 | 0.20 | - | - | - | - |
| Mundwasser Aroma (Anwendungsbeispiel 3F) | - | - | - | - | - | - | 0.20 | 0.20 | - | - |
| Mundwasser Aroma (Anwendungsbeispiel 3I) | - | - | - | - | - | - | - | - | 0.20 | 0.20 |
| Total | 100 | 100 | 100 | 100 | 100 | 100 | 100 | 100 | 100 | 100 |

### Anwendungsbeispiel 10: Zahncreme und Mundwasser als 2-in-1 Produkt

Alle Angaben sind in Gew-%.

| **Bestandteil** | **A** | **B** | **C** | **D** | **E** | **F** | **G** |
|---|---|---|---|---|---|---|---|
| Ethanol, 96%ig | 5 | 5 | 5 | 5 | 5 | 5 | 5 |
| Sorbitol, 70 %ig in Wasser | 40 | 40 | 40 | 40 | 40 | 40 | 40 |
| Glycerin | 20 | 20 | 20 | 20 | 20 | 20 | 20 |
| Saccharin | 0.20 | 0.20 | 0.20 | 0.20 | 0.20 | 0.20 | 0.20 |
| Na-Monofluorphosphat | 0.76 | 0.76 | 0.76 | 0.76 | 0.76 | 0.76 | 0.76 |
| Solbrol M, Na-Salz | 0.15 | 0.15 | 0.15 | 0.15 | 0.15 | 0.15 | 0.15 |
| Abrasivkieselsäure (Sident 9) | 20 | 20 | 20 | 20 | 20 | 20 | 20 |
| Verdickungskieselsäure (Sident 22S) | 2 | 2 | 2 | 2 | 2 | 2 | 2 |
| Na-Carboxymethylcellu lose | 0.30 | 0.30 | 0.30 | 0.30 | 0.30 | 0.30 | 0.30 |
| Natriumlaurylsulfat | 1.20 | 1.20 | 1.20 | 1.20 | 1.20 | 1.20 | 1.20 |
| Grüner Farbstoff (1%ig in Wasser) | 0.50 | 0.50 | 0.50 | 0.50 | 0.50 | 0.50 | 0.50 |
| Wasser dest. | 9.39 | 9.39 | 9.39 | 9.39 | 8.89 | 8.89 | 8.89 |
| Aromakomposition (Anwendungsbeispiel 3A) | 0.50 | - | - | - | - | - | - |
| Aromakomposition (Anwendungsbeispiel 3B) | - | 0.50 | - | - | - | - | - |
| Aromakomposition (Anwendungsbeispiel 3C) | - | - | 0.50 | - | - | - | - |
| Aromakomposition (Anwendungsbeispiel 3D) | - | - | - | 0.50 | - | - | - |
| Aromakomposition (Anwendungsbeispiel 3F) | - | - | - | - | 1.00 | - | - |
| Aromakomposition (Anwendungsbeispiel 3H) | - | - | - | - | - | 1.00 | - |
| Aromakomposition (Anwendungsbeispiel 3I) | - | - | - | - | - | - | 1.00 |
| Total | 100 | 100 | 100 | 100 | 100 | 100 | 100 |

### Anwendungsbeispiel 11: Anwendung in Kombination mit einem scharfen Pflanzenextrakt als Alkoholverstärker

**Vergleichsprobe Likörbase 10%vol**

| | |
|---|---|
| 7.39 kg | Alkohol, p.A.-Ware |
| 20 kg | Invertzuckersirup, 66.5 % Trockenmasse |
| 72.61 kg | Wasser |
| Summe 100 kg | |

**Likorbase 5,5%vol**

| | |
|---|---|
| 4.06 kg | Alkohol, p.A.-Ware |
| 20 kg | Invertzuckersirup, 66.5 % Trockenmasse |
| 75.94 kg | Wasser |
| Summe 100 kg | |

Version A: Likörbase 5.5 %vol + 0.3 % einer 10 %igen Lösung eines Paradieskörnerextrakts in Ethanol

Version B: Likörbase 5.5 %vol + 0.075% einer 10 %igen Lösung eines Paradieskörnerextrakts in Ethanol + 0.2% einer Lösung von Ethyl-2-(4-hydroxy-3-methoxy-phenyl)acetat (**17**) 1%ig in Ethanol (entspricht 20 ppm).

Version C: Likörbase 5.5 %vol + 0.075% einer 10 %igen Lösung eines Paradieskörnerextrakts in Ethanol + 0.01% einer Lösung von 3-Phenylpropyl 2-(4-hydroxy-3-methoxy-phenyl)acetat (**22**) 1%ig in Ethanol (entspricht 1 ppm).

Bei Versionen B und C wird sensorisch die Alkoholschärfe der Vergleichsprobe besser imitiert als in Version A. Version A und die Vergleichsprobe sind sensorisch sehr ähnlich zu bewerten.

### Anwendungsbeispiel 12: Fußbalsam

| | **Gew.-%** | **Inhaltsstoff (INCI)** |
|---|---|---|
| A | 2.00 | Ceteareth-6, Stearylalkohol |
| | 2.00 | Ceteareth-25 |
| | 5.00 | Cetearylethylhexanoat |
| | 4.00 | Cetylalkohol |
| | 4.00 | Glycerylstearat |
| | 5.00 | Mineralöl |
| | 0.20 | Menthol |
| | 0.50 | Kampfer |
| B | 69.30 | Aqua dem. |
| | q.s. | Konservierungsmittel |
| C | 1.00 | Bisabolol |
| | 1.00 | Tocopherylacetat |
| D | 1.00 | wässrige Lösung mit 1-1.5 % Pentyl-2-(4-hydroxy-3-methoxy-phenyl)acetat (20) |
| | 5.00 | Zaubernussextrakt |
| | 100 | Total |

Herstellung: Die Komponenten der Phasen A und B getrennt voneinander auf ca. 80 °C erwärmen. Phase B in Phase A unter Homogenisieren einrühren. Unter Rühren abkühlen auf ca. 40 °C, die Phasen C und D hinzugeben und kurz nachhomogenisieren. Unter Rühren auf Raumtemperatur abkühlen.

### Anwendungsbeispiel 13: Zuckerfreie Hartkaramelle

| **Inhaltsstoff** | **Gehalt [Gew.-%]** |
|---|---|
| Palatinit, Typ M | 75.47 |
| Wasser | 24.03 |
| Pfefferminzaroma | 0.10 |
| Aromazubereitung (Beispiel 1) | 0.40 |
| Total | 100 |

Zunächst wurde Palatinit mit Wasser gemischt. Die Mischung wurde dann bei 165 °C aufgeschmolzen und anschließend auf 115°C abgekühlt. Dann wurden das Pfefferminzaroma und die Aromazubereitung (Beispiel 1) zugegeben. Nach dem Durchmischen wurden die Mischungen in Formen gegossen, nach dem Erstarren aus den Formen entfernt und anschließend einzeln verpackt.

### Anwendungsbeispiel 14: Würzmischung, Typ "Pfeffer"

A = Vergleichszubereitung
B, C, D, E = erfindungsgemäße Zubereitungen

Alle Angaben sind in Gew-%.

| **Bestandteil** | **A*** | **B** | **C** | **D** | **E** | **F** | **G** |
|---|---|---|---|---|---|---|---|
| Milchprotein | 0.80 | 0.80 | 0.80 | 0.80 | 0.80 | 0.80 | 0.80 |
| Johannisbrotkernmehl | 2.00 | 2.00 | 2.00 | 2.00 | 2.00 | 2.00 | 2.00 |
| Maisstärke | 24.00 | 23.00 | 23.00 | 23.00 | 23.00 | 23.00 | 23.00 |
| Kochsalz | 14.00 | 14.00 | 14.00 | 14.00 | 14.00 | 14.00 | 14.00 |
| Paprikapulver | 12.00 | 12.00 | 12.00 | 12.00 | 12.00 | 12.00 | 12.00 |
| Tomatenpulver | 12.00 | 12.00 | 12.00 | 12.00 | 12.00 | 12.00 | 12.00 |
| Saccharose | 4.00 | 4.00 | 4.00 | 4.00 | 4.00 | 4.00 | 4.00 |
| Knoblauchpulver | 0.50 | 0.50 | 0.50 | 0.50 | 0.50 | 0.50 | 0.50 |
| Gehärtetes Pflanzenfett | 8.00 | 8.00 | 8.00 | 8.00 | 8.00 | 8.00 | 8.00 |
| Fettpulver | 11.00 | 11.00 | 11.00 | 11.00 | 11.00 | 11.00 | 11.00 |
| Natriumglutamat | 6.00 | 6.00 | 6.00 | 6.00 | 6.00 | 6.00 | 6.00 |
| Lebensmittelfarbstoff Rote Bete und Paprika | 2.00 | 2.00 | 2.00 | 2.00 | 2.00 | 2.00 | 2.00 |
| Aroma Typ "Pfeffer" | 2.00 | 2.00 | 2.00 | 2.00 | 2.00 | 2.00 | 2.00 |
| Aroma Typ "Pizza" | 1.20 | 1.20 | 1.20 | 1.20 | 1.20 | 1.20 | 1.20 |
| Aroma Typ "Tomate" | 0.40 | 0.40 | 0.40 | 0.40 | 0.40 | 0.40 | 0.40 |
| Extrakt aus schwarzem Pfeffer | 0.10 | 0.10 | 0.10 | 0.10 | 0.10 | 0.10 | 0.10 |
| Aromakomposition (Anwendungsbeispiel 1A) | - | 1.00 | - | - | - | - | - |
| Aromakomposition (Anwendungsbeispiel 1B) | - | - | 1.00 | - | - | - | - |
| Aromakomposition (Anwendungsbeispiel 1C) | - | - | - | 1.00 | - | - | - |
| Aromakomposition (Anwendungsbeispiel 1D) | - | - | - | - | 1.00 | - | - |
| Aromakomposition (Anwendungsbeispiel 1E) | - | - | - | - | - | 1.00 | - |
| Aromakomposition (Anwendungsbeispiel 1G) | - | - | - | - | - | - | 1.00 |
| Total | 100 | 100 | 100 | 100 | 100 | 100 | 100 |

| | | | | | | | |
|---|---|---|---|---|---|---|---|
| *nicht erfindungsgemäß | | | | | | | |

### Anwendungsbeispiel 15: Würzmischung für Kartoffelchips

A = Vergleichszubereitung
B, C, D, E = erfindungsgemäße Zubereitungen

Alle Angaben sind in Gew-%.

| **Bestandteil** | **A*** | **B** | **C** | **D** | **E** | **F** | **G** |
|---|---|---|---|---|---|---|---|
| Natriumglutamat | 3.50 | 3.50 | 3.50 | 3.50 | 3.50 | 3.50 | 3.50 |
| Käsepulver | 10.00 | 10.00 | 10.00 | 10.00 | 10.00 | 10.00 | 10.00 |
| Knoblauchpulver | 2.00 | 2.00 | 2.00 | 2.00 | 2.00 | 2.00 | 2.00 |
| Molkenpulver | 38.86 | 36.86 | 36.86 | 36.86 | 36.86 | 36.86 | 36.86 |
| Würzextraktöl | 0.20 | 0.20 | 0.20 | 0.20 | 0.20 | 0.20 | 0.20 |
| Paprikapulver | 9.80 | 9.80 | 9.80 | 9.80 | 9.80 | 9.80 | 9.80 |
| Kochsalz | 21.00 | 21.00 | 21.00 | 21.00 | 21.00 | 21.00 | 21.00 |
| Tomatenpulver | 9.00 | 9.00 | 9.00 | 9.00 | 9.00 | 9.00 | 9.00 |
| Trockenaroma | 2.50 | 2.50 | 2.50 | 2.50 | 2.50 | 2.50 | 2.50 |
| Siliciumdioxid | 0.02 | 0.02 | 0.02 | 0.02 | 0.02 | 0.02 | 0.02 |
| Pflanzenöl | 0.02 | 0.02 | 0.02 | 0.02 | 0.02 | 0.02 | 0.02 |
| Zwiebelpulver | 3.00 | 3.00 | 3.00 | 3.00 | 3.00 | 3.00 | 3.00 |
| Sahne Aromakonzentrat | 0.03 | 0.03 | 0.03 | 0.03 | 0.03 | 0.03 | 0.03 |
| Käse Aroma | 0.03 | 0.03 | 0.03 | 0.03 | 0.03 | 0.03 | 0.03 |
| Tomaten Aromakonzentrat | 0.04 | 0.04 | 0.04 | 0.04 | 0.04 | 0.04 | 0.04 |
| Sprühgetrocknete Zusammensetzung gemäß Anwendungsbeispiel 2A | - | 2.00 | - | - | - | | |
| Sprühgetrocknete Zusammensetzung gemäß Anwendungsbeispiel 2B | - | - | 2.00 | - | - | | |
| Sprühgetrocknete Zusammensetzung gemäß Anwendungsbeispiel 2C | - | - | - | 2.00 | - | | |
| Sprühgetrocknete Zusammensetzung gemäß Anwendungsbeispiel 2D | - | - | - | - | 2.00 | | |
| Sprühgetrocknete Zusammensetzung gemäß Anwendungsbeispiel 2E | | | | | | 2.00 | |
| Sprühgetrocknete Zusammensetzung gemäß Anwendungsbeispiel 2H | | | | | | | 2.00 |
| Total | 100 | 100 | 100 | 100 | 100 | 100 | 100 |

| | | | | | | | |
|---|---|---|---|---|---|---|---|
| nicht erfindungsgemäß | | | | | | | |

6 g der Würzmischung wurden auf 94 g Kartoffelchips aufgezogen.

### Anwendungsbeispiel 16: Anwendung in einem Grünteegetränk

| **Inhaltsstoff** | **Einsatz in Gew.-%** | |
|---|---|---|
| | A | B |
| Grünteekonzentrat | 18.00 | 18.00 |
| 1% ige Lösung Ethyl-2-(4-hydroxy-3-methoxyphenyl)acetat (**17**) | 0.40 | - |
| 1% ige Lösung 3-Phenylpropyl 2-(4-hydroxy-3-methoxy-phenyl)acetat (**22**) | - | 0.015 |
| demineralisiertes Wasser | 81.60 | 81.985 |
| Total | 100 | 100 |

Das Grünteekonzentrat wird im Fall von Getränk A mit der 1 %igen Lösung Ethyl-2-(4-hydroxy-3-methoxy-phenyl)acetat (17) in Propylenglycol und im Fall von Getränk B mit der 1 %igen Lösung 3-Phenylpropyl 2-(4-hydroxy-3-methoxy-phenyl)acetat (**22**) in Propylenglycol vermischt. Anschließend wird mit demineralisiertem Wasser aufgefüllt und erneut gründlich durchmischt. Dann wird das Produkt filtriert, verbrauchsfertig verpackt und bei 118°C sterilisiert. Der Geschmack der Getränke A und B wird durch ein Panel ausgebildeter Testpersonen als deutlich bevorzugt gegenüber dem nicht aromatisierten Grünteekonzentrat bewertet. Die Bitterkeit und Adstringenz wird durch die Zugabe der erfindungsgemäßen Verbindungen reduziert.

### Anwendungsbeispiel 17: Verwendung in einem Eistee-Getränk (Schwarztee)

Die Homovanillinsäureester wurden jeweils 10 % oder 1% in Ethanol vorgelöst. Schwarztee-Extrakt wurde in Wasser gelöst und zusammen mit Zucker, einer Aromazubereitung (Pfirsichgeschmack), sowie den ethanolischen Lösungen der Homovanillinsäurelester in einem Becherglas verrührt.

| **Inhaltsstoff** | **Einsatz in Gew.-%** | | | |
|---|---|---|---|---|
| | **A** | **B** | **C** | **D** |
| Schwarztee-Extrakt | 1.40 | 1.40 | 1.40 | 1.40 |
| Wasser | 89.5 | 89.51 | 89.51 | 89.515 |
| Aromazubereitung (Typ Pfirsich) | 0.67 | 0.67 | 0.67 | 0.67 |
| Zucker | 7 | 7 | 7 | 7 |
| Citronensäure (kristallin) | 1.20 | 1.20 | 1.20 | 1.20 |
| Ascorbinsäure | 0.20 | 0.20 | 0.20 | 0.20 |
| 10 % in Ethanol | 0.03 | - | - | - |
| Ethyl-2-(4-hydroxy-3-methoxy-phenyl)acetat (**17**) | | | | |
| 10 % in Ethanol | - | 0.02 | - | - |
| Propyl-2-(4-hydroxy-3-methoxy-phenyl)acetat (**18**) | | | | |
| 1% in Ethanol | - | - | 0.02 | - |
| 3-Phenylpropyl 2-(4-hydroxy-3-methoxy-phenyl)acetat (**22**) | | | | |
| 1% in Ethanol | - | - | - | 0.015 |
| [(E)-Cinnamyl] 2-(4-hydroxy-3-methoxy-phenyl)acetat (**2**) | | | | |
| Total | 100 | 100 | 100 | 100 |

### Anwendungsbeispiel 18: Anwendung in einer Boullion

A = Vergleichszubereitung
B, C, D = erfindungsgemäße Zubereitungen

Alle Angaben sind in Gew-%.

| **Bestandteil** | **A*** | **B** | **C** | **D** | **E** | **F** |
|---|---|---|---|---|---|---|
| Fettpulver | 8.77 | 8.77 | 8.77 | 8.77 | 8.77 | 8.77 |
| Natriumglutamat | 8.77 | 8.77 | 8.77 | 8.77 | 8.77 | 8.77 |
| Hefeextrakt Pulver | 12.28 | 12.28 | 12.28 | 12.28 | 12.28 | 12.28 |
| Kochsalz | 29.83 | 29.83 | 29.83 | 29.83 | 29.83 | 29.83 |
| Maltodextrin | 37.28 | 36.68 | 36.98 | 36.88 | 37.265 | 37.27 |
| Natürlicher Gemüseextrakt | 3.07 | 3.07 | 3.07 | 3.07 | 3.07 | 3.07 |
| Ethyl-2-(4-hydroxy-3-methoxy-phenyl)acetat (**17**) | - | 0.60 | - | - | - | - |
| Butyl-2-(4-hydroxy-3-methoxy-phenyl)acetat (**19**) | - | - | 0.30 | - | | - |
| Isobutyl-2-(4-hydroxy-3-methoxy-phenyl)acetat (**9**) | - | - | - | 0.40 | - | - |
| 3-Phenylpropyl 2-(4-hydroxy-3-methoxyphenyl)acetat (**22**) | - | - | - | - | 0.015 | - |
| [(E)-Cinnamyl] 2-(4-hydroxy-3-methoxyphenyl)acetat (**2**) | - | - | - | - | - | 0.01 |
| Total | 100 | 100 | 100 | 100 | 100 | 100 |

| | | | | | | |
|---|---|---|---|---|---|---|
| *nicht erfindungsgemäß | | | | | | |

15 g der jeweiligen Pulvermischung wurden mit je 1000 mL heißem Wasser aufgegossen.

### Anwendungsbeispiel 19: Instantsuppe, Typ Lauch-Creme

A = Vergleichszubereitung
B, C, D, E = erfindungsgemäße Zubereitungen

Alle Angaben sind in Gew-%.

| **Bestandteil** | **A*** | **B** | **C** | **D** | **E** | **F** | **G** |
|---|---|---|---|---|---|---|---|
| Kartoffelstärke | 20.00 | 20.00 | 20.00 | 20.00 | 20.00 | 20.00 | 20.00 |
| Fettpulver | 25.00 | 25.00 | 25.00 | 25.00 | 25.00 | 25.00 | 25.00 |
| Lactose | 20.00 | 20.00 | 20.00 | 20.00 | 20.00 | 20.00 | 20.00 |
| Maltodextrin | 11.73 | 11.65 | 11.68 | 11.67 | 11.66 | 11.727 | 11.725 |
| Kochsalz | 8.00 | 8.00 | 8.00 | 8.00 | 8.00 | 8.00 | 8.00 |
| Natriumglutamat | 3.00 | 3.00 | 3.00 | 3.00 | 3.00 | 3.00 | 3.00 |
| Spinatpulver | 2.00 | 2.00 | 2.00 | 2.00 | 2.00 | 2.00 | 2.00 |
| Grünes Lauchpulver | 2.00 | 2.00 | 2.00 | 2.00 | 2.00 | 2.00 | 2.00 |
| Citronensäure, als Pulver | 0.30 | 0.30 | 0.30 | 0.30 | 0.30 | 0.30 | 0.30 |
| Gehärtetes Pflanzenfett | 3.00 | 3.00 | 3.00 | 3.00 | 3.00 | 3.00 | 3.00 |
| Gefriergetrockneter Lauch | 1.00 | 1.00 | 1.00 | 1.00 | 1.00 | 1.00 | 1.00 |
| Huhnaroma | 1.00 | 1.00 | 1.00 | 1.00 | 1.00 | 1.00 | 1.00 |
| Würzmischung Typ "grüner Lauch", Pulver | 2.00 | 2.00 | 2.00 | 2.00 | 2.00 | 2.00 | 2.00 |
| Würzmischung, Typ "gekochte Zwiebel" | 0.60 | 0.60 | 0.60 | 0.60 | 0.60 | 0.60 | 0.60 |
| Hefe-Würzmischung, Typ "Gemüsebrühe", Pulver | 0.30 | 0.30 | 0.30 | 0.30 | 0.30 | 0.30 | 0.30 |
| Curcuma-Extrakt | 0.07 | 0.07 | 0.07 | 0.07 | 0.07 | 0.07 | 0.07 |
| Ethyl-2-(4-hydroxy-3-methoxyphenyl)acetat (**17**) | - | 0.08 | - | - | 0.06 | | |
| Propyl-2-(4-hydroxy-3-methoxyphenyl)acetat (**18**) | - | - | 0.05 | | | | |
| Isobutyl-2-(4-hydroxy-3-methoxyphenyl)acetat (**8**) | - | - | - | 0.06 | 0.01 | | |
| 3-Phenylpropyl 2-(4-hydroxy-3-methoxyphenyl)acetat (**22**) | | | | | | 0.003 | |
| [(E)-Cinnamyl] 2-(4-hydroxy-3-methoxyphenyl)acetat (**2**) | | | | | | | 0.005 |
| Total | 100 | 100 | 100 | 100 | 100 | 100 | 100 |

5 g der jeweiligen Pulvermischung wurden mit je 100 mL heißem Wasser aufgegossen, um eine verzehrfertige Suppe zu erhalten.

### Anwendungsbeispiel 20: Instantsuppe, Typ Hühnersuppe mit Nudeln

A = Vergleichszubereitung
B, C, D, E = erfindungsgemäße Zubereitungen

Alle Angaben sind in Gew-%.

| **Bestandteil** | **A*** | **B** | **C** | **D** | **E** | **F** | **G** |
|---|---|---|---|---|---|---|---|
| Stärke | 16.16 | 16.06 | 16.12 | 16.13 | 16.11 | 16.155 | 16.157 |
| Kochsalz | 7.00 | 7.00 | 7.00 | 7.00 | 7.00 | 7.00 | 7.00 |
| Saccharose, raffiniert | 3.20 | 3.20 | 3.20 | 3.20 | 3.20 | 3.20 | 3.20 |
| Natriumglutamat | 3.20 | 3.20 | 3.20 | 3.20 | 3.20 | 3.20 | 3.20 |
| Natriuminosinat / Natriumguanylat im Verhältnis 1:1 | 0.80 | 0.80 | 0.80 | 0.80 | 0.80 | 0.80 | 0.80 |
| Säurehydrolysiertes Pflanzenprotein | 8.00 | 8.00 | 8.00 | 8.00 | 8.00 | 8.00 | 8.00 |
| Fettpulver | 2.00 | 2.00 | 2.00 | 2.00 | 2.00 | 2.00 | 2.00 |
| Gemüsefett, sprühgetrocknet | 1.00 | 1.00 | 1.00 | 1.00 | 1.00 | 1.00 | 1.00 |
| Gefrierg etrocknetes Hühnerfleisch, in Stückchen | 2.00 | 2.00 | 2.00 | 2.00 | 2.00 | 2.00 | 2.00 |
| Suppen-Nudeln | 32.00 | 32.00 | 32.00 | 32.00 | 32.00 | 32.00 | 32.00 |
| Maltodextrin | 12.00 | 12.00 | 12.00 | 12.00 | 12.00 | 12.00 | 12.00 |
| Chinesisches Gemüse, gefriergetrocknet | 4.60 | 4.60 | 4.60 | 4.60 | 4.60 | 4.60 | 4.60 |
| Huhnaroma | 8.00 | 8.00 | 8.00 | 8.00 | 8.00 | 8.00 | 8.00 |
| Lebensmittelfarbstoff Riboflavin | 0.04 | 0.04 | 0.04 | 0.04 | 0.04 | 0.04 | 0.04 |
| Ethyl-2-(4-hydroxy-3-methoxy-phenyl)acetat (17) | - | 0.10 | - | - | 0.04 | - | - |
| Butyl-2-(4-hydroxy-3-methoxy-phenyl)acetat (19) | - | - | 0.04 | - | 0.01 | - | - |
| Hexyl-2-(4-hydroxy-3-methoxy-phenyl)acetat (21) | - | - | - | 0.031 | - | - | - |
| 3-Phenylpropyl 2-(4-hydroxy-3-methoxy-phenyl)acetat (22) | - | - | - | - | - | 0.005 | - |
| [(E)-Cinnamyl] 2-(4-hydroxy-3-methoxy-phenyl)acetat (2) | - | - | - | - | - | - | 0.003 |
| Total | 100 | 100 | 100 | 100 | 100 | 100 | 100 |

| | | | | | | | |
|---|---|---|---|---|---|---|---|
| *nicht erfindungsgemäß | | | | | | | |

4.60 g der jeweiligen Pulvermischung wurden 10 Minuten in je 100 mL Wasser gekocht, um eine verzehrfertige Suppe zu erhalten.

### Anwendungsbeispiel 21: Herstellung von dunklen Chilli-Schokoladen unter Verwendung der erfindungsgemäßen Substanzen.

Alle Angaben, soweit nicht anders vermerkt, in Gew-%.
A = Vergleichszubereitung dunkle Schokolade
B = kalorienreduzierte dunkle Schokolade
C = kalorienreduzierte dunkle Schokolade
D = kalorienreduzierte dunkle Schokolade
E = kalorienreduzierte Vollmilchschokolade
F = dunkle Schokolade
G = dunkle Schokolade

| **Bestandteil** | **A*** | **B** | **C** | **D** | **E** | **F** | **G** |
|---|---|---|---|---|---|---|---|
| Kakaobutter | 14.49 | 12.99 | 13.49 | 9.48 | 14.00 | 14.49 | 14.50 |
| Cacaomasse | 41.00 | 39.00 | 42.00 | 44.00 | 23.00 | 41.00 | 41.00 |
| Erythritol | - | 47.45 | - | - | - | - | - |
| Maltitol, kristallin | - | - | - | 23.00 | | - | - |
| Inulin | - | - | - | 23.00 | | - | - |
| Sorbitol | - | - | 44.00 | - | - | - | - |
| Lactitol | - | - | - | - | 38.55 | - | - |
| Polydextrose | - | - | - | - | 9.70 | - | - |
| Vollmilchpulver | - | - | - | - | 14.00 | - | - |
| Sucrose | 43.98 | - | - | - | - | 44.00 | 44.00 |
| Lecitin | 0.48 | 0.48 | 0.40 | 0.48 | 0.50 | 0.48 | 0.48 |
| Vanillin | 0.02 | 0.02 | 0.02 | 0.02 | 0.20 | 0.02 | 0.02 |
| Aspartam | - | 0.03 | 0.06 | - | 0.03 | - | - |
| Capsicum-Extrakt (1.000.000 SHU) | 0.03 | 0.02 | 0.02 | 0.01 | - | 0.01 | - |
| Hexyl-2-(4-hydroxy-3-methoxy-phenyl)acetat (**21**) | - | 0.01 | - | 0.01 | 0.01 | - | - |
| Butyl-2-(4-hydroxy-3-methoxy-phenyl)acetat (**19**) | - | - | 0.01 | - | 0.01 | - | - |
| 3-Phenylpropyl 2-(4-hydroxy-3-methoxyphenyl)acetat (**22**) | - | - | - | - | - | 0.001 | - |
| [(E)-Cinnamyl] 2-(4-hydroxy-3-methoxyphenyl)acetat (**2**) | - | - | - | - | - | - | 0.002 |
| Total | 100 | 100 | 100 | 100 | 100 | 100 | 100 |

| | | | | | | | |
|---|---|---|---|---|---|---|---|
| *nicht erfindungsgemäß | | | | | | | |

Die in den vorstehenden Anwendungsbeispielen gefundenen Effekte lassen sich - gegebenenfalls durch vom Fachmann unschwer vorzunehmende Modifikationen - auf alle Produkte der jeweiligen Produktgruppe, d.h. insbesondere auf Zahnpasten, Kaugummis, Mundwässer, Halsbonbons, Gelatinekapseln, Kaubonbons und Tee in Beuteln übertragen. Dabei ist es für den Fachmann aufgrund der vorliegenden Beschreibung unschwer erkennbar, dass ohne großen Aufwand die erfindungsgemäßen Verbindungen und Mischungen - eventuell unter geringfügigen Modifikationen - untereinander austauschbar sind. Das bedeutet, dass die in den Produkten der Anwendungsbeispiele verwendete erfindungsgemäße Verbindung als Platzhalter auch für die anderen erfindungsgemäßen Verbindungen und Mischungen aufgefasst werden muss. Auch die Konzentration der verwendeten erfindungsgemäßen Verbindung oder Mischung ist für den Fachmann leicht erkenntlich variierbar. Außerdem sind die produktspezifischen weiteren Bestandteile in dem jeweiligen Anwendungsbeispiel für den Fachmann leicht nachvollziehbar ebenfalls gegen weitere produkttypische Bestandteile austauschbar bzw. durch solche ergänzbar. Eine Vielzahl solcher produktspezifischen Bestandteile ist in der oben stehenden Beschreibung offenbart.

## Patentansprüche

1. Verwendung einer Verbindung der Formel (I) 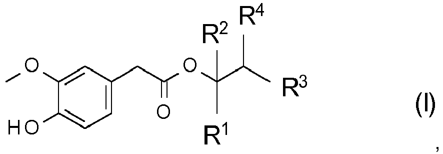 wobei
(i) R¹ und R² unabhängig voneinander ein Wasserstoffatom oder einen Alkylrest mit 1-2 Kohlenstoffatomen darstellen,
R³ und R⁴ unabhängig voneinander ein Wasserstoffatom oder einen linearen oder verzweigten Alkylrest mit 1 bis 5 Kohlenstoffatomen, einen Phenylrest, einen Alkylphenylrest oder einen Phenylalkylrest oder einen linearen oder verzweigten Alkenylrest mit 2 bis 4 Kohlenstoffatomen oder einen Alkenylphenylrest oder einen Phenylalkenylrest darstellen,
oder
(ii) R¹ und R³ zusammen mit den sie verknüpfenden Kohlenstoffatomen einen Cyclohexylring bilden, der optional mit einem zusätzlichen Rest R⁵ substituiert ist, wobei R⁵ ein Alkylrest mit 1-2 Kohlenstoffatomen ist,
R² ein Wasserstoffatom oder einen Alkylrest mit 1-2 Kohlenstoffatomen darstellt,
R⁴ ein Wasserstoffatom oder einen linearen oder verzweigten Alkylrest mit 1 bis 5 Kohlenstoffatomen, einen Phenylrest, einen Alkylphenylrest oder einen Phenylalkylrest oder einen linearen oder verzweigten Alkenylrest mit 2 bis 4 Kohlenstoffatomen oder einen Alkenylphenylrest oder einen Phenylalkenylrest darstellt,
oder eines physiologisch akzeptablen Salzes davon, wobei die phenolische Hydroxygruppe in Formel (I) deprotoniert ist, oder einer Mischung umfassend eine oder mehrere unterschiedliche Verbindungen der Formel (I) und/oder physiologisch akzeptable Salze davon, wobei jeweils die phenolische Hydroxygruppe in Formel (I) deprotoniert ist, oder bestehend aus mehreren unterschiedlichen Verbindungen der Formel (I) und/oder Salzen davon, wobei jeweils die phenolische Hydroxygruppe in Formel (I) deprotoniert ist,
als Aromastoff bzw. Scharfstoff mit einem wärme- und/oder schärfeerzeugenden Effekt,
und als Aromastoff zum Verstärken eines angenehmen Geschmackseindrucks, ausgewählt aus der Gruppe bestehend aus wärmend, scharf und kühlend.

2. Verwendung nach Anspruch 1, wobei für die Verbindung der Formel (I) bzw. eine, mehrere oder sämtliche Verbindungen der Formel (I) unabhängig voneinander in der Mischung gilt:
(i) R¹ und R² stellen unabhängig voneinander ein Wasserstoffatom oder Methyl dar,
R³ und R⁴ stellen unabhängig voneinander ein Wasserstoffatom oder einen linearen oder verzweigten Alkylrest mit 1 bis 5 Kohlenstoffatomen oder einen Phenylrest, einen Alkylphenylrest oder einen Phenylalkylrest oder einen Alkenylphenylrest oder einen Phenylalkenylrest dar,
oder
(ii) Formel (I) entspricht der folgenden Formel (la) 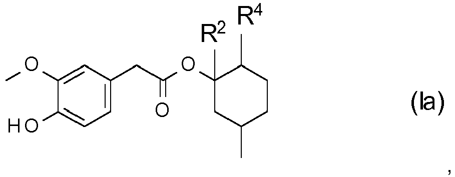 R² stellt ein Wasserstoffatom dar,
R⁴ stellt 2-Propyl dar.

3. Verwendung nach einem der vorangehenden Ansprüche, wobei für die Verbindung der Formel (I) bzw. eine, mehrere oder sämtliche Verbindungen der Formel (I) unabhängig voneinander in der Mischung gilt:
R¹ und R² stellen jeweils ein Wasserstoffatom dar,
R³ stellt ein Wasserstoffatom oder einen linearen oder verzweigten Alkylrest mit 1 bis 4 Kohlenstoffatomen oder einen Phenylrest, einen Alkylphenylrest oder einen Phenylalkylrest oder einen Alkenylphenylrest oder einen Phenylalkenylrest dar,
R⁴ stellt ein Wasserstoffatom dar.

4. Verwendung nach einem der vorangehenden Ansprüche, wobei die Verbindung der Formel (I) bzw. eine, mehrere oder sämtliche Verbindungen der Formel (I) in der Mischung ausgewählt ist bzw. sind aus der Gruppe bestehend aus
2-Phenylethyl-2-(4-hydroxy-3-methoxy-phenyl)acetat (**1**) 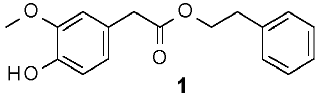
[(*E*)-Cinnamyl]-2-(4-hydroxy-3-methoxy-phenyl)acetat (**2**) 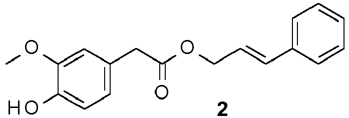
1-Ethylbutyl-2-(4-hydroxy-3-methoxy-phenyl)acetat (**3**) 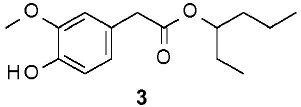
3-Methylbut-2-enyl-2-(4-hydroxy-3-methoxy-phenyl)acetat (**4**) 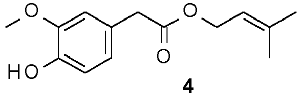
[(*E*)-Hex-2-enyl]-2-(4-hydroxy-3-methoxy-phenyl)acetat (**5**) 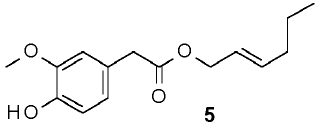
[(Z)-Hex-3-enyl]-2-(4-hydroxy-3-methoxy-phenyl)acetat (**6**) 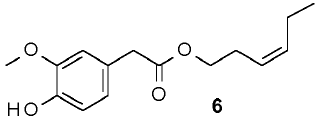
Isopropyl-2-(4-hydroxy-3-methoxy-phenyl)acetat (**7**) 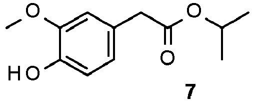
sec-Butyl-2-(4-Hydroxy-3-methoxy-phenyl)acetat (**8**) 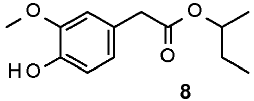
Isobutyl-2-(4-hydroxy-3-methoxy-phenyl)acetat (**9**) 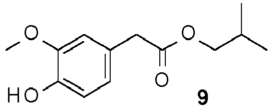
1,1-Dimethylpropyl 2-(4-hydroxy-3-methoxy-phenyl)acetat (**10**) 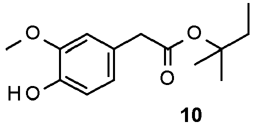
Isopentyl-2-(4-hydroxy-3-methoxy-phenyl)acetat (**11**) 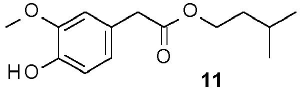
2-Methylbutyl-2-(4-hydroxy-3-methoxy-phenyl)acetat (**12**) 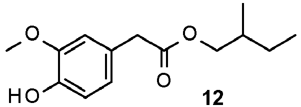
1-Methylpentyl-2-(4-hydroxy-3-methoxy-phenyl)acetat (**13**) 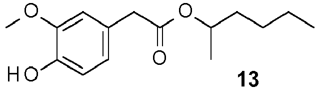
Heptyl-2-(4-hydroxy-3-methoxy-phenyl)acetat **(14)** 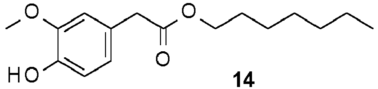
1-Methylhexyl-2-(4-hydroxy-3-methoxy-phenyl)acetat (**15**) 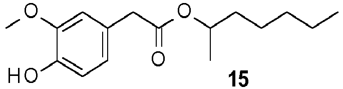
(2-Isopropyl-5-methyl-cyclohexyl)-2-(4-hydroxy-3-methoxy-phenyl)acetat (**16**) 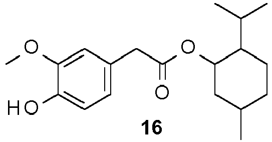
Ethyl-2-(4-hydroxy-3-methoxy-phenyl)acetat (**17**) 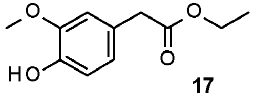
Propyl-2-(4-hydroxy-3-methoxy-phenyl)acetat (**18**) 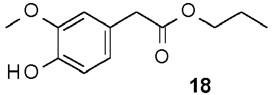
Butyl-2-(4-hydroxy-3-methoxy-phenyl)acetat (**19**) 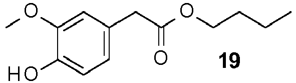
Pentyl-2-(4-hydroxy-3-methoxy-phenyl)acetat (**20**) 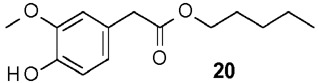
Hexyl-2-(4-hydroxy-3-methoxy-phenyl)acetat (**21**) 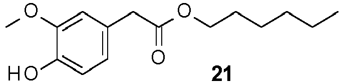
3-Phenylpropyl-2-(4-hydroxy-3-methoxy-phenyl)acetat (**22**) 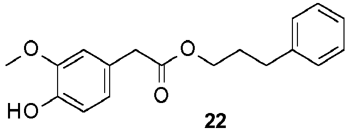
4-Phenylbutyl-2-(4-hydroxy-3-methoxy-phenyl)acetat (**23**)

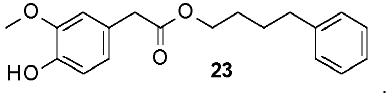

5. Verwendung nach einem der vorangehenden Ansprüche in einer pharmazeutischen, einer der Ernährung, der Mundhygiene oder dem Genuss dienenden Zubereitung, vorzugsweise wobei die Gesamtmenge an Verbindung(en) der Formel (I) und/oder Salz(en) davon in der Zubereitung ausreicht, um
bei Gebrauch oder Verzehr der Zubereitung sensorisch einen wärmenden und/oder scharfen Effekt auf der Zunge oder im Mundraum hervorzurufen,
und einen angenehmen Geschmackseindruck, ausgewählt aus der Gruppe bestehend aus wärmend, scharf und kühlend, zu verstärken.

6. Verbindung der Formel (I) oder physiologisch akzeptables Salz davon, wobei die phenolische Hydroxygruppe in Formel (I) deprotoniert ist, oder Mischung umfassend eine oder mehrere unterschiedliche Verbindungen der Formel (I) und/oder ein oder mehrere physiologisch akzeptable Salze davon, wobei die phenolische Hydroxygruppe in Formel (I) jeweils deprotoniert ist, oder bestehend aus mehreren unterschiedlichen Verbindungen der Formel (I) und/oder physiologisch akzeptablen Salzen davon, wobei die phenolische Hydroxygruppe in Formel (I) jeweils deprotoniert ist, 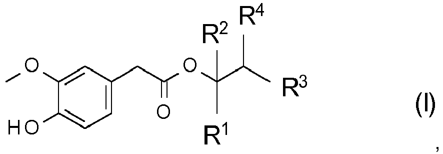 wobei
(i) R¹ und R² unabhängig voneinander ein Wasserstoffatom oder einen Alkylrest mit 1-2 Kohlenstoffatomen darstellen,
R³ und R⁴ unabhängig voneinander ein Wasserstoffatom oder einen linearen oder verzweigten Alkylrest mit 1 bis 5 Kohlenstoffatomen, einen Phenylrest, einen Alkylphenylrest oder einen Phenylalkylrest oder einen linearen oder verzweigten Alkenylrest mit 2 bis 4 Kohlenstoffatomen oder einen Alkenylphenylrest oder einen Phenylalkenylrest darstellen,
oder
(ii) R¹ und R³ zusammen mit den sie verknüpfenden Kohlenstoffatomen einen Cyclohexylring bilden, der optional mit einem zusätzlichen Rest R⁵ substituiert ist, wobei R⁵ ein Alkylrest mit 1-2 Kohlenstoffatomen ist,
R² ein Wasserstoffatom oder einen Alkylrest mit 1-2 Kohlenstoffatomen darstellt,
R⁴ ein Wasserstoffatom oder einen linearen oder verzweigten Alkylrest mit 1 bis 5 Kohlenstoffatomen, einen Phenylrest, einen Alkylphenylrest oder einen Phenylalkylrest oder einen linearen oder verzweigten Alkenylrest mit 2 bis 4 Kohlenstoffatomen oder einen Alkenylphenylrest oder einen Phenylalkenylrest darstellt,
mit der Maßgabe, dass
die Verbindung der Formel (I) bzw. eine, mehrere oder sämtliche Verbindungen der Formel (I) in der Mischung ausgewählt ist bzw. sind aus der Gruppe bestehend aus
[(*E*)-Cinnamyl]-2-(4-hydroxy-3-methoxy-phenyl)acetat (**2**) 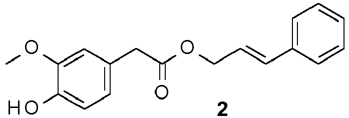
1-Ethylbutyl-2-(4-hydroxy-3-methoxy-phenyl)acetat (**3**) 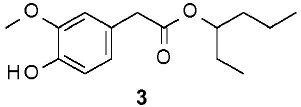
3-Methylbut-2-enyl-2-(4-hydroxy-3-methoxy-phenyl)acetat (**4**) 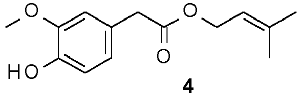
[(*E*)-Hex-2-enyl]-2-(4-hydroxy-3-methoxy-phenyl)acetat (**5**) 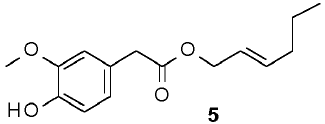
[(*Z*)-Hex-3-enyl]-2-(4-hydroxy-3-methoxy-phenyl)acetat (**6**) 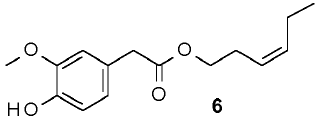
1,1-Dimethylpropyl 2-(4-hydroxy-3-methoxy-phenyl)acetat (**10**) 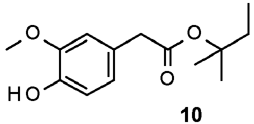
2-Methylbutyl-2-(4-hydroxy-3-methoxy-phenyl)acetat **(12)** 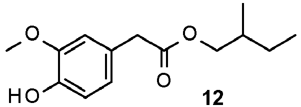
1-Methylpentyl-2-(4-hydroxy-3-methoxy-phenyl)acetat (**13**) 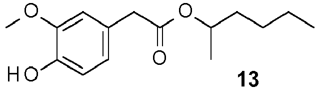
1-Methylhexyl-2-(4-hydroxy-3-methoxy-phenyl)acetat (**15**) 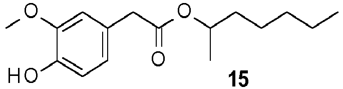
(2-Isopropyl-5-methyl-cyclohexyl)-2-(4-hydroxy-3-methoxy-phenyl)acetat (**16**) 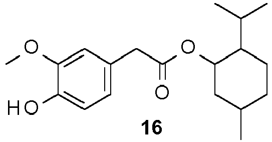
4-Phenylbutyl 2-(4-hydroxy-3-methoxy-phenyl)acetat (**23**)

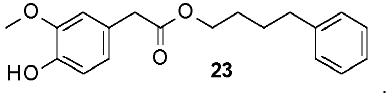

7. Aromakomposition
umfassend oder bestehend aus einer Mischung nach Anspruch 6, vorzugsweise wobei
die Gesamtmenge an Verbindung(en) der Formel (I) und/oder Salz(en) davon in der Aromakomposition, bezogen auf das Gesamtgewicht der Aromakomposition, im Bereich von 100 bis 100.000 mg/kg liegt, vorzugsweise im Bereich von 250 bis 40.000 mg/kg, besonders bevorzugt im Bereich von 250 bis 15.000 mg/kg.

8. Aromakomposition nach Anspruch 7,
wobei
die Gesamtmenge an Verbindung(en) der Formel (I) und/oder Salz(en) davon in der Aromakomposition, bezogen auf das Gesamtgewicht der Aromakomposition, im Bereich von 100 bis 100.000 mg/kg liegt, vorzugsweise im Bereich von 250 bis 40.000 mg/kg, besonders bevorzugt im Bereich von 250 bis 15.000 mg/kg,
zudem umfassend einen oder mehrere weitere, nicht der Formel (I) entsprechende Aromastoffe, vorzugsweise ausgewählt aus der Gruppe bestehend aus
a) Wärme verursachende oder Scharfstoffe, bevorzugt ausgewählt aus der Liste bestehend aus: Capsaicinoiden, wie zum Beispiel Capsaicin, Dihydrocapsaicin oder Nonivamid; Gingerolen, wie zum Beispiel Gingerol-6, Gingerol-8, oder Gingerol-10; Shogaolen wie Shogaol-6, Shogaol-8, Shogaol-10; Gingerdionen, wie zum Beispiel Gingerdion-6, Gingerdion-8 oder Gingerdion-10; Paradolen, wie zum Beispiel Paradol-6, Paradol-8 oder Paradol-10; Dehydrogingerdionen, wie zum Beispiel Dehydrogingerdion-6, Dehydrogingerdion-8 oder Dehydrogingerdion-10; Piperin und Piperinderivaten;
b) als stechend oder beißend wahrnehmbare Stoffe, bevorzugt ausgewählt aus der Gruppe bestehend aus: aromatischen Isothiocyanaten, wie zum Beispiel Phenylethylisothiocyanat, Allylisothiocyanat, Cyclopropylisothiocyanat, Butylisothiocyanat, 3-Methylthiopropylisothiocyanat, 4-Hydroxybenzylisothiocyanat, 4-Methoxybenzylisothiocyanat;
c) als kribbelnd (tingling) beschriebenen Alkamide, vorzugsweise ausgewählt aus der Gruppe bestehend aus 2E,4E-Decadiensäure-N-isobutylamid (trans-Pellitorin) (insbesondere solche wie beschrieben in WO 2004/043906, die bezüglich der darin offenbarten entsprechenden Verbindungen auf dem Wege der Verweisung Bestandteil dieser Anmeldung wird); 2E,4Z- Decadiensäure-N-isobutylamid (cis-Pellitorin) (insbesondere solche wie beschrieben in WO 2004/000787, die bezüglich der darin offenbarten entsprechenden Verbindungen auf dem Wege der Verweisung Bestandteil dieser Anmeldung wird); 2Z,4Z- Decadiensäure-N-isobutylamid; 2Z,4E- Decadiensäure-N-isobutylamid; 2E,4E-Decadiensäure-N-([2S]-2-methylbutyl)amid; 2E,4E-Decadiensäure-N-([2S]-2-methylbutyl)amid; 2E,4E-Decadiensäure-N-([2R]-2-methylbutylamid); 2E,4Z-Decadiensäure-N-(2-methylbutyl)amid; 2E,4E-Decadiensäure-N-piperid (Achilleamid); 2E,4E-Decadiensäure-N-piperid (Sarmentin); 2E-Decensäure-N-isobutylamid; 3E-Decensäure-N-isobutylamid; 3E-Nonensäure-N-isobutylamid; 2E,6Z,8E-Decatriensäure-N-isobutylamid (Spilanthol); 2E,6Z,8E-Decatriensäure-N-([2S]-2-methylbutyl)amid (Homospilanthol); 2E,6Z,8E-Decatriensäure-N-([2R]-2-methylbutyl)amid; 2E-Decen-4-insäure-N-isobutylamid; 2Z-Decen-4-insäure-N-isobutylamid; 2E,6Z,8E,10E-Dodecatetraensäure-N-(2-methylpropyl)amid (alpha-Sanshool); 2E,6Z,8E,10E-Dodecatetraensäure-N-(2-hydroxy-2-methylpropyl)amid (alpha-Hydroxysanshool); 2E,6E,8E,10E-Dodecatetraensäure-N-(2-hydroxy-2-methyl propyl)amid (gamma-Hydroxysanshool); 2E, 4E, 8Z, 10E, 12E-Tetradecapentaensäure-N-(2-hydroxy-2-methylpropyl)amid (gamma-Hydroxysanshool); 2E, 4E, 8E,10E, 12E-Tetradecapentaensäure-N-(2-hydroxy-2-methylpropyl)amid (gamma-Hydroxyisosanshool); 2E, 4E, 8Z, 10E, 12E-Tetradecapentaensäure-N-(2-methyl-2-propenyl)amid (gamma-Dehydrosanshool); 2E, 4E, 8Z, 10E, 12E-Tetradecapentaensäure-N-(2-methylpropyl)amid (gamma-Sanshool); 2E,4E,8Z,11Z-Tetradecatetraensäure-N-(2-hydroxy-2-methylpropyl)amid (Bungeanool); 2E,4E,8Z, 11E-Tetradecatetraensäure-N-(2-hydroxy-2-methylpropyl)amid (Isobungeanool); 2E,4E,8Z-Tetradecatriensäure-N-(2-hydroxy-2-methylpropyl)amid (Dihydrobungeanool) und 2E,4E -Tetradecadiensäure-N-(2-hydroxy-2-methylpropyl)amid (Tetrahydrobungeanool);
d) Stoffe mit physiologischer Kühlwirkung, vorzugsweise ausgewählt aus der folgenden Liste: Menthol und Mentholderivate (z.B. L-Menthol, D-Menthol, racemisches Menthol, Isomenthol, Neoisomenthol, Neomenthol) Menthylether (z.B. (L-Menthoxy)-1,2-propandiol, (L-Menthoxy)-2-methyl-1,2-propandiol, L-Menthyl-methylether), Menthylester (z.B. Menthylformiat, Menthylacetat, Menthylisobutyrat, Menthyllactate, L-Menthyl-L-lactat, L-Menthyl-D-lactat, Menthyl-(2-methoxy)acetat, Menthyl-(2-methoxyethoxy)acetat, Menthylpyroglutamat), Menthylcarbonate (z.B. Menthylpropylenglycolcarbonat, Menthylethylenglycolcarbonat, Menthylglycerincarbonat oder deren Gemischen), die Halbester von Mentholen mit einer Dicarbonsäure oder deren Derivate (z.B. Mono-Menthylsuccinat, Mono-Menthylglutarat, Mono-Menthylmalonat, O-Menthylbernsteinsäureester-N,N-(dimethyl)amid, O-Menthylbernsteinsäureesteramid), andere als in der vorliegenden Erfindung genannte Menthancarbonsäureamide (z.B. Menthancarbonsäure-N-ethylamid [WS3], Menthancarbonsäure-N-(p-methoxyphenyl)amid [SC1], Nα-(Menthancarbonyl)glycinethylester [WS5], Menthancarbonsäure-N-(4-cyanophenyl)amid, Menthancarbonsäure-N-(alkoxyalkyl)amide), Menthon und Menthonderivate (z.B. L-Menthonglycerinketal), 2,3-Dimethyl-2-(2-propyl)-butansäurederivate (z.B. 2,3-Dimethyl-2-(2-propyl)-butansäure-N-methylamid [WS23]), Isopulegol oder seine Ester (I-(-)-Isopulegol, I-(-)-Isopulegolacetat), Menthanderivate (z.B. p-Menthan-3,8-diol), Cubebol oder synthetische oder natürliche Mischungen, enthaltend Cubebol, Pyrrolidonderivate von Cycloalkyldionderivaten (z.B. 3-Methyl-2(1-pyrrolidinyl)-2-cyclopenten-1-on) oder Tetrahydropyrimidin-2-one (z.B. Icilin oder verwandte Verbindungen, wie in WO 2004/026840 beschrieben), weitere Kühlwirkstoffe wie in WO2011061330 beschrieben, insbesondere Derivate verschieden substituierter Zimt- und 2-Phenoxysäuren, besonders bevorzugt Methylendioxyzimtsäure-N,N-diphenylamid, Methylendioxyzimtsäure-N-ethyl-N-phenylamid, Methylendioxyzimt-säure-N-pyridyl-N-phenylamid;
e) Stoffe mit adstringierender Wirkung, vorzugsweise ausgewählt aus der folgenden Liste: Catechine wie z.B. Epicatechine, Gallocatechine, Epigallocatechine sowie deren jeweiligen Gallussäureester, z.B. Epigallocatechingallat oder Epicatechingallat, dern Oligomere (Procyanidine, Proanthocyanidine, Prodelphinidine, Procyanirine, Thearubigenine, Theogalline) sowie deren C- und O-Glycoside; Dihydroflavonoide wie Dihydromyricetin, Taxifolin, sowie deren C- und O-Glycoside, Flavonole wie Myricetin, Quercetin sowie deren C- und O-Glycoside wie Quercetrin, Rutin, Gallussäaureester von Kohlenhydraten wie Tannin, Pentagalloylglucose oder deren Reaktionsprodukte wie Elligatannin, Aluminiumsalze, z.B. Alaun.

9. Aromakomposition nach Anspruch 7 oder 8, zudem umfassend einen oder mehrere nicht der Formel (I) entsprechende Stoffe mit unangenehmer, insbesondere bitterer Geschmacksqualität, oder einer adstringierenden, bitteren, trockenen, staubigen, mehligen, kalkigen und/oder metallischen Note, vorzugsweise ausgewählt aus der Gruppe bestehend aus:
f) Xanthinalkaloide, Xanthine (Coffein, Theobromin, Theophyllin und Methylxanthine), Alkaloide (Chinin, Brucin, Strychnin, Nicotin), phenolische Glycoside (z.B. Salicin, Arbutin), Flavonoidglycoside (z.B. Neohespereidin, Hesperidin, Naringin, Quercitrin, Rutin, Hyperosid, Quercetin-3-O-glucosid, Myricetin-3-O-glycoside), Chalcone oder Chalconglycoside (z.B. Phloridzin, Phloridzinxyloside), hydrolisierbare Tannine (Gallus- oder Elagsäureester von Kohlenhydraten, z.B. Pentagalloylglucose, Tanninsäuren), nichthydrolisierbare Tannine (ggfs. galloylierte Catechine, Gallocatechine, Epigallocatechine oder Epicatechine und deren Oligomeren, z.B. Proanthyocyanidine oder Procyanidine, Thearubigenin), Flavone (z.B. Quercetin, Taxifolin, Myricetin), Phenole wie z.B. Salicin, Polyphenole (z.B. gamma-Oryzanol, Kaffeesäure oder deren Ester (z. B. Chlorogensäure und Isomere)), terpenoide Bitter- und Gerbstoffe (z.B. Limonoide wie Limonin oder Nomilin aus Zitrusfrüchten, Lupolone und Humolone aus Hopfen, Iridoide, Secoiridoide), Absinthin aus Wermut, Amarogentin aus Enzian, metallische Salze (insbesondere Kalium-, Magnesium- und Calciumsalze, Kaliumchlorid, Kaliumgluconat, Kaliumcarbonat, Kaliumsulfat, Kaliumlactat, Kaliumglutamat, Kaliumsuccinat, Kaliummalat, Natriumsulfat, Magnesiumsulfat, Aluminiumsalze, Zinksalze, Zinnsalze, Eisen-(II)-salze, Eisen-(III)-salze, Chrom-(II)-picolinat), pharmazeutische Wirkstoffe (z.B. Fluorchinolon-Antibiotika, Paracetamol, Aspirin, beta-Lactam-Antibiotika, Ambroxol, Propylthiouracil [PROP], Guaifenesin), Vitamine (beispielsweise Vitamin H, Vitamine aus der B-Reihe wie Vitamin B1, B2, B6, B12, Niacin, Panthotensäure), Denatoniumbenzoat, Sucraloseoctaacetat, Eisensalze, Aluminiumsalze, Zinksalze, Harnstoff, ungesättigte Fettsäuren, insbesondere ungesättigte Fettsäuren in Emulsionen, bitter/adstringierend schmeckende Aminosäuren (z.B. Leucin, Isoleucin, Valin, Tryptophan, Prolin, Histidin, Tyrosin, Lysin oder Phenylalanin) und bitter oder adstringierend schmeckende Peptide oder Proteine (insbesondere Peptide mit einer Aminosäure aus der Gruppe Leucin, Isoleucin, Valin, Tryptophan, Prolin oder Phenylalanin am N- oder C-Terminus), Saponine, insbes. Sojasaponine, Isoflavonoide (insbes. Genistein, Daidzein, Genistin, Daidzin, deren Glycoside und acylierten Glycoside);
g) Stoffe mit einem nicht unangenehmen Primärgeschmack (beispielsweise süß, salzig, würzig, sauer) und/oder -geruch, vorzugsweise ausgewählt aus der Gruppe der Süßstoffe oder Zuckeraustauschstoffe, vorzugsweise Kaliumsalze (insbesondere Kaliumchlorid, Kaliumgluconat, Kaliumcarbonat, Kaliumsulfat, Kaliumlactat, Kaliumglutamat, Kaliumsuccinat, Kaliummalat), Aspartam, Acesulfam K, Neotam, Superaspartam, Saccharin, Sucralose, Tagatose, Monellin, Stevioside, Rebaudioside, Rebaudiosid A, Rebaudiosid B, Rebaudiosid C, Rebaudiosid D, Rebaudiosid X, Rubusoside, Hernandulcin, Thaumatin, Miraculin, Glycyrrhizin, Glycyrrhetinsäure, Balansin A oder Balansin B, oder deren Derivate, Cyclamat oder die pharmazeutisch akzeptablen Salze der vorgenannten Verbindungen.

10. Pharmazeutische Zubereitung, der Ernährung, der Mundhygiene oder dem Genuss dienende Zubereitung, umfassend
(A) eine Mischung nach Anspruch 6,
vorzugsweise wobei
die Gesamtmenge an Verbindung(en) der Formel (I) und/oder Salz(en) davon in der Zubereitung, bezogen auf das Gesamtgewicht der Zubereitung, im Bereich von 0,1 bis 1.000 mg/kg liegt, vorzugsweise im Bereich von 1 bis 1.000 mg/kg, vorzugsweise im Bereich von 1 bis 750 mg/kg, besonders bevorzugt im Bereich von 5 bis 500 mg/kg,
oder
(C) eine Aromakomposition nach Anspruch 7, 8 oder 9,
vorzugsweise wobei
die Gesamtmenge an Verbindung(en) der Formel (I) und/oder Salz(en) davon in der Zubereitung, bezogen auf das Gesamtgewicht der Zubereitung, im Bereich von 0,1 bis 1.000 mg/kg liegt, vorzugsweise im Bereich von 1 bis 1.000 mg/kg, vorzugsweise im Bereich von 1 bis 750 mg/kg, besonders bevorzugt im Bereich von 5 bis 500 mg/kg,
wobei die Gesamtmenge an Verbindung(en) der Formel (I) und/oder Salz(en) davon in der Zubereitung ausreicht, um
bei Gebrauch oder Verzehr der Zubereitung sensorisch einen wärmenden und/oder scharfen Effekt auf der Zunge oder im Mundraum hervorzurufen,
und einen angenehmen Geschmackseindruck ausgewählt aus der Gruppe bestehend aus wärmend, scharf und kühlend, zu verstärken.

11. Zubereitung nach Anspruch 10, zudem umfassend
ein oder mehrere übliche Grund-, Hilfs- und Zusatzstoffe in einer Menge von, bezogen auf das Gesamtgewicht der Zubereitung, 5 bis 99,9999 Gew.-%, vorzugsweise 10 bis 80 Gew.-%,
und/oder
Wasser in einer Menge, bezogen auf das Gesamtgewicht der Zubereitung, bis zu 99,9999 Gew.-%, vorzugsweise in einer Menge von 5 bis 80 Gew.-%.

12. Verfahren zum Herstellen einer Zubereitung nach einem der Ansprüche 10 bis 11, umfassend folgende Schritte:
i) Bereitstellen
(A) einer Mischung nach Anspruch 6,
vorzugsweise wobei
die Gesamtmenge an Verbindung(en) der Formel (I) und/oder Salz(en) davon so ausgewählt ist, dass die Gesamtmenge in der herzustellenden Zubereitung, bezogen auf das Gesamtgewicht der Zubereitung, im Bereich von 0,1 bis 1.000 mg/kg liegt, vorzugsweise im Bereich von 1 bis 1.000 mg/kg, vorzugsweise im Bereich von 1 bis 750 mg/kg, besonders bevorzugt im Bereich von 5 bis 500 mg/kg,
oder
(C) eine Aromakomposition nach Anspruch 7, 8 oder 9,
vorzugsweise wobei
die Gesamtmenge an Verbindung(en) der Formel (I) und/oder Salz(en) so ausgewählt ist, dass die Gesamtmenge in der herzustellenden Zubereitung, bezogen auf das Gesamtgewicht der Zubereitung, im Bereich von 0,1 bis 1.000 mg/kg liegt, vorzugsweise im Bereich von 1 bis 1.000 mg/kg, vorzugsweise im Bereich von 1 bis 750 mg/kg, besonders bevorzugt im Bereich von 5 bis 500 mg/kg,
ii) Bereitstellen eines oder mehrerer weiterer Bestandteile der herzustellenden Zubereitung, und
iii) Inkontaktbringen oder Mischen der in Schritt ii) bereitgestellten weiteren Bestandteile mit dem/den in Schritt i) bereitgestellten Bestandteil(en), vorzugsweise in einer sensorisch wirksamen Menge.

## Claims

1. Use of a compound of the formula (I) 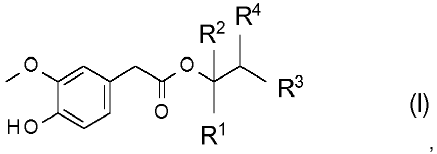 wherein
(i) R¹ and R² represent, independently of each other, a hydrogen atom or an alkyl residue with 1-2 carbon atoms,
R³ and R⁴ represent, independently of each other, a hydrogen atom or a linear or branched alkyl residue with 1 to 5 carbon atoms, a phenyl residue, an alkylphenyl residue or a phenylalkyl residue or a linear or branched alkenyl residue with 2 to 4 carbon atoms or an alkenylphenyl residue or a phenylalkenyl residue,
or
(ii) R¹ and R³ along with the carbon atoms linking them form a cyclohexyl ring, which optionally is substituted with an additional residue R⁵, wherein R⁵ is an alkyl residue with 1-2 carbon atoms,
R² represents a hydrogen atom or an alkyl residue with 1-2 carbon atoms, R⁴ represents a hydrogen atom or a linear or branched alkyl residue with 1 to 5 carbon atoms, a phenyl residue, an alkylphenyl residue or a phenylalkyl residue or a linear or branched alkenyl residue with 2 to 4 carbon atoms or an alkenylphenyl residue or a phenylalkenyl residue,
or of a physiologically acceptable salt thereof, wherein the phenolic hydroxy group in formula (I) is deprotonated, or of a mixture, comprising one or several different compounds of formula (I) and/or physiologically acceptable salts thereof, wherein each phenolic hydroxy group in formula (I) is deprotonated, or consisting of a plurality of different compounds of formula (I) and/or salts thereof, wherein each phenolic hydroxy group in formula (I) is deprotonated,
as flavouring or, respectively, pungent substance that creates a warm and/or pungent effect,
and as flavouring for enhancing a pleasant taste sensation selected from the group consisting of warming, pungent and cooling.

2. Use according to claim 1, wherein the following applies to the compound of formula (I) or, respectively, one, several or all of the compounds of formula (I), independently of each other, in the mixture:
(i) R¹ and R² represent, independently of each other, a hydrogen atom or methyl group,
R³ and R⁴ represent, independently of each other, a hydrogen atom or a linear or branched alkyl residue with 1 to 5 carbon atoms or a phenyl residue, an alkylphenyl residue or a phenylalkyl residue or an alkenylphenyl residue or a phenylalkenyl residue,
or
(ii) formula (I) corresponds to the following formula (Ia) 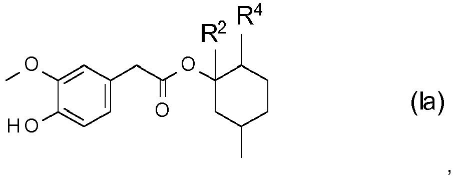 R² represents a hydrogen atom,
R⁴ represents 2-propyl.

3. Use according to any one of the preceding claims, wherein the following applies to the compound of formula (I) or, respectively, one, several or all of the compounds of formula (I), independently of each other, in the mixture:
R¹ and R² each represent a hydrogen atom,
R³ represents a hydrogen atom or a linear or branched alkyl residue with 1 to 4 carbon atoms or a phenyl residue, an alkylphenyl residue or a phenylalkyl residue or an alkenylphenyl residue or a phenylalkenyl residue,
R⁴ represents a hydrogen atom.

4. Use according to any one of the preceding claims, wherein the compound of formula (I) or, respectively, one or several or all compounds of the formula (I) in the mixture is or are selected from the group consisting of
2-phenylethyl-2-(4-hydroxy-3-methoxyphenyl) acetate (**1**) 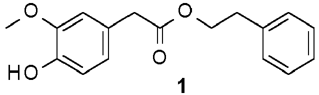
[(*E*)-cinnamyl]-2-(4-hydroxy-3-methoxyphenyl) acetate (**2**) 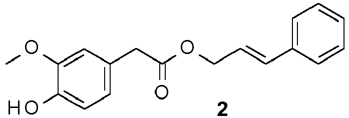
1-ethylbutyl-2-(4-hydroxy-3-methoxyphenyl) acetate (**3**) 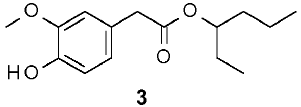
3-methylbut-2-enyl-2-(4-hydroxy-3-methoxyphenyl) acetate (**4**) 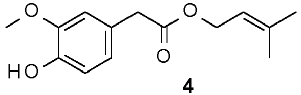
[(*E*)-hex-2-enyl]-2-(4-hydroxy-3-methoxyphenyl) acetate (**5**) 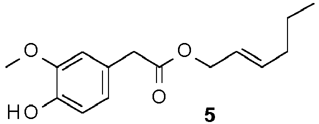
[(Z)-hex-3-enyl]-2-(4-hydroxy-3-methoxyphenyl) acetate (**6**) 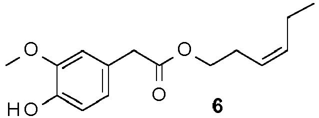
isopropyl-2-(4-hydroxy-3-methoxy-phenyl) acetate (**7**) 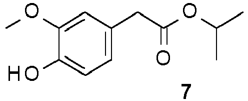
sec-butyl-2-(4-hydroxy-3-methoxyphenyl) acetate (**8**) 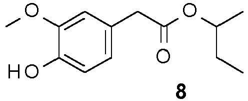
isobutyl-2-(4-hydroxy-3-methoxy-phenyl) acetate (**9**) 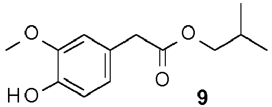
1,1-dimethylpropyl 2-(4-hydroxy-3-methoxyphenyl) acetate (**10**) 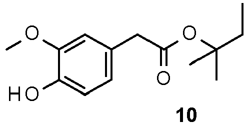
isopentyl-2-(4-hydroxy-3-methoxyphenyl) acetate (**11**) 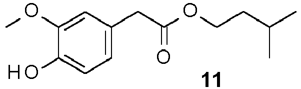
2-methylbutyl-2-(4-hydroxy-3-methoxyphenyl) acetate (**12**) 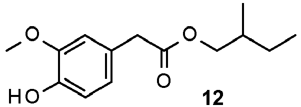
1-methylpentyl-2-(4-hydroxy-3-methoxyphenyl) acetate (**13**) 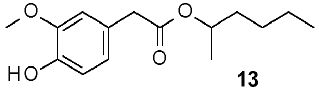
heptyl-2-(4-hydroxy-3-methoxyphenyl) acetate (**14**) 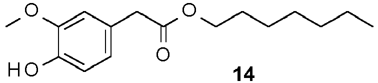
1-methylhexyl-2-(4-hydroxy-3-methoxyphenyl) acetate (**15**) 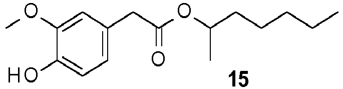
(2-isopropyl-5-methyl-cyclohexyl)-2-(4-hydroxy-3-methoxyphenyl) acetate (**16**) 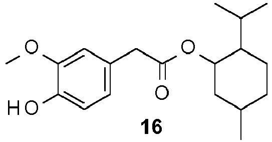
ethyl-2-(4-hydroxy-3-methoxyphenyl) acetate (**17**) 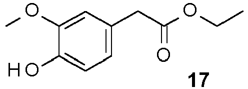
propyl-2-(4-hydroxy-3-methoxyphenyl) acetate (18) 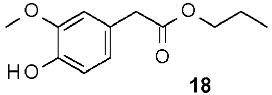
butyl-2-(4-hydroxy-3-methoxyphenyl) acetate (**19**) 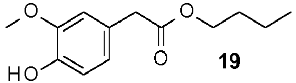
pentyl-2-(4-hydroxy-3-methoxyphenyl) acetate (**20**) 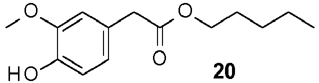
hexyl-2-(4-hydroxy-3-methoxyphenyl) acetate (**21**) 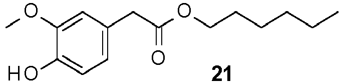
3-phenylpropyl 2-(4-hydroxy-3-methoxyphenyl) acetate (**22**) 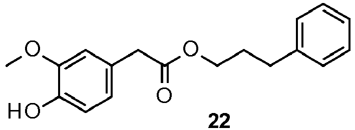
4-phenylbutyl 2-(4-hydroxy-3-methoxyphenyl) acetate (**23**)

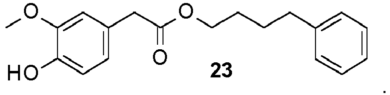

5. Use according to any one of the preceding claims in a pharmaceutical preparation, a preparation serving nutrition, oral hygiene or pleasure, preferably wherein the total quantity of compound(s) of formula (I) and/or salt(s) thereof in the preparation is sufficient to
sensorially create a warming and/or pungent effect on the tongue or in the oral cavity when the preparation is used or consumed,
and
enhance a pleasant taste sensation selected from the group consisting of warming, pungent and cooling.

6. Compound of the formula (I) or a physiologically acceptable salt thereof, wherein the phenolic hydroxy group in formula (I) is deprotonated, or a mixture comprising one or several different compounds of formula (I) and/or one or several physiologically acceptable salts thereof, wherein each phenolic hydroxy group in formula (I) is deprotonated, or consisting of a plurality of different compounds of formula (I) and/or physiologically acceptable salts thereof, wherein each phenolic hydroxy group in formula (I) is deprotonated, 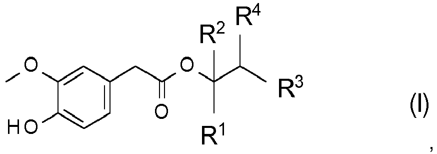 wherein
(i) R¹ and R² represent, independently of each other, a hydrogen atom or an alkyl residue with 1-2 carbon atoms,
R³ and R⁴ represent, independently of each other, a hydrogen atom or a linear or branched alkyl residue with 1 to 5 carbon atoms, a phenyl residue, an alkylphenyl residue or a phenylalkyl residue or a linear or branched alkenyl residue with 2 to 4 carbon atoms or an alkenylphenyl residue or a phenylalkenyl residue,
or
(ii) R¹ and R³ along with the carbon atoms linking them form a cyclohexyl ring, which optionally is substituted with an additional residue R⁵, wherein R⁵ is an alkyl residue with 1-2 carbon atoms,
R² represents a hydrogen atom or an alkyl residue with 1-2 carbon atoms,
R⁴ represents a hydrogen atom or a linear or branched alkyl residue with 1 to 5 carbon atoms, a phenyl residue, an alkylphenyl residue or a phenylalkyl residue or a linear or branched alkenyl residue with 2 to 4 carbon atoms or an alkenylphenyl residue or a phenylalkenyl residue,
provided that
the compound of formula (I) or, respectively, one or several or all compounds of the formula (I) in the mixture is or are selected from the group consisting of
[(*E*)-cinnamyl]-2-(4-hydroxy-3-methoxyphenyl) acetate (**2**) 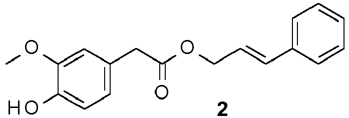
1-ethylbutyl-2-(4-hydroxy-3-methoxyphenyl) acetate (**3**) 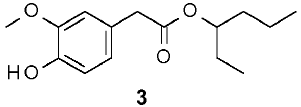
3-methylbut-2-enyl-2-(4-hydroxy-3-methoxyphenyl) acetate (**4**) 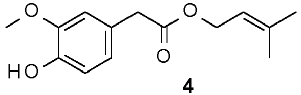
[(*E*)-hex-2-enyl]-2-(4-hydroxy-3-methoxyphenyl) acetate (**5**) 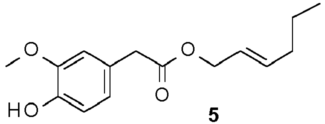
[(Z)-hex-3-enyl]-2-(4-hydroxy-3-methoxyphenyl) acetate (**6**) 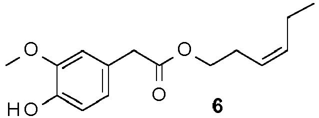
1,1-dimethylpropyl 2-(4-hydroxy-3-methoxyphenyl) acetate (**10**) 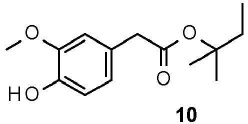
2-methylbutyl-2-(4-hydroxy-3-methoxyphenyl) acetate **(12)** 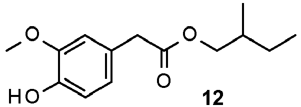
1-methylpentyl-2-(4-hydroxy-3-methoxyphenyl) acetate (**13**) 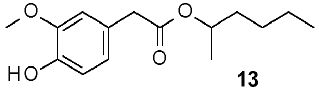
1-methylhexyl-2-(4-hydroxy-3-methoxyphenyl) acetate (**15**) 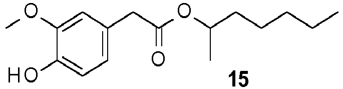
(2-isopropyl-5-methyl-cyclohexyl)-2-(4-hydroxy-3-methoxyphenyl) acetate (**16**) 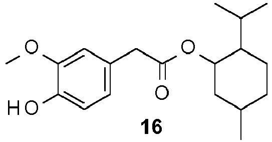
4-phenylbutyl 2-(4-hydroxy-3-methoxyphenyl) acetate (23)

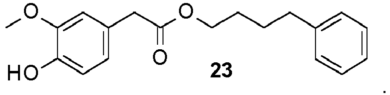

7. Flavour composition
comprising or consisting of a mixture according to claim 6,
preferably wherein
the total quantity of compound(s) of formula (I) and/or salt(s) thereof in the flavour composition is in the range of from 100 to 100,000 mg/kg, preferably in the range of from 250 to 40,000 mg/kg, particularly preferably in the range of from 250 to 15,000 mg/kg, based on the total weight of the flavour composition.

8. Flavour composition according to claim 7,
wherein
the total quantity of compound(s) of formula (I) and/or salt(s) thereof in the flavour composition is in the range of from 100 to 100,000 mg/kg, preferably in the range of from 250 to 40,000 mg/kg, particularly preferably in the range of from 250 to 15,000 mg/kg, based on the total weight of the flavour composition
additionally comprising one or several further flavours, which do not correspond to the formula (I), preferably selected from the group consisting of:
a) warming or pungent substances, preferably selected from the list consisting of: capsaicinoids, such as for example, capsaicin, dihydrocapsaicin or nonivamide; gingerols, such as for example, gingerol-6, gingerol-8, or gingerol-10; shogaols such as shogaol-6, shogaol-8, shogaol-10; gingerdiones, such as for example gingerdione-6, gingerdione-8 or gingerdione-10; paradols such as for example paradol-6, paradol-8 or paradol-10; dehydrogingerdiones such as for example dehydrogingerdione-6, dehydrogingerdione-8 or dehydrogingerdione-10; piperine and piperine derivatives;
b) substances perceivable as pungent or biting, preferably selected from the group consisting of: aromatic isothiocyanates, such as for example, phenylethyl isothiocyanate, allyl isothiocyanate, cyclopropylisothiocyanate, butyl isothiocyanate, 3-methylthiopropyl isothiocyanate, 4-hydroxybenzyl isothiocyanate, 4-methoxybenzyl isothiocyanate;
c) alkamides described as causing a tingling sensation, preferably selected from the group consisting of 2E,4E-decadienoic acid-N-isobutylamide (trans-pellitorine) (particularly such as described in WO 2004/043906; the respective compounds disclosed therein are herein incorporated by reference); 2E,4Z-decadienoic acid-N-isobutylamide (cis-pellitorine) (particularly such as described in WO 2004/000787; the respective compounds disclosed therein are herein incorporated by reference); 2Z,4Z-decadienoic acid-N-isobutylamide; 2Z,4E-decadienoic acid-N-isobutylamide; 2E,4E-decadienoic acid-N-([2S]-2-methylbutyl)amide; 2E,4E-decadienoic acid-N-([2S]-2-methylbutyl)amide; 2E,4E-decadienoic acid N-([2R]-2-methylbutylamide); 2E,4Z-decadienoic acid-N-(2-methylbutyl)amide; 2E,4E-decadienoic acid-N-piperide (achilleamide); 2E,4E-decadienoic acid-N-piperide (sarmentine); 2E-decenoic acid-N-isobutylamide; 3E-decenoic acid-N-isobutylamide; 3E-nonenoic acid-N-isobutylamide; 2E,6Z,8E-decatrienoic acid-N-isobutylamide (spilanthol); 2E,6Z,8E-decatrienoic acid-N-([2S] -2-methylbutyl)amide (homospilanthol); 2E,6Z,8E-decatrienoic acid-N-([2R]-2-methylbutyl)amide; 2E-decen-4-yne-acid-N-isobutylamide; 2Z-decen-4-yne-acid-N-isobutylamide; 2E, 6Z, 8E, 10E-dodecatetraenoic acid-N-(2-methylpropyl)amide (alpha-sanshool); 2E, 6Z, 8E, 10E-dodecatetraenoic acid-N-(2-hydroxy-2-methylpropyl)amide (alpha-hydroxysanshool); 2E, 6E, 8E, 10E-dodecatetraenoic acid-N-(2-hydroxy-2-methylpropyl)amide (gamma-hydroxysanshool); 2E, 4E, 8Z, 10E, 12E-tetradecapentaenoic acid, N-(2-hydroxy-2-methylpropyl)amide (gamma-hydroxysanshool); 2E, 4E, 8E, 10E, 12E-tetradecapentaenoic acid-N-(2-hydroxy-2-methylpropyl)amide (gamma-hydroxyisosanshool); 2E,4E,8Z,10E, 12E-tetradecapentaenoic acid-N-(2-methyl-2-propenyl)amide (gamma-dehydrosanshool); 2E, 4E, 8Z, 10E, 12E-tetradecapentaenoic acid-N-(2-methylpropyl)amide (gamma-sanshool); 2E,4E,8Z,11Z-tetradecatetraenoic acid-N-(2-hydroxy-2-methylpropyl)amide (bungeanool); 2E,4E,8Z,11E-tetradecatetraenoic acid-N-(2-hydroxy-2-methylpropyl)amide (isobungeanool); 2E,4E,8Z-tetradecatrienoic acid-N-(2-hydroxy-2-methylpropyl)amide (dihydrobungeanool) and 2E,4E-tetradecadienoic acid-N-(2-hydroxy-2-methylpropyl)amide (tetrahydrobungeanool);
d) substances with physiological cooling effect, preferably selected from the following list: menthol and derivatives thereof (e.g. L-menthol, D-menthol, racemic menthol, isomenthol, neoisomenthol, neomenthol) menthyl ether (e.g. (L-menthoxy)-1,2-propanediol, (L-menthoxy)-2-methyl-1,2-propanediol, L-menthylmethyl ether), menthyl ester (e.g. menthyl formate, menthyl acetate, menthyl isobutyrate, menthyl lactate, L-menthyl-L-lactate, L-menthyl-D-lactate, menthyl-(2-methoxy) acetate, menthyl-(2-methoxyethoxy) acetate, menthyl pyroglutamate), menthyl carbonate (e.g. menthyl propylene glycol carbonate, menthyl ethylene glycol carbonate, menthyl glycerol carbonate or mixtures thereof), the half-esters of menthols with a dicarboxylic acid or derivatives thereof (e.g.mono-menthyl succinate, mono-menthyl glutarate, mono-menthyl malonate, O-menthyl succinic acid ester-N, N-(dimethyl)amide, O-menthylsuccinic acid esteramide), menthanecarboxamides other than those mentioned in the present invention (e.g. menthane carboxylic acid-N-ethylamide [WS3], menthane carboxylic acid-N-(p-methoxyphenyl)amide [SC1], Na-(menthane carbonyl) glycine ethyl ester [WS5], menthane carboxylic acid-N-(4-cyanophenyl)amide, menthane carboxylic acid-N-(alkoxyalkyl)amides), menthone and derivatives thereof (e.g. L-menthone glycerine ketal), 2,3-dimethyl-2-(2-propyl) butanoic acid derivatives (e.g. 2,3-dimethyl-2-(2-propyl) butanoic acid -N-methylamide [WS23]), isopulegol or its esters (I-(-)-isopulegol, I-(-)-isopulegol acetate), menthane derivates (e.g. p-menthane-3,8-diol), cubebol or synthetic or natural mixtures, containing cubebol, pyrrolidone derivatives of cycloalkyldione derivatives (e.g. 3-methyl-2(1-pyrrolidinyl)-2-cyclopenten-1-one) or tetrahydropyrimidin-2-ones (e.g. icilin or related compounds, as described in WO 2004/026840), other coolants as described in WO2011061330, in particular, derivatives of differently substituted cinnamic and 2-phenoxy acids, particularly preferred methylene dioxy cinnamic acid-N,N-diphenylamide, methylene dioxy cinnamic acid-N-ethyl-N-phenylamide, methylene dioxy cinnamic acid-N-pyridyl-N-phenylamide;
e) substances having astringent effect, preferably selected from the following list: catechins such as e.g. epicatechins, gallocatechins, epigallocatechins and their respective gallic acid esters, e.g. epigallocatechin gallate or epicatechin gallate, their oligomers (procyanidines, proanthocyanidins, prodelphinidines, procyanirins, thearubigenins, theogallines) as well as their C- and O-glycosides; dihydroflavonoids such as dihydromyricetin, taxifolin as well as their C- and O-glycosides, flavonols such as myricetin, quercetin as well as their C- and O-glycosides such as quercetrin, rutin, gallic acid esters of carbohydrates such as tannin, pentagalloyl glucose or their reaction products, such as elligatannine, aluminium salts, e.g. alum.

9. Flavour composition according to claim 7 or 8, additionally comprising one or several substances which do not correspond to the formula (I), with an unpleasant, in particular, bitter taste, or an astringent, bitter, dry, dusty, floury, chalky and/or metallic touch, preferably selected from the group consisting of:
f) xanthin alkaloids, xanthines (caffeine, theobromine, theophylline and methylxanthine), alkaloids (quinine, brucine, strychnine, nicotine), phenolic glycosides (e.g. salicin, arbutin), flavonoid glycosides (e.g. neohespereidin, hesperidin, naringin, quercitrin, rutin, hyperosid, quercetin 3-O-glucoside, myricetin-3-O-glycosides), chalcones or chalcone glycosides (e.g. phloridzine, phloridzinxyloside), hydrolyzable tannins (gallic or ellagic acid esters of carbohydrates, e.g. pentagalloyl glucose, tannic acids), non-hydrolyzable tannins (if applicable galloylated catechins, gallocatechins, epigallocatechings or epicatechins and their oligomers, e.g. proanthyocyanidins or procyanidins, thearubigenin), flavones (e.g. quercetin, taxifolin, myricetin), phenols such as e.g. salicin, polyphenols (e.g. gamma-oryzanol, caffeic acid or esters thereof (e.g. chlorogenic acid and isomers)), terpenoid bitter and tanning agents (e.g. limonoides such as limonin or nomilin from citrus fruits, lupolones and humulones from hops, iridoids, secoiridoids), absinthin from wormwood, amarogentin from gentian, metallic salts (especially potassium, magnesium and calcium salts, potassium chloride, potassium gluconate, potassium carbonate, potassium sulphate, potassium lactate, potassium glutamate, potassium succinate, potassium malate, sodium sulphate, magnesium sulphate, aluminium salts, zinc salts, tin salts, iron (II) salts, iron (III) salts, chromium (II) picolinate), active pharmaceutical ingredients (e.g. fluoroquinolone antibiotics, paracetamol, aspirin, beta-lactam antibiotics, ambroxol, propylthiouracil [PROP], guaifenesin), vitamins (e.g. vitamin H, vitamins from the B-series, such as vitamin B1, B2, B6, B12, niacin, pantothenic acid), denatonium benzoate, sucralose octaacetate, iron salts, aluminium salts, zinc salts, urea, unsaturated fatty acids, especially unsaturated fatty acids in emulsions, amino acids with a bitter/astringent taste (e.g. leucine, isoleucine, valine, tryptophan, proline, histidine, tyrosine, lysine or phenylalanine) and peptides or proteins with a bitter/astringent taste (especially peptides with an amino acid from the group of leucine, isoleucine, valine, tryptophan, proline or phenylalanine at the N- or C-terminus), saponins, in particular, soya saponins, isoflavonoids (in particular, genistein, daidzein, genistein, daidzin, their glycosides and acylated glycosides);
g) substances with a non-unpleasant primary taste (e.g. sweet, salty, spicy, sour) and/or smell, preferably selected from the group of sweeteners or sugar substitutes, or selected from the group consisting of potassium salts (especially potassium chloride, potassium gluconate, potassium carbonate, potassium sulphate, potassium lactate, potassium glutamate, potassium succinate, potassium malate), aspartame, acesulfame K, neotame, superaspartame, saccharin, sucralose, tagatose, monellin, stevioside, rebaudiosides, rebaudioside A, rebaudioside B, rebaudioside C, rebaudioside D, rebaudioside X, rubusoside, hernandulcin, thaumatin, miculin, glycyrrhizin, glycyrrhetinic acid, balansin A or balansin B, or derivatives thereof, cyclamate or the pharmaceutically acceptable salts of the above-mentioned compounds.

10. Pharmaceutical preparation, preparation serving nutrition, oral hygiene or pleasure, comprising
(A) a mixture according to claim 6,
preferably wherein
the total quantity of compound(s) of formula (I) and/or salt(s) thereof in the preparation is in the range of from 0.1 to 1,000 mg/kg, preferably in the range of from 1 to 1,000 mg/kg, preferably in the range of from 1 to 750 mg/kg, particularly preferably in the range of from 5 to 500 mg/kg, based on the total weight of the preparation
or
(B) a flavour composition according to claim 7, 8 or 9,
preferably wherein
the total quantity of compound(s) of formula (I) and/or salt(s) thereof in the preparation is in the range of from 0.1 to 1,000 mg/kg, preferably in the range of from 1 to 1,000 mg/kg, preferably in the range of from 1 to 750 mg/kg, particularly preferably in the range of from 5 to 500 mg/kg, based on the total weight of the preparation
wherein the total quantity of compound(s) of the formula (I) and/or salt (s) thereof in the preparation is sufficient to
sensorially create a warming and/or pungent effect on the tongue or in the oral cavity when the preparation is used or consumed,
and
enhance a pleasant taste sensation selected from the group consisting of warming, pungent and cooling.

11. Preparation according to claim 10, additionally comprising
one or several usual base materials, auxiliaries and additives in a quantity of 5 - 99.9999 wt.-%, preferably 10 to 80 wt.-%, based on the total weight of the preparation,
and/or
water in a quantity of up to 99.9999 wt.-%, preferably in a quantity of up to 5 to 80 wt.-%, based on the total weight of the preparation.

12. Method for producing a preparation according to any one of the claims 10 or 11, comprising the following steps:
i) providing
(A) a mixture according to claim 6,
preferably wherein
the total quantity of compound(s) of formula (I) and/or salt(s) thereof is selected such that the total quantity in the preparation to be made is in the range of from 0.1 to 1,000 mg/kg, preferably in the range of from 1 to 1,000 mg/kg, preferably in the range of from 1 to 750 mg/kg, particularly preferably in the range of from 5 to 500 mg/kg, based on the total weight of the preparation,
or
(C) a flavour composition according to claim 7, 8 or 9,
preferably wherein
the total quantity of compound(s) of formula (I) and/or salt(s) thereof is selected such that the total quantity in the preparation to be made is in the range of from 0.1 to 1,000 mg/kg, preferably in the range of from 1 to 1,000 mg/kg, preferably in the range of from 1 to 750 mg/kg, particularly preferably in the range of from 5 to 500 mg/kg, based on the total weight of the preparation,
ii) providing of one or several further components of the preparation to be made, and
iii) contacting or mixing the further components provided in step ii) with the component(s) provided in step i), preferably in a sensorially effective quantity.

## Revendications

1. Utilisation d'un composé de formule (I) 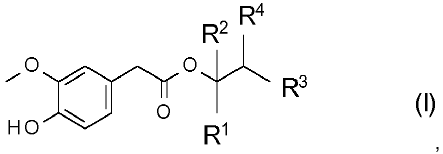 sachant que
(i) R¹ et R² représentent indépendamment l'un de l'autre un atome d'hydrogène ou un radical alkyle avec 1-2 atomes de carbone,
R³ et R⁴ représentent indépendamment l'un de l'autre un atome d'hydrogène ou un radical alkyle linéaire ou ramifié avec 1 à 5 atomes de carbone, un radical phényle, un radical alkylphényle ou un radical phénylalkyle ou un radical alcényle linéaire ou ramifié avec 2 à 4 atomes de carbone ou un radical alcénylphényle ou un radical phénylalcényle,
ou
(ii) R¹ et R³ forment ensemble avec les atomes de carbone qui les lient un cycle cyclohexyle éventuellement substitué par un radical R⁵ supplémentaire, où R⁵ est un radical alkyle avec 1-2 atomes de carbone,
R² représente un atome d'hydrogène ou un radical alkyle avec 1-2 atomes de carbone,
R⁴ représente un atome d'hydrogène ou un radical alkyle linéaire ou ramifié avec 1 à 5 atomes de carbone, un radical phényle, un radical alkylphényle ou un radical phénylalkyle ou un radical alcényle linéaire ou ramifié avec 2 à 4 atomes de carbone ou un radical alcénylphényle ou un radical phénylalcényle,
ou d'un sel physiologiquement acceptable dudit composé, le groupe hydroxyle phénolique étant déprotoné dans la formule (I), ou d'un mélange contenant un ou plusieurs composés différents de formule (I) et/ou des sels physiologiquement acceptables de tels composés, le groupe hydroxyle phénolique dans la formule (I) étant chaque fois déprotoné, ou constitué de plusieurs composés de formule (I) différents et/ou de sels de ces derniers, le groupe hydroxyle phénolique dans la formule (I) étant chaque fois déprotoné,
en tant que substance aromatique ou substance piquante ayant un effet générateur de chaleur et/ou de piquant,
et en tant que substance aromatique pour renforcer une sensation gustative agréable, sélectionnée dans le groupe constitué par la sensation de chaud, de piquant et de froid.

2. Utilisation selon la revendication 1, dans laquelle est valable pour le composé de formule (I) ou pour un, plusieurs ou la totalité des composés de formule (I) dans le mélange, indépendamment les uns des autres :
(i) R¹ et R² représentent indépendamment l'un de l'autre un atome d'hydrogène ou du méthyle,
R³ et R⁴ représentent indépendamment l'un de l'autre un atome d'hydrogène ou un radical alkyle linéaire ou ramifié avec 1 à 5 atomes de carbone ou un radical phényle, un radical alkylphényle ou un radical phénylalkyle ou un radical alcénylphényle ou un radical phénylalcényle,
ou
(ii) la formule (I) correspond à la formule suivante (la) 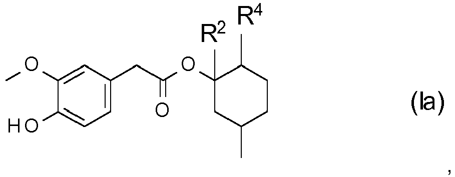 R² représente un atome d'hydrogène,
R⁴ représente un 2-propyle.

3. Utilisation selon l'une des revendications précédentes, dans laquelle est valable pour le composé de formule (I) ou pour un, plusieurs ou la totalité des composés de formule (I) dans le mélange, indépendamment les uns des autres :
R¹ et R² représentent chacun un atome d'hydrogène,
R³ représente un atome d'hydrogène ou un radical alkyle linéaire ou ramifié avec 1 à 4 atomes de carbone ou un radical phényle, un radical alkylphényle ou un radical phénylalkyle ou un radical alcénylphényle ou un radical phénylalcényle,
R⁴ représente un atome d'hydrogène.

4. Utilisation selon l'une des revendications précédentes, dans laquelle le composé de formule (I) ou un, plusieurs ou tous les composés de formule (I) dans le mélange est ou sont sélectionné(s) dans le groupe composé de :
2-(4-hydroxy-3-méthoxy-phényl)acétate de 2-phényléthyle (**1**) 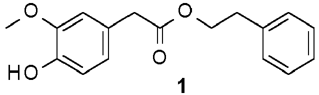
2-(4-hydroxy-3-méthoxy-phényl)acétate de (*E*)-cinnamyle (**2**) 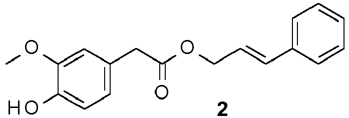
2-(4-hydroxy-3-méthoxy-phényl)acétate de 1-éthylbutyle (**3**) 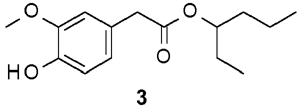
2-(4-hydroxy-3-méthoxy-phényl)acétate de 3-méthylbut-2-ényle (**4**) 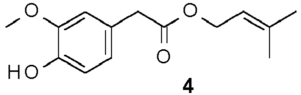
2-(4-hydroxy-3-méthoxy-phényl)acétate de (*E*)-hex-2-ényle (**5**) 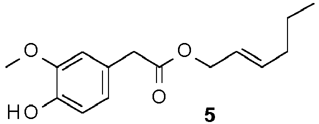
2-(4-hydroxy-3-méthoxy-phényl)acétate de (Z)-hex-3-ényle (**6**) 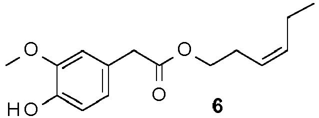
2-(4-hydroxy-3-méthoxy-phényl)acétate d'isopropyle (**7**) 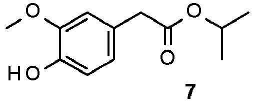
2-(4-hydroxy-3-méthoxy-phényl)acétate de *sec-*butyle (**8**) 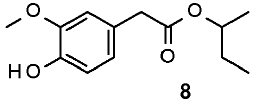
2-(4-hydroxy-3-méthoxy-phényl)acétate d'isobutyle (**9**) 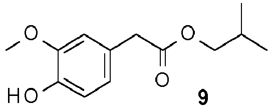
2-(4-hydroxy-3-méthoxy-phényl)acétate de 1,1-diméthylpropyle (**10**) 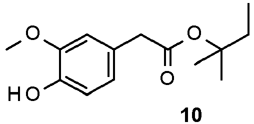
2-(4-hydroxy-3-méthoxy-phényl)acétate d'isopentyle (**11**) 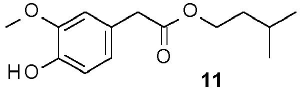
2-(4-hydroxy-3-méthoxy-phényl)acétate de 2-méthylbutyle (**12**) 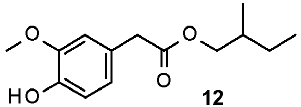
2-(4-hydroxy-3-méthoxy-phényl)acétate de 1-méthylpentyle (**13**) 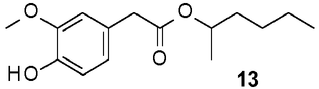
2-(4-hydroxy-3-méthoxy-phényl)acétate d'heptyle (**14**) 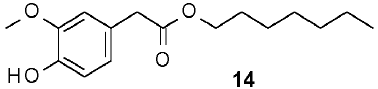
2-(4-hydroxy-3-méthoxy-phényl)acétate de 1-méthylhexyle (**15**) 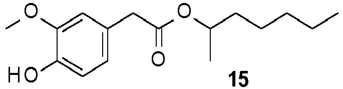
2-(4-hydroxy-3-méthoxy-phényl)acétate de 2-isopropyl-5-méthyl-cyclohexyle (**16**) 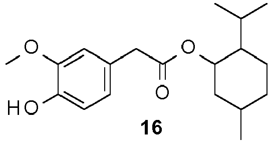
2-(4-hydroxy-3-méthoxy-phényl)acétate d'éthyle (17) 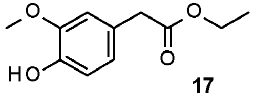
2-(4-hydroxy-3-méthoxy-phényl)acétate de propyle (**18**) 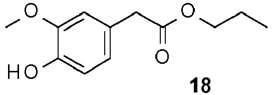
2-(4-hydroxy-3-méthoxy-phényl)acétate de butyle (**19**) 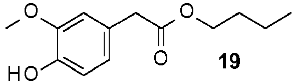
2-(4-hydroxy-3-méthoxy-phényl)acétate de pentyle (**20**) 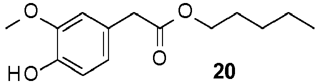
2-(4-hydroxy-3-méthoxy-phényl)acétate d'hexyle (**21**) 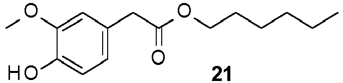
2-(4-hydroxy-3-méthoxy-phényl)acétate de 3-phénylpropyle (**22**) 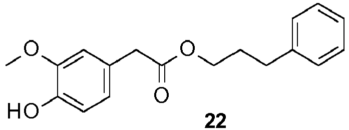
2-(4-hydroxy-3-méthoxy-phényl)acétate de 4-phénylbutyle (**23**)

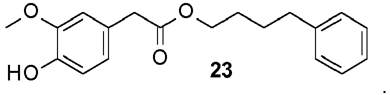

5. Utilisation selon l'une des revendications précédentes dans une préparation pharmaceutique, dans une préparation servant à la nutrition, à l'hygiène buccale ou à l'agrément, de préférence dans laquelle la quantité totale du/de composé(s) de formule (I) et/ou de sel(s) d'un tel/de tels composé(s) dans la composition est suffisante pour,
après l'utilisation ou la consommation de la préparation, produire un effet sensoriel de chaud et/ou un effet piquant sur la langue ou dans la cavité buccale,
renforcer une sensation gustative agréable, sélectionnée dans le groupe constitué par la sensation de chaud, de piquant et de froid.

6. Composé de formule (I) ou sel physiologiquement acceptable dudit composé, le groupe hydroxyle phénolique étant déprotoné dans la formule (I), ou mélange contenant un ou plusieurs composés de formule (I) différents et/ou un ou plusieurs sels physiologiquement acceptables de tels composés, le groupe hydroxyle phénolique dans la formule (I) étant chaque fois déprotoné, ou constitué de plusieurs composés de formule (I) différents et/ou de sels physiologiquement acceptables de ces derniers, le groupe hydroxyle phénolique dans la formule (I) étant chaque fois déprotoné, 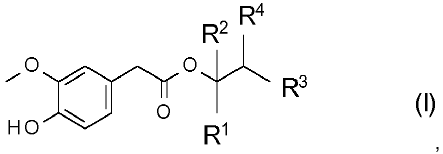 sachant que
(i) R¹ et R² représentent indépendamment l'un de l'autre un atome d'hydrogène ou un radical alkyle avec 1-2 atomes de carbone,
R³ et R⁴ représentent indépendamment l'un de l'autre un atome d'hydrogène ou un radical alkyle linéaire ou ramifié avec 1 à 5 atomes de carbone, un radical phényle, un radical alkylphényle ou un radical phénylalkyle ou un radical alcényle linéaire ou ramifié avec 2 à 4 atomes de carbone ou un radical alcénylphényle ou un radical phénylalcényle,
ou
(ii) R¹ et R³ forment ensemble avec les atomes de carbone qui les lient un cycle cyclohexyle éventuellement substitué par un radical R⁵ supplémentaire, où R⁵ est un radical alkyle avec 1-2 atomes de carbone,
R² représente un atome d'hydrogène ou un radical alkyle avec 1-2 atomes de carbone,
R⁴ représente un atome d'hydrogène ou un radical alkyle linéaire ou ramifié avec 1 à 5 atomes de carbone, un radical phényle, un radical alkylphényle ou un radical phénylalkyle ou un radical alcényle linéaire ou ramifié avec 2 à 4 atomes de carbone ou un radical alcénylphényle ou un radical phénylalcényle,
à condition que
le composé de formule (I) ou un, plusieurs ou la totalité des composés de formule (I) dans le mélange soit ou soient sélectionné(s) dans le groupe composé de :
2-(4-hydroxy-3-méthoxy-phényl)acétate de (*E*)-cinnamyle (**2**) 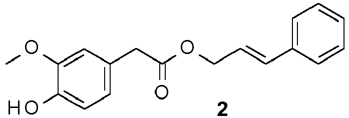
2-(4-hydroxy-3-méthoxy-phényl)acétate de 1-éthylbutyle (**3**) 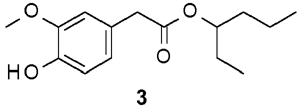
2-(4-hydroxy-3-méthoxy-phényl)acétate de 3-méthylbut-2-ényle (**4**) 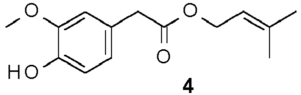
2-(4-hydroxy-3-méthoxy-phényl)acétate de (*E*)-hex-2-ényle (**5**) 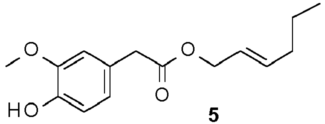
2-(4-hydroxy-3-méthoxy-phényl)acétate de (Z)-hex-3-ényle (**6**) 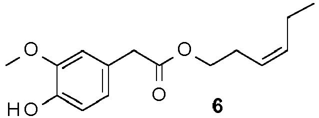
2-(4-hydroxy-3-méthoxy-phényl)acétate de 1,1-diméthylpropyle (**10**) 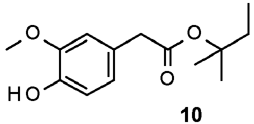
2-(4-hydroxy-3-méthoxy-phényl)acétate de 2-méthylbutyle (**12**) 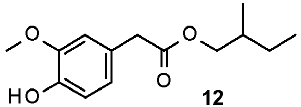
2-(4-hydroxy-3-méthoxy-phényl)acétate de 1-méthylpentyle (**13**) 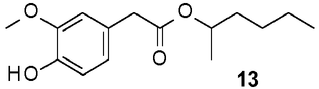
2-(4-hydroxy-3-méthoxy-phényl)acétate de 1-méthylhexyle (**15**) 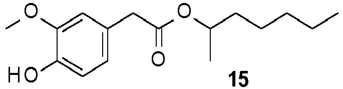
2-(4-hydroxy-3-méthoxy-phényl)acétate de 2-isopropyl-5-méthyl-cyclohexyle (**16**) 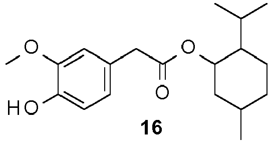
2-(4-hydroxy-3-méthoxy-phényl)acétate de 4-phénylbutyle (**23**)

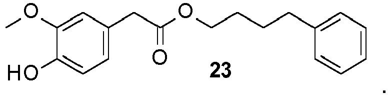

7. Composition aromatique
contenant ou consistant en un mélange selon la revendication 6, dans laquelle, de préférence,
la quantité totale de composé(s) de formule (I) et/ou de sel(s) de ce/ces composé(s) dans la composition aromatique, par rapport au poids total de la composition aromatique, se situe dans la plage de 100 à 100 000 mg/kg, de préférence dans la plage de 250 à 40 000 mg/kg, de manière particulièrement préférée dans la plage de 250 à 15 000 mg/kg.

8. Composition aromatique selon la revendication 7,
sachant que
la quantité totale de composé(s) de formule (I) et/ou de sel(s) de ce/ces composé(s) dans la composition aromatique, par rapport au poids total de la composition aromatique, se situe dans la plage de 100 à 100 000 mg/kg, de préférence dans la plage de 250 à 40 000 mg/kg, de manière particulièrement préférée dans la plage de 250 à 15 000 mg/kg,
contenant en outre une ou plusieurs substances aromatiques supplémentaires ne correspondant pas à la formule (I), de préférence sélectionnées dans le groupe constitué de
a) substances provoquant la chaleur ou piquantes, de préférence sélectionnées dans la liste constituée de : capsaïcinoïdes, tels que la capsaïcine, la dihydro-capsaïcine ou le nonivamide ; gingéroles tels que le gingérol-6, le gingérol-8 ou le gingérol-10 ; shogaols, tels que le shogaol-6, le shogaol-8 ou le shogaol-10 ; gingerdiones, telles que la gingerdione-6, la gingerdione-8 ou la gingerdione-10 ; paradols, tels que le paradol-6, le paradol-8 ou le paradol-10 ; déhydrogingerdiones, telles que la déhydrogingerdione-6, la déhydrogingerdione-8 ou la déhydrogingerdione-10 ; pipérine et dérivés de la pipérine ;
b) en tant que substances perçues comme piquantes ou âcres, de préférence sélectionnées dans le groupe constitué par : isothiocyanates aromatiques, tels que l'isothiocyanate de phényléthyle, l'isothiocyanate d'allyle, l'isothiocyanate de cyclopropyle, l'isothiocyanate de butyle, l'isothiocyanate de 3-méthylthiopropyle, l'isothiocyanate de 4-hydroxybenzoyle, l'isothiocyanate de 4-méthoxybenzoyle ;
c) alcamides décrits comme produisant un effet de picotement (tingling), sélectionnés de préférence dans le groupe composé du N-isobutylamide de l'acide 2E,4E-décadiénoïque (trans-pellitorine) (en particulier celui tel que décrit dans le document WO 2004/043906, qui devient partie de la présente demande de brevet par voie de référence pour les composés correspondants qui y sont divulgués) ; du N-isobutylamide de l'acide 2E,4Z-décadiénoïque (cis-pellitorine), (en particulier celui tel que décrit dans le document WO 2004/000787, qui devient partie de la présente demande de brevet par voie de référence pour les composés correspondants qui y sont divulgués) ; du N-isobutylamide de l'acide 2Z,4Z-décadiénoïque ; du N-isobutylamide de l'acide 2Z,4E-décadiénoïque ; du N-([2S]-2-méthylbutyl)amide de l'acide 2E,4E-décadiénoïque ; du N-([2S]-2-méthylbutyl)amide de l'acide 2E,4E-décadiénoïque ; du N-([2R]-2-méthylbutylamide de l'acide 2E,4E-décadiénoïque ; du N-(2-méthylbutyl)amide de l'acide 2E,4Z-décadiénoïque ; du N-pipéride de l'acide 2E,4E-décadiénoïque (achilleamide) ; du N-pipéride de l'acide 2E,4E-décadiénoïque (sarmentine) ; du N-isobutylamide de l'acide 2E-décénoïque ; du N-isobutylamide de l'acide 3E-décénoïque ; du N-isobutylamide de l'acide 3E-nonénoïque ; du N-isobutylamide de l'acide 2E,6Z,8E-décatriénoïque (spilanthol) ; du N-([2S]-2-méthylbutyl)amide de l'acide 2E,6Z,8E-décatriénoïque (homospilanthol); du N-([2R]-2-méthylbutyl)amide de l'acide 2E,6Z,8E-décatriénoïque ; du N-isobutylamide de l'acide 2E-décén-4-inique ; du N-isobutylamide de l'acide 2Z-décén-4-inique ; du N-(2-méthylpropyl)amide de l'acide 2E,6Z,8E,10E-dodécatétraénoïque (alpha-sanshool) ; du N-(2-hydroxy-2-méthylpropyl)amide de l'acide 2E,6Z,8E,10E-dodécatétraénoïque (alpha-hydroxysanshool) ; de N-(2-hydroxy-2-méthylpropyl)amide de l'acide 2E,6E,8E,10E-dodécatétraénoïque (gamma-hydroxysanshool) ; du N-(2-hydroxy-2-méthylpropyl)amide de l'acide 2E,4E,8Z,10E,12E-tétradécapentaénoïque (gamma-hydroxysanshool) ; du N-2-hydroxy-2-méthylpropyl)amide de l'acide 2E,4E,8E,10E,12E-tétradécapentaénoïque (gamma-hydroxyisosanshool) ; du N-(2-méthyl-2-propényl)amide de l'acide 2E,4E,8Z,10E,12E-tétradécapentaénoïque (gamme-déhydrosanshool) ; du N-(2-méthylpropyl)amide de l'acide 2E,4E,8Z,10E,12E-tétradécapentaénique (gammasanshool) ; du N-(2-hydroxy-2-méthylpropyl)amide de l'acide 2E,4E,8Z,11Z-tétradécatétraénoïque (bungéanool) ; du N-(2-hydroxy-2-méthylpropyl)amide de l'acide 2E,4E,8Z,11E-tétradécatétraénoïque (isobungéanool) ; du N-(2-hydroxy-2-méthylpropyl)amide de l'acide 2E,4E,8Z-tétradécatriénoïque (dihydrobungéanool) et du N-(2-hydroxy-2-méthylpropyl)amide de l'acide 2E,4E-tétradécadiénoïque (tétrahydrobungéanool) ;
d) substances produisant un effet de refroidissement physiologique, sélectionnés de préférence dans la liste suivante : menthol et dérivés de menthol (par ex. L-menthol, d-menthol, menthol racémique, isomenthol, néoisomenthol, néomenthol), menthyléthers, (par ex. (L-menthoxy)-1,2-propanediol, (L-menthoxy)-2-méthyl-1,2-propanediol, L-menthyl-méthyléther), esters de menthyle (par ex. formiate de menthyle, acétate de menthyle, isobutyrate de menthyle, lactates de menthyle, L-lactate de L-menthyle, D-lactate de L-menthyle, (2-méthoxy)acétate de menthyle, (2-méthoxyéthoxy)acétate de menthyle, pyroglutamate de menthyle, carbonates de menthyle (par ex. propylèneglycolcarbonate de menthyle, éthylèneglycérolcarbonate de menthyle, glycérolcarbonate de menthyle ou leurs mélanges), les hémiesters de menthols avec un acide dicarboxylique ou leurs dérivés (par ex. succinate de monomenthyle, glutarate de monomenthyle, malonate de monomenthyle, ester-N-N-(diméthyl)amide d'acide O-menthylsuccinique, ester-amide d'acide O-menthylsuccinique), autres amides d'acide menthanecarboxylique que ceux cités dans la présente invention, (par ex. N-éthylamide d'acide menthanecarboxylique [WS3], N-(p-méthoxyphényl)amide d'acide menthanecarboxylique [SC1], ester éthylique de Na(menthanecarbonyl)glycine [WS5], N-(4-cyanophényl)amide d'acide menthanecarboxylique, N-(alcoxyalkyl)amides d'acide menthanecarboxylique), menthone et dérivés de menthone (par ex. L-menthoneglycérolcétal), dérivés d'acide 2,3-diméthyl-2-(2-propyl)butyrique (par ex. N-métylamide d'acide 2,3-diméthyl-2-(2-propyl)butyrique [WS23]), isopulégol et ses esters (I-(-)-isopulégol, acétate de I-(-)-isopulégol), dérivés de menthane (par ex. p-menthane-3,8-diol), cubébol ou mélanges synthétiques ou naturels à base de cubébol, dérivés de pyrrolidone de dérivés de cycloalkyldione (par ex. 3-méthyl-2(1-pyrrolidinyl)-2-cyclopentèn-1-one) ou tétrahydropyrimidin-2-one (par exemple iciline ou composés apparentés, tels que décrits dans le document WO 2004/026840), autres substances réfrigérantes telles que décrites dans le document WO 2011061330, en particulier, dérivés d'acide cinnamique et 2-phénoxy différemment substitués, de manière particulièrement préférée, N,N-diphénylamide d'acide méthylènedioxycinnamique, N-éthyl-N-phénylamide d'acide méthylènedioxycinnamique, N-pyridyl-N-phénylamide d'acide méthylènedioxycinnamique ;
e) substances produisant un effet astringent, sélectionnés de préférence dans la liste suivante : catéchines telles que les épicatéchines, gallocatéchines, épigallocatéchines et leurs esters d'acide gallique respectifs, par ex. gallate d'épigallocatéchine ou gallate d'épicatéchine, leurs oligomères (procyanidines, proanthocyanidines, prodelphinidines, procyanirines, théarubigénines, théogallines) ainsi que leurs C- et O-glycosides ; dihydroflavonoïdes tels que dihydromyricétine, taxifoline ainsi que leurs C- et O-glycosides, flavonols tels que myricétine, quercétine ainsi que leurs C- et O-glycosides tels que quercétine, rutine, esters d'acides gallique avec des glucides tels que tannin, pentagalloylglucose ou leurs produits de réaction tels que l'elligatanin, sels d'aluminium, par ex. alun.

9. Composition aromatique selon la revendication 7 ou 8, contenant en outre une ou plusieurs substances ne correspondant pas à la formule (I) ayant une qualité gustative désagréable, en particulier amère, ou une note astringente, amère, sèche, poussiéreuse, farineuse, calcaire et/ou métallique, sélectionnées de préférence dans le groupe composé de :
f) alcaloïdes de xanthine, xanthines (caféine, théobromine, théophylline et méthylxanthines), alcaloïdes (quinine, brucine, strychnine, nicotine), glycosides phénoliques (par ex. salicine, arbutine), glycosides flavonoïdes (par ex. néohespéridine, hespéridine, naringine, quercitrine, rutine, hypéroside, quercétine-3-O-glucoside, myricétine-3-O-glycosides), chalcones ou glycosides de chalcones (par ex. phloridzine, phloridzinxylosides), tanins hydrolysables (esters d'acide gallique ou ellagique de carbohydrates, par ex. pentagalloylglucose, acides tanniques), tanins non hydrolysables (le cas échéant, catéchines galloylées, gallocatéchines, épigallocatéchines ou épicatéchines et leurs oligomères, par ex. proanthyocyanidines ou procyanidines, théarubigénine), flavones (par ex. quercétine, taxifoline, myricétine), phénols tels que salicine, polyphénols (p. ex. gamma-oryzanol, acide caféique ou ses esters (p. ex. acide chlorogénique et isomères), les substances amères et tannantes terpénoïdes (par ex. limonoïdes tels que limonine ou nomiline des agrumes, lupolones et humolones du houblon, iridoïdes, sécoiridoïdes), absinthine de l'absinthe, amarogentine de la gentiane, sels métalliques (en particulier sels de potassium, de magnésium et de calcium, chlorure de potassium, gluconate de potassium, carbonate de potassium, sulfate de potassium, lactate de potassium, glutamate de potassium, succinate de potassium, malate de potassium, sulfate de sodium, sulfate de magnésium, sels d'aluminium, sels de zinc, sels d'étain, sels de fer (II), sels de fer (III), picolinate de chrome (II)), principes actifs pharmaceutiques (par ex. antibiotiques de la famille des fluoroquinolones, paracétamol, aspirine, antibiotiques de la famille des (3-lactames, ambroxol, propylthiouracile [PROP], guaïfénésine), vitamines (par ex. vitamine H, vitamines de la série B telles que les vitamines B1, B2, B6, B12, niacine, acide panthoténique), benzoate de dénatonium, octaacétate de sucralose, sels de fer, sels d'aluminium, sels de zinc, urée, acides gras insaturés, en particulier acides gras insaturés dans les émulsions, acides aminés au goût amer/astringent (par ex. leucine, isoleucine, valine, tryptophane, proline, histidine, tyrosine, lysine ou phénylalanine) et peptides ou protéines au goût amer ou astringent (en particulier peptides avec un acide aminé du groupe constitué de leucine, isoleucine, valine, tryptophane, proline ou phénylalanine à l'extrémité N- ou C-terminale), saponines, en particulier saponines de soja, isoflavonoïdes (en particulier génistéine, daidzéine, génistine, daidzine, leurs glycosides et glycosides acylés) ;
g) Substances ayant un goût et/ou une odeur primaire non désagréable (par ex. sucré, salé, épicé, acide), sélectionnées de préférence dans le groupe des édulcorants ou des édulcorant de charge, de préférence de préférence sels de potassium, (en particulier chlorure de potassium, gluconate de potassium, carbonate de potassium, sulfate de potassium, lactate de potassium, glutamate de potassium, succinate de potassium, malate de potassium), aspartame, acésul-fame K, néotame, superaspartame, saccharine, sucralose, tagatose, monelline, stévio-sides, rébaudiosides, rébaudioside A, rébaudioside B, rébaudioside C, rébaudioside D, rébaudioside X, rubusosides, hernandulcine, thaumatine, miraculine, glycyrrhizine, acide glycyrrhizique, balansine A ou balansine B, ou leurs dérivés, cyclamate ou les sels pharmaceutiquement acceptables des composés mentionnés ci-dessus.

10. Préparation pharmaceutique, préparation servant à la nutrition, à l'hygiène buccale ou à l'agrément, contenant
(A) un mélange selon la revendication 6,
dans laquelle, de préférence,
la quantité totale de composé(s) de formule (I) et/ou de sel(s) de ce/ces composé(s) dans la préparation, par rapport au poids total de la préparation, se situe dans la plage de 0,1 à 1 000 mg/kg, de préférence dans la plage de 1 à 1 000 mg/kg, de préférence dans la plage de 1 à 750 mg/kg, de manière particulièrement préférée dans la plage de 5 à 500 mg/kg,
ou
(C) une composition aromatique selon la revendication 7, 8 ou 9,
dans laquelle, de préférence,
la quantité totale de composé(s) de formule (I) et/ou de sel(s) de ce/ces composé(s) dans la préparation, par rapport au poids total de la préparation, se situe dans la plage de 0,1 à 1 000 mg/kg, de préférence dans la plage de 1 à 1 000 mg/kg, de préférence dans la plage de 1 à 750 mg/kg, de manière particulièrement préférée dans la plage de 5 à 500 mg/kg,
dans laquelle la quantité totale du/de composé(s) de formule (I) et/ou de sel(s) d'un tel/de tels composé(s) dans la préparation est suffisante pour,
après l'utilisation ou la consommation de la préparation, produire un effet sensoriel de chaud et/ou un effet piquant sur la langue ou dans la cavité buccale,
et renforcer une sensation gustative agréable, sélectionnée dans le groupe constitué par la sensation de chaud, de piquant et de froid.

11. Préparation selon la revendication 10, contenant en outre
un(e) ou plusieurs substances de base, adjuvants et additifs usuel(le)s en une quantité de 5 à 99,9999 % en poids, de préférence de 10 à 80 % en poids, par rapport au poids total de la préparation,
et/ou
de l'eau en une quantité atteignant 99,9999 % en poids, de préférence en une quantité de 5 à 80 % en poids par rapport au poids total de la préparation.

12. Procédé pour préparer une préparation selon l'une des revendications 10 à 11, comprenant les étapes consistant à :
i) fournir
(A) un mélange selon la revendication 6,
dans lequel, de préférence,
la quantité totale de composé(s) de formule (I) et/ou de sel(s) de ce/ces composé(s) est choisie de sorte que la quantité totale dans la préparation devant être préparée, par rapport au poids total de la préparation, se situe dans une plage de 0,1 à 1 000 mg/kg, de préférence de 1 à 1 000 mg/kg, de préférence de 1 à 750 mg/kg, de manière particulièrement préférée, de 5 à 500 mg/kg,
ou
(C) une composition aromatique selon la revendication 7, 8 ou 9,
dans laquelle, de préférence,
la quantité totale de composé(s) de formule (I) et/ou de sel(s) est choisie de sorte que la quantité totale dans la préparation devant être préparée, par rapport au poids total de la préparation, se situe dans la plage de 0,1 à 1 000 mg/kg, de préférence dans la plage de 1 à 1 000 mg/kg, de préférence dans la plage de 1 à 750 mg/kg, de manière particulièrement préférée dans la plage de 5 à 500 mg/kg,
ii) fournir un ou plusieurs composants supplémentaires de la préparation devant être préparée, et
iii) mettre en contact ou mélanger les composants supplémentaires fournis à l'étape (ii) avec le/les composant(s) fourni(s) à l'étape (i), de préférence en une quantité efficace sur le plan sensoriel.
